(19) Europäisches Patentamt / European Patent Office / Office européen des brevets

(11) **EP 1 802 646 B1**

(12) **EUROPEAN PATENT SPECIFICATION**

(45) Date of publication and mention of the grant of the patent: **06.09.2017 Bulletin 2017/36**

(21) Application number: **05857896.4**

(22) Date of filing: **25.07.2005**

(51) Int Cl.: ***C07K 16/00*** *(2006.01)* ***C12P 21/02*** *(2006.01)* ***C07K 14/47*** *(2006.01)*

(86) International application number: **PCT/US2005/026224**

(87) International publication number: **WO 2007/015691 (08.02.2007 Gazette 2007/06)**

(54) **METHODS AND COMPOSITIONS FOR INCREASING LONGEVITY AND PROTEIN YIELD FROM A CELL CULTURE**

VERFAHREN UND ZUSAMMENSETZUNGEN ZUR ERHÖHUNG VON LEBENSDAUER UND PROTEINERTRAG EINER ZELLKULTUR

PROCEDES ET COMPOSITIONS DESTINES A ACCROITRE LA LONGEVITE ET LA PRODUCTION DE PROTEINES DANS UNE CULTURE CELLULAIRE

(84) Designated Contracting States: **AT BE BG CH CY CZ DE DK EE ES FI FR GB GR HU IE IS IT LI LT LU LV MC NL PL PT RO SE SI SK TR**

(30) Priority: **23.07.2004 US 590349 P**

(43) Date of publication of application: **04.07.2007 Bulletin 2007/27**

(73) Proprietor: **Immunomedics Inc. Morris Plains, NJ 07950 (US)**

(72) Inventors:

- **GOLDENBERG, David M. Morris Plains, NJ 07950 (US)**
- **QU, Zhengxing Morris Plains, NJ 07950 (US)**
- **HORAK, Eva Morris Plains, NJ 07950 (US)**
- **HORAK, Ivan D. Morris Plains, NJ 07950 (US)**
- **CHANG, Chien-hsing Ken Morris Plains, NJ 07950 (US)**
- **ROSSI, Edmund A. Morris Plains, NJ 07950 (US)**
- **YANG, Jeng-dar Morris Plains, NJ 07950 (US)**

(74) Representative: **Bohmann, Armin K. Bohmann Anwaltssozietät Nymphenburger Straße 1 80335 München (DE)**

(56) References cited:

**US-A1- 2003 064 510**
**US-A1- 2003 219 871**
**US-B1- 6 586 206**
**US-B2- 6 964 199**
**US-B2- 7 537 930**

- **ARDEN N ET AL: "Life and death in mammalian cell culture: strategies for apoptosis inhibition" TRENDS IN BIOTECHNOLOGY, ELSEVIER PUBLICATIONS, CAMBRIDGE, GB, vol. 22, no. 4, 1 April 2004 (2004-04-01), pages 174-180, XP004497320 ISSN: 0167-7799**
- **STOREY ALAN: "Papillomaviruses: Death-defying acts in skin cancer" TRENDS IN MOLECULAR MEDICINE, vol. 8, no. 9, September 2002 (2002-09), pages 417-421, XP002544259 ISSN: 1471-4914**
- **JACKSON SARAH ET AL: "E6 proteins from diverse cutaneous HPV types inhibit apoptosis in response to UV damage" ONCOGENE, vol. 19, no. 4, 27 January 2000 (2000-01-27), pages 592-598, XP002544260 ISSN: 0950-9232**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 802 646 B1

- ROMANOS MA. PRODUCTION OF A PHOSPHORYLATED GST:HPV-6 E7 FUSION PROTEIN USING A YEAST EXPRESSION VECTOR AND GLUTATHIONE S-TRANSFERASE FUSIONS. vol. 152, no. 1, January 1995, pages 137 - 8, XP008120868
- DENG X ET AL: "Mono- and multisite phosphorylation enhances Bcl2's antiapoptotic function and inhibition of cell cycle entry functions", PROCEEDINGS OF THE NATIONAL ACADEMY OF SCIENCES OF THE UNITED STATES OF AMERICA 200401 US,, vol. 101, no. 1, 1 January 2004 (2004-01-01), pages 153-158, XP002508956, DOI: 10.1073/PNAS.2533920100

## Description

The present invention is related to a method of making a recombinant protein and a host cell transfected with an expression vector.

### BACKGROUND OF THE INVENTION

Culturing cells in vitro, especially in large bioreactors, has been the basis of the production of numerous biotechnology products, and involves the elaboration by these cells of protein products into the support medium, from which these products are isolated and further processed prior to use clinically. The quantity of protein production over time from the cells growing in culture depends on a number of factors, such as, for example, cell density, cell cycle phase, cellular biosynthesis rates of the proteins, condition of the medium used to support cell viability and growth, and the longevity of the cells in culture (i.e., how long before they succumb to programmed cell death, or apoptosis). Various methods of improving the viability and lifespan of the cells in culture have been developed, together with methods of increasing productivity of a desired protein by, for example, controlling nutrients, cell density, oxygen and carbon dioxide content, lactate dehydrogenase, pH, osmolarity, catabolites, etc. For example, increasing cell density can make the process more productive, but can also reduce the lifespan of the cells in culture. Therefore, it may be desirous to reduce the rate of proliferation of such cells in culture when the maximal density is achieved, so as to maintain the cell population in its most productive state as long as possible. This results in increasing or extending the bioreactor cycle at its production peak, elaborating the desired protein products for a longer period, and this results in a higher yield from the bioreactor cycle.

Many different approaches have been pursued to increase the bioreactor cycle time, such as adjusting the medium supporting cell proliferation, addition of certain growth-promoting factors, as well as inhibiting cell proliferation without affecting protein synthesis. One particular approach aims to increase the lifespan of cultured cells via controlling the cell cycle by use of genes or antisense oligonucleotides to affect cell cycle targets, whereby a cell is induced into a pseudo-senescence stage by transfecting, transforming, or infecting with a vector that prevents cell cycle progression and induces a so-called pseudo-senescent state that blocks further cell division and expands the protein synthesis capacity of the cells in culture; in other words, the pseudo-senescent state can be induced by transforming the cells with a vector expressing a cell cycle inhibitor (Bucciarelli et al., US Patent 2002/0160450 A1; idem., WO 02/16590 A2). The latter method, by inhibiting cell duplication, seeks to force cells into a state that may have prolonged cell culture lifetimes, as described by Goldstein and Singal (Exp Cell Res 88, 359-64, 1974; Brenner et al., Oncogene 17:199-205, 1998), and may be resistant to apoptosis (Chang et al., Proc Natl Acad Sci USA 97, 4291-6, 2000; Javeland et al., Oncogene 19, 61-8, 2000).

Still another approach involves establishing primary, diploid human cells or their derivatives with unlimited proliferation following transfection with the adenovirus E1 genes. The new cell lines, one of which is PER.C6 (ECACC deposit number 96022940), which expresses functional Ad5 E1A and E1B gene products, can produce recombinant adenoviruses, as well as other viruses (e.g., influenza, herpes simplex, rotavirus, measles) designed for gene therapy and vaccines, as well as for the production of recombinant therapeutic proteins, such as human growth factors and human antibodies (Vogels et al., WO 02/40665 A2).

Other approaches have focused on the use of caspase inhibitors for preventing or delaying apoptosis in cells. See, for example, US Patent No. 6,586,206. Still other approaches have tried to use apoptosis inhibitors such as members of the Bcl-2 family for preventing or delaying apoptosis in cells. See Arden et al., Bioprocessing Journal, 3:23-28 (2004). These approaches have yielded unpredictable results; for example, in one study, expression of Bcl-2 increased cell viability but did not increase protein production. See Tey et al. Biotechnol. Bioeng. 68:31-43 (2000). Another example described overexpression of Bcl-2 proteins to delay apoptosis in CHO cells, but Bcl-xL increased protein production whereas Bcl-2 decreased protein production (see WO03/083093). A further example described experiments using expression of Bcl-2 proteins to prolong the survival of Sp2/0-Ag14 (ATCC # CRL-1581, hereafter referred to as Sp2/0) cells in cultures; however, the cell density of the Bcl-2 expressing clones were 20 to 50% lower than that of their parental cultures, raising concerns for their practical application in biopharmaceutical industry (see WO03/040374). It is apparent, therefore, that improved host cells for high level expression of recombinant proteins and methods for reliably increasing recombinant protein production, in particular the production of antibodies and antibody fragments, multispecific antibodies, fragments and single-chain constructs, peptides, enzymes, growth factors, hormones, interleukins, interferons, and vaccines, in host cells are greatly to be desired.

US 6,964,199 is related to methods and compositions for providing enhanced growth of, and/or production from, cultured mammalian host cells used for the production of commercially useful amounts of expressed proteins, by the use of at least one Bcl2 encoding nucleic acid provided or transcribed in the host cell.

SUMMARY OF THE INVENTION

Accordingly, it is an object of the present invention to provide improved host cells and methods to increase the longevity and recombinant protein yields of a cell culture by introducing into the cells agents that inhibit senescence or that promote cell survival, e.g., anti-apoptotic agents. The use of such agents preferentially increases the lifespan and viability of cells in culture used for the production of a desired recombinant protein, concomitantly increasing the productivity of such cells in culture, and thereby the optimal yield of the desired protein.

The problem underlying the present invention is solved by the subject matter of the attached independent claims. Preferred embodiments may be taken from the attached dependent claims.

More specifically, the problem underlying the present invention is solved by a method of making a recombinant protein, comprising culturing a host cell in a suitable serum-free medium under conditions suitable for expression of said recombinant protein, wherein said host cell is a myeloma Sp2/0 cell comprising a nucleic acid sequence encoding said recombinant protein, wherein said host cell has been transfected with an expression vector encoding the Bcl-2 triple mutant T69E, S70E and S87 E and wherein said host cell, upon transfection with the expression vector encoding the Bcl-2 triple mutant, is capable of expressing the recombinant protein.

In an embodiment of the first aspect, said mutant Bcl-2 protein is encoded by a nucleic acid sequence integrated in the chromosomal DNA of said host cell.

In an embodiment of the first aspect, said medium further comprises at least one caspase inhibitor.

In an embodiment of the first aspect, said caspase inhibitor is selected from the group consisting of caspase-1, caspase-3, caspase-9, caspase-12 and pan-caspase inhibitors.

In an embodiment of the first aspect, said inhibitor is selected from the group consisting of Z-VAD-fink, Ac-DEVD-cho, Aven and XIAP.

In an embodiment of the first aspect, said medium further comprises an exogenously added agent that inhibits apoptosis and/or functions as a cytoprotective agent.

In an embodiment of the first aspect, said exogenous agent is a member of the cytokine type I superfamily.

In an embodiment of the first aspect, said member of the cytokine type I superfamily is erythropoietin.

In an embodiment of the first aspect, said recombinant protein is selected from the group consisting of immunoglobulins, peptides, enzymes, growth factors, hormones, vaccines, lymphokines and cytokines.

In an embodiment of the first aspect, said recombinant protein is an immunoglobulin selected from the group consisting of antibodies, antibody fragments, multispecific antibodies, and single chain antibodies.

In an embodiment of the first aspect, said protein is a growth factor selected from the group consisting of erythropoietin, G-CSF, GM-CSF, EGF, VEGF, and thrombopoietin.

In an embodiment of the first aspect, said protein is a lymphokine selected from the group consisting of any of IL-1 to IL-31, alpha interferon, beta interferon, gamma interferon and consensus interferon.

More specifically, the problem underlying the present invention is solved in a second aspect by a host cell transfected with an expression vector encoding the Bcl-2 triple mutant T69E, S70E and S87E and wherein said host cell is a myeloma Sp2/0 cell comprising a nucleic acid sequence encoding a recombinant protein of interest and which is capable of expressing the recombinant protein in serum-free medium.

In an embodiment of the second aspect, said recombinant protein is selected from the group consisting of immunoglobulins, peptides, enzymes, growth factors, hormones, vaccines, lymphokines and cytokines.

In an embodiment of the first aspect, the modification of said host cell results in an increase in cell culture longevity.

In an embodiment of the first aspect, wherein said host cell is cultured in a perfusion reactor or said host cell is cultured in suspension, resulting in an increase in cell culture longevity of at least 2 days.

In an embodiment of the first aspect, said host cell is cultured in a perfusion reactor, said host cell is cultured in a fed-batch culture or said host cell is cultured in suspension, resulting in an increase in cell culture longevity of at least 4 days.

In an embodiment of the first aspect, said host cell is cultured in a perfusion reactor, said host cell is cultured in a fed-batch culture or said host cell is cultured in suspension, resulting in an increase in cell culture longevity of at least 6 days.

Alternately, the longevity and recombinant protein yields of a cell clone can be improved by introducing into the cell agents that down-regulate the level of intracellular pro-apoptotic proteins, such as p53 and Rb, or up-regulate intracellular anti-apoptotic proteins, such as Bcl-2. The regulatory agents may include, but are not limited to, human papillomavirus type 16 (HPV-16) oncoproteins E6 and E7, and combinations thereof. Additionally, caspase inhibitors, as described herein, may also contribute to blocking or reducing apoptosis, thus increasing cell survival and increasing the production of recombinant proteins by said cells in culture. A further class of anti-apoptotic agents that can be used in these cultures to enhance production of recombinant proteins includes certain members of the cytokine type I superfamily, such as erythropoietin (EPO). EPO, as a prototype molecule of this class, is a major modifier of apoptosis of multiple cell types, not just erythrocytes, and thus has more general cytoprotective functions, such as in endothelial cells, myocardial cells, tubular epithelial cells of the kidney, skin, and neurons [cf. review by P. Ghezzi and M. Brines, Cell Death and Differentiation 11 (suppl. 1), s37-s44, July 2004].

Also disclosed are cell culture methods incorporating novel combinations of factors including, but not limited to, transfection vectors, screening and selection of cell clones with desired properties, cell culture media, growth conditions, bioreactor configurations, and cell types to create cell culture conditions in which the longevity of the cell culture is increased and/or made optimal and the yield of a desired recombinant protein is increased. These cell culture methods include suspension, perfusion, and fed-batch methods of production. See Tey et al., J. Biotechnol. 79: 147-159 (2000); Zhang et al., J. Chem. Technol. Biotechnol. 79: 171-181 (2004); Zhou et al., Biotechnol. Bioeng. 55: 783-792 (1997).

Unless otherwise defined, all technical and scientific terms used in the invention have the same meaning as commonly understood by one of ordinary skill in the art.

BRIEF DESCRIPTION OF DRAWINGS/FIGURES

Figure 1 shows visual images of Sp2/0 and Sp-E26 cells treated with cycloheximide (+ CHX) or untreated (- CHX).

Figure 2 shows the results of screening HPV E6/E7 transduced cells that are more resistant to CHX treatment. A total of 55 clones were screened; in the first experiment, 31 clones were screened (top panel); in the second experiment, 24 clones were screened (bottom panel). Healthy cells of each clone were split into two equal portions. One was treated with CHX for 2 h and the other left untreated. The viable cells in these two cultures were then measured by MTT assay and the ratios of viable cell populations treated ($CHX^+$) vs. untreated ($CHX^-$) were plotted. As shown in the top panel, CHX treatment resulted in 30% reduction of viability in Sp2/0 cells, while only 6% reduction in Sp-E26 cells. Seven of the 31 clones screened (indicated by *) performed significantly better (<20% reduction of viability) than Sp2/0 but not as well as Sp-E26. For the 24 clones screened in the second experiment (bottom panel), CHX treatment resulted in ~50% reduction of viability in Sp2/0 cells and <20% reduction of viability in Sp-E26. Ten of the 24 clones (indicated by * or **) screened performed significantly better (<30% reduction) than Sp2/0, and 6 of them (indicated by **) matched or were better than Sp-E26 (<20%). E28 and E36 are two additional control clones that perform better than Sp2/0 but not as well as Sp-E26.

Figure 3 shows the dot plots of Guava Nexin V assay. The percentage of early apoptotic cells (Nexin V-positive and 7-AAD-negative) is indicated in the lower-right quadrant.

Figure 4 shows the DNA fragmentation in Sp2/0 treated by CHX. In contrast, Sp-E26 cells are resistant to the treatment.

Figure 5 shows the growth profiles of Sp2/0 and Sp-E26 cells in T-flasks. Healthy cells (>95% viability) were seeded in T-flasks at an initial cell density of 200,000/ml. Viable and dead cells were counted daily using Guava ViaCount reagent (Guava technologies, Inc.) and PCA instrumentation (Guava Technologies, Inc.). Accumulation of $NH_4^+$ and lactate also was monitored.

Figure 6 compares the growth profiles of Sp2/0 and Sp-E26 cells as determined for a batch culture in 3-L bioreactors. Healthy cells (>95% viability) were seeded in the bioreactors at an initial cell density of 250,000/ml. Cells were counted daily by trypan blue and microscope.

Figure 7 shows a representative immunoblot stained with Bcl-2 (100) antibody (Santa Cruz Biotech.) and developed with enhanced chemiluminescence for screening of clones for Bcl-2-EEE expression.

Figure 8 shows a graph of flow cytometry results using Guava Express. Cells were fixed and permeabilized before staining with phycoerythrin-conjugated anti-Bcl-2 antibody (Santa Cruz Biotechnology, Inc.) Several sub-clones are compared.

Figure 9 shows a graph of flow cytometry results using Guava Express. Cells were fixed and permeabilized before staining with phycoerythrin conjugated anti-Bcl-2 antibody (Santa Cruz Biotechnology, Inc.). Sp2/0, Raji and Daudi cells were compared to Bcl-2-EEE clones.

Figure 10 shows the results of immunoblot analyses of 665.B4.1C1, Sp2/0, Raji, Daudi, Sp-EEE (87-29 clone) and Sp-EEE (7-16 clone) cell lysates. A. Blots stained with a human Bcl-2 specific antibody (Santa Cruz Biotechnology, Inc). B. Blot stained with an anti-Bcl-2 antibody (Santa Cruz Biotechnology, Inc) that recognizes mouse and human Bcl-2.

Figure 11 shows growth curves (A) and viability (B) of Sp-EEE clones compared to Sp2/0 cells grown in media supplemented with 10% fetal bovine serum.

Figure 12 shows growth curves (A) and viability (B) of Sp-EEE clones compared to Sp2/0 cells grown in media supplemented with 1% fetal bovine serum.

Figure 13 shows growth curves (A) and viability (B) of Sp-EEE clones compared to Sp2/0 cells grown in serum-free media.

Figure 14 shows methotrexate kill curves for Sp-EEE (87-29 clone) cells.

Figure 15 shows a graph of flow cytometry results using Guava Express comparing Sp-EEE clones grown in the presence or absence of 1mg/ml zeocin. Cells were fixed and permeabilized before staining with phycoerythrin conjugated anti-Bcl-2 antibody (Santa Cruz Biotechnology, Inc).

Figure 16 shows the map of the pdHL2 vector used to transfect Sp2/0 cells to obtain the 665.2B9 clone with humanized antibody sequences and the SV40 promoter and enhancer sequences.

Figure 17 shows the map of DNA plasmid with incorporated *Bcl-2* gene, used for transfection of clone 665.2B9

Figures 18 and 19 show the growth profiles of Bcl-2 transfected clones 665.2B9#4, Bcl-2 negative clones and untransfected control. Healthy cells (>95% viability) were seeded in 24-well plates at an initial cell density of 400,000/ml. Viable and dead cells were counted daily using Guava ViaCount reagent and PCA instrumentation.

Figures 20 and 21 show growth profiles of Bcl-2 transfected clone 665.259 #4 and Bcl-2 negative clones in different MTX concentration. Healthy cells (>95% viability) were seeded in T-flasks at initial cell density of 100,000/ml. Viable cell density and viability were counted daily using Guava ViaCount reagent and PCA instrumentation.

Figure 22 shows the levels of human Bcl-2 expressed by clone 665.2B9#4 in increasing concentrations of MTX and clone #13 detected by Western blotting.

Figures 23 and 24 show the profiles of cell viability and viable cell density, respectively, of clone 665.2B9#4 cultured in 0.6 and 1 $\mu$M of MTX and the Bcl-2-negative clone #13 cultured in 0.3 $\mu$M MTX with or without spiking L-glutamine and glucose. Healthy cells (>95% viability) were seeded in roller bottles at an initial cell density of 200,000/ml. On day 2 and 4 (arrows indicated), a nutrient supplement solution containing glucose and L-glutamine was added to the "spiked" culture. Viable and dead cells were counted daily using Guava ViaCount reagent and PCA instrumentation.

Figure 25 shows the process schematics for bioeractor feeding strategies.

Figure 26 shows the growth curves (VCD and the viability) of 665.2B9.1E4 and 665.B4.1C1 cell lines by Process #1, which does not feed recombinant insulin, and Process #2, which is based on Process #1 with a modified linoleic acid and lipid feeding schedule and an additional feeding of insulin.

Figure 27 shows the antibody yields of 665.2B9.1E4 and 665.B4.1C1 cell lines in Processes #1 and #2. The final yield of 665.2B9.1E4 cells was 0.42 g/L in Process #1 and 0.55 g/L in Process #2. For comparison, 665.B4.1C1 cells delivered a higher final yield of 1.5 g/L in both processes.

Figure 28 shows the daily specific antibody productivities (per cell basis). As shown in the figure, the 665.2B9.1E4 cells had an average daily $Q_{[MAb]}$ of approximately 15 pg/cell/day throughout the course of cultivation for both processes. The additional day of grown at the highest VCD in Process #2 resulted in a higher final antibody concentration.

## DETAILED DESCRIPTION OF THE INVENTION

The present ivention as defined in the claims provides improved host cells and methods for enhanced production of recombinant proteins in such cell lines. Cell lines have been created that constitutively express one or more anti-apoptotic genes and that can be transfected with an expression construct encoding a protein or peptide of interest, where expression of the anti-apoptotic gene(s) prolongs survival of the transfected cell in culture and provides for enhanced yields of the protein or peptide of interest.

Specifically, the present inventors have created from Sp2/0 myeloma cell line two novel cell lines, referred to as Sp-E26 and Sp-EEE, which show enhanced survival in batch culture. Sp-E26 and Sp-EEE constitutively express the E6 and E7 proteins of HPV-16 and a Bcl-2 mutant, referred to as Bcl-2-EEE, respectively. In addition, recombinant protein production, and particularly production of recombinant antibodies and antibody fragments, can be improved upon transfecting either Sp-E26 or Sp-EEE with an expression vector for the recombinant protein of interest. The E6/E7 or Bcl-2-EEE proteins delay induction of apoptosis in the host cells and permit enhanced recombinant protein production in the host cells. Protein production can be boosted still further by addition of one or more caspase inhibitors (e.g., caspase 1 and/or 3 inhibitors) (Bin Yang et al. Nephron Experimental Nephrology 2004;96:e39-e51), and/or by addition of one or more members of the cytokine type I superfamily, such as erythropoietin (EPO), into the growth medium of the cells. A pan-caspase inhibitor is particularly effective in this regard.

The present inventors also have found that production of recombinant proteins, such as antibodies or antibody fragments, can be significantly enhanced in the host cell by co-expression of an apoptosis inhibitor, such as Bcl-2. In particular, protein production is significantly enhanced in a myeloma cell line, such as Sp2/0, that is stably transfected with an expression vector encoding an antibody or antibody fragment and that is co-transfected with an expression vector encoding an apoptosis inhibitor, such as Bcl-2. Increased production of antibody can also be obtained from a host cell transfected with the E6/E7 gene. Recombinant protein production can be boosted still further by addition of one or more caspase inhibitors into the growth medium of the cells. A pan-caspase inhibitor is particularly effective in this regard. Also, recombinant protein production can be enhanced by feeding EPO, or another anti-apoptotic cytokine, into the medium of the cell culture.

Physiological, or programmed, cell death, referred to as apoptosis (Kerr et al., Br J Cancer., 26:239-257, 1972) is essential for proper tissue development and maintenance and is controlled by an intrinsic genetic program that has been conserved in evolution (Ellis et al., Annu Rev Cell Biol, 7, 663-698, 1991). Hence, when cells grow in artificial environments, such as *ex vivo* cultures, this genetic endowment results in a finite lifespan. Therefore, the utility of such cell cultures for the production of proteins used in medicine and industry, as well as research, is dependent on maintaining such cultures for extended lifespan, or cycles, before they die according to apoptotic mechanisms.

Methods and agents have been discovered that act independently on cell proliferation and cell death events, by differentiating cell cycle from apoptotic effects. Bcl-2, a well-known intracellular regulator of apoptosis (Vaux et al., Nature 335, 440-2, 1988), is a proto-oncogene that has been found to have an anti-apototic effect that is genetically different from its inhibitory influence on cell cycle entry (Huang et al., EMBO J 16, 4628-38, 1997). Two homologues of Bcl-2, Bcl-$x_L$ and Bcl-w, also extend cell survival, but other members of the Bcl-2 family, such as Bax and Bak, are pro-apoptotic (Oltvai et al., Cell 74, 609-19, 1993; Chittenden et al., Nature 374, 733-6, 1995; Farrow et al., Nature 374, 731-3, 1995; Kiefer et al., Nature 374, 736-9, 1995). Other anti-apoptotic genes include *Bcl-6* and *Mcl-1.*

Thus, Bcl-2 and certain of its family members exert protection against apoptosis, and it was therefore hypothesized as a method to increase the lifespan of certain host cells in culture that are used for the production of proteins, thereby enhancing the amount of proteins produced and isolated. Since antibodies are produced by B-lymphocytes, particularly by myeloma cells, over-expression of an anti-apoptotic Bcl-2 family member, such as Bcl-2, Bcl-$x_L$, Bcl-w or mutant varieties of these proteins, inhibits apoptosis, resulting in increased cell density and longer culture survival. Hence, transfection of anti-apoptotic Bcl-2 family genes avoids the necessity to prolong the cell culture by interfering with the cell cycle per se, as others have proposed (*ibid.*). Similarly, transfection of fibroblasts with genes for Bcl-2 results in over-expression of Bcl-2 in these cells, resulting again in an antagonism of apoptosis and increasing the lifespan of these cells, with a concomitant increase in the production and isolation of recombinant proteins. It has also been observed that upon cytokine withdrawal, interleukin-6 (IL-6)-dependent murine myeloma cells expire as if they undergo apoptosis. It was also found that IL-6-receptors in such cells could be regulated by Bcl-2 or Bcl-xL in extending apoptosis (Schwarz et al., Cancer Res 55:2262-5, 1995).

Recent literature has also demonstrated that a mutant Bcl-2 possessing three point mutations (T69E, S70E and S87E) exhibited significantly more anti-apoptotic activity compared to wild type or single point mutants (Deng et al., PNAS (101) 153 -158, 2004). Thus, it is taught the construction of an expression vector for a Bcl-2-EEE triple mutant, which was then used to transfect Sp2/0 cells to create Sp-EEE clones and subclones that show improved longevity and recombinant protein production.

Other agents, such as oncogenic viruses, can also oppose apoptosis as part of their eliciting cellular immortalization and ultimately complete malignant transformation, such as high-risk type HPV oncoproteins E6 and E7 (Finzer et al., Cancer Lett 188, 15-24, 2002). For example, the viral E6 protein effectively blocks the epidermal apoptotic response to ultraviolet light (Storey, Trends Mol Med 8, 417-21, 2002). It has also been suggested, from indirect evidence, that the human papillomavirus may cause reduced apoptosis in squamous (but not basal cell) carcinoma (Jackson et al., Br J Cancer 87, 319-23, 2002). However, not all papillomavirus oncoproteins have anti-apoptotic effect. For example, other studies have reported that the papillomavirus E6 protein of bovine species sensitizes cells to apoptosis (Liu et al., Virology 295, 230-7, 2002), which is in contrast to other studies showing that HPV-16 E7 gene protects astrocytes against apoptosis induced by certain stimuli (Lee et al., Yonsei Med J 42, 471-9, 2001). By use of E6-binding peptide aptamers, direct experimental evidence was obtained that HPV E6 oncoprotein has anti-apoptotic activity in HPV-positive tumor cells (Butz et al, Proc Natl Acad Sci USA 97, 6693-7, 2000). However, other HPV oncoproteins can have the opposite effect; the E2 protein induces apoptosis in the absence of other HPV proteins (Webster et al., J Biol Chem 275, 87-94, 2000). Continuous expression of both the E6 and E7 proteins is known to be required for optimal proliferation of cervical cancer cells and that the two viral proteins exert distinct effects on cell survival (DeFilippis et al., J Virol 77, 1551-63, 2003). The primary intracellular target attributed to HPV-16 E6 is p53. E6 forms a ternary complex with p53 and a cellular ubiquitin ligase, E6AP, resulting in the ubiquitination and degradation of p53 through the proteosome pathway and inactivation of p53. On the other hand, HPV-16 E7 protein interacts and destabilizes the tumor suppressor protein Rb. Moreover, levels of a variety of other intracellular proteins involved in apoptosis and cell cycle pathways were reported to be regulated by E6 and E7 transformation, such as Bcl-2, Bcl-xL, p73, MDM2, p21, cyclins and cdc, cdk proteins, etc. Changes in the expression of these proteins will greatly influence the physiological properties of the cell. The present inventors therefore hypothesized that transfection of cells in culture by HPV-16 E6 and E7 would be very effective in generating genetically modified clones that are resistant to aging-culture-condition induced apoptosis and, therefore, prolong the lifespan of the cell culture. It was also postulated that introduction into a cell of either HPV-16 oncoprotein E7 or E6 alone might be sufficient to generate genetically modified clones with improved resistance to aging-culture-condition induced apoptosis. When the cell is a recombinant protein-producing clone, the improved physiological properties would in turn translate into enhanced overall protein productivity.

Generation of New Host Cells Expressing Viral Anti-apoptotic Genes

Host cells, such as myeloma host cells, can be generated that constitutively express viral anti-apoptotic genes, such as HPV-16 E6 and E7 proteins. These host cells can be transfected with an expression vector that encodes a recombinant protein of interest and co-expression of the anti-apoptotic genes results in significantly increased production of the recombinant protein.

The host cell can be essentially any host cell suitable for recombinant protein production that can be stably transformed with the viral anti-apoptosis genes. For many recombinant proteins, host cells such as CHO and COS cells are advantageous, while for other proteins, such as antibodies, host cells such as myeloma cells and CHO cells are the common choices. The viral genes can be introduced into the host cell by any suitable method that results in constitutive or inducible expression of the genes, *i.e.,* any method that permits stable integration of the genes into the host cell chromosome while permitting expression of the genes. Methods for stable transformation of host cells with a gene of interest are well known in the art. A particularly advantageous method is to use a retroviral vector that encodes the viral anti-apoptosis genes. Suitable vectors include the LSXN vector (Miller et al. Biotechniques 7, 980-90, 1989).

Advantageously, the vector used to transfect the host cell contains a selectable marker that permits selection of cells containing the vector. Suitable selection markers, such as enzymes that confer antibiotic resistance on transfected cells, are well known in the art. After transfection, cells are maintained in a medium containing the selection agent, such as an antibiotic, and screened for resistance to the marker. Cells can be selected and cloned by limiting dilution using conventional methods.

The ability of the viral anti-apoptosis genes to increase cell viability can be tested by challenging the cells with an agent that induces apoptosis, such as cycloheximide (CHX). Cells that do not express the viral anti-apoptosis genes tend to demonstrate significant onset of apoptosis, whereas cells expressing the genes exhibit drastically reduced apoptotic activity. Methods of detecting apoptosis are well known in the art and include, for example, cell surface FITC-Annexin V binding assay, DNA laddering assay and TUNEL assay.

Upon selection of suitable cells expressing the viral anti-apoptosis genes, the cells can be transfected with an expression vector encoding the recombinant protein of choice. The expression vector can be a vector suitable for transient expression or, advantageously, can be an episomal vector containing a eukaryotic origin of replication, or an amplifiable vector that permits stable integration and subsequent gene amplification of the expression cassette. Suitable vectors are well known in the art and include, for example, the pdHL2 vector, which is particularly suited for production of antibodies and antibody fragments. When an amplifiable expression cassette is used, it advantageously contains a selectable marker that is different from the selectable marker used in the retroviral vector, to allow selection of transfected cells. Once again, suitably transfected cells can be selected and then cloned by limiting dilution.

Upon selection of suitable clones, the cells can be placed in a suitable medium and cultured to produce the desired protein of interest. The medium can contain serum or, preferably, be serum-free. In addition, cell longevity and protein production also can be increased by adding one or more caspase inhibitors (e.g., caspase 1 or 3) to the culture medium. Preferably the caspase inhibitor acts to inhibit one or more of caspase 3, caspase 9 and/or caspase 12. A cell-penetrating caspase inhibitor advantageously is used, and a pan-caspase inhibitor is particularly advantageous. Suitable inhibitors such as Z-VAD-fmk and Ac-DEVD-cho are well known in the art. Alternatively, the cell line can be further transfected to express a caspase inhibitor, such as Aven or XIAP, to enhance its growth properties by affecting apoptosis. In this regard, certain members of the cytokine type I superfamily, such as EPO, can also increase cell survival by having anti-apoptotic and cytoprotective actions. The methods described above generate a cell line that can be used for transfection with essentially any desired gene. However, the skilled artisan will recognize that established cell lines that constitutively express a desired protein, and particularly a recombinant protein, can be subsequently transformed with a suitable vector encoding the viral or Bcl-2 family anti-apoptosis genes. See Example 2 below.

The protein of interest can be essentially any protein that can be produced in detectable quantities in the host cell. Examples include traditional IgG type antibodies, $F(ab')_2$ or Fab fragments, scFv, diabody, IgG-scFv or Fab-scFv fusion antibodies, IgG- or Fab-peptide toxin fusion proteins, or vaccines [e.g., including not limited to, Hepatitis A, B or C; HIV, influenza viruses, respiratory syncytial virus, papilloma viruses, Herpes viruses, Hantaan virus, Ebola viruses, Rota virus, Cytomegalovirus, Leishmania RNA viruses, SARS, malaria, tuberculosis (Mycobacteria), Anthrax, Smallpox, Tularemia, and others listed in www.vaccines.org,]. The host cells described herein are particularly suitable for highly efficient production of antibodies and antibody fragments in myeloma cell lines as described in Examples 1 and 2, as well as recombinant growth factors (e.g., EPO, G-CSF, GM-CSF, EGF, VEGF, thrombopoietin), hormones, interleukins (e.g., IL-1 through IL-31), interferons (e.g., alpha, beta, gamma, and consensus), and enzymes. These methods could be applied to any number of cell lines that are used for production of recombinant proteins, including other myeloma cell lines, such as murine NSO or rat YB2/0; epithelial lines, such as CHO and HEK 293; mesenchymal cell lines, such as fibroblast lines COS-1 or COS-7; and neuronal cells, such as retinal cells, as well as glial and glioma cells.

Recombinant Antibody Expression in Cells Expressing Apoptosis Inhibitors

Prior work has described the effects of co-expressing Bcl-2, a naturally occurring apoptosis inhibitor, in recombinant CHO cells producing a chimeric antibody. See Tey et al., Biotechnol. Bioeng. 68:31-43 (2000). Although increased cell culture life was observed, antibody production did not increase over equivalent cells that lacked Bcl-2 expression. However, the present inventors have found that production of recombinant antibody from myeloma cells is significantly increased when the cells also express Bcl-2.

Advantageously, the myeloma cell line is stably transfected with an expression cassette encoding the antibody or antibody fragment. A suitable expression cassette contains one or more promoters that controls expression of the antibody heavy and light chains (of single chain in the case of an scFv) together with a selectable marker as described above. A particularly useful vector is pdHL2, which contains a selectable marker gene comprising a promoter operatively linked to a DNA sequence encoding a selectable marker enzyme; a transcription unit having a promoter operatively linked to a DNA sequence encoding the protein of interest; an enhancer element between the selectable marker gene and the transcription unit, which stimulates transcription of both the selectable marker gene and the first transcription unit compared to the transcription of both the selectable marker gene and the first transcription unit in the absence of the first enhancer. The vector also contains a blocking element having a promoter placed between the first enhancer and the selectable marker gene, which selectively attenuates the stimulation of transcription of the selectable marker gene. $V_H$ and $V_L$ sequences can be ligated into pdHL2, which is an amplifiable vector containing sequences for the human light chain constant region, the heavy chain constant region, and an amplifiable *dhfr* gene, each controlled by separate promoters. See Leung et al., Tumor Targeting 2:184, (1996) and Losman et al., Cancer 80:2660-2667, (1997). This vector can be transfected into cells by, for example, electroporation. Selection can be performed by the addition of 0.1 μM or a suitable concentration of methotrexate (MTX) into the culture media. Amplification can be carried out in a stepwise fashion with increasing concentration of MTX, up to 3 μM or higher. Cells stably transfected with the expression cassette and that constitutively express the antibody of interest can therefore be obtained and characterized using methods that are well known in the art. See also Example 4, below. After selection and cloning, the antibody-expressing cell line can then be transfected with an expression vector that encodes an anti-apoptosis gene, such as Bcl-2. For example, the vector pZeoSV (Invitrogen, Carlsbad, CA) containing the *Bcl-2* gene fused to an SV40 promoter is transfected into the cell using a suitable method such as electroporation, and selection and gene amplification can be carried out if necessary. Antibody production using the resulting cell line can be carried out as above and compared to production in cells that do not express an apoptosis inhibitor.

The methods describe initial preparation of a cell line expressing an antibody or antibody fragment that is subsequently transfected with a vector expressing Bcl-2 or a similar inhibitor. However, the skilled artisan will recognize that cell lines can be established that constitutively express Bcl-2 or another anti-apoptotic protein, which can be subsequently transformed with a suitable vector encoding the antibody or antibody fragment. Representative examples to illustrate the present invention are given below. Example 1 describes the incorporation of HPV-16 E6/E7 into Sp2/0 cell leads to an improved cell clone, Sp-E26, showing characteristics of reduced/delayed apoptosis. Example 2 describes a method to improve host cell lines by over-expression of the HPV-16 E7 element alone. Example 3 describes using the improved cell, Sp-E26, as a host to develop cell clones producing a recombinant Ab. Example 4 describes the enhanced production of Mab observed for an antibody-producing cell line that co-expresses the E6/E7 element. Example 5 describes the generation and characterization of a modified Sp2/0 cell line that constitutively expresses a mutant Bcl-2 (Bcl-2-EEE) possessing three point mutations, resulting in improved longevity. Example 6 describes the improved growth properties of an antibody-producing cell line that expresses Bcl-2. Example 7 describes the enhanced production of MAb observed for the Bcl-2 expressing cell line of Example 6. Example 8 describes the methods to improve a cell clone producing low-level recombinant protein by introduction of Bcl-2 expression in the cell. Example 9 describes the methods to improve Sp-E26 by introduction of Bcl-2 expression in the cell. Example 10 describes using the improved cell line, Sp-EEE, as a host to develop cell clones producing a recombinant Ab. Example 11 describes the use of fed-batch reactor profiles and feeding schedules to optimize yield.

**Example 1. Generation of apoptosis-resistance cell clones by stable expression of HPV-16 E6 and E7 genes.**

Selection of cell clones resistant to CHX treatment

Sp2/0 cells were transduced with an LXSN retroviral vector containing the expression cassette of HPV-16 E6 and E7 genes at an MOI (multiple of infection) of 10:1. After recovery for 24 h, the infected cells were selected in G418 (1000 μg/ml) for 10 days. G418-resistant cells were cloned in 96-well cell culture plates by limiting dilution (0.5 cells/well). Stable infectants were screened for resistance to treatment by cycloheximide (CHX), a potent apoptosis-inducing agent. Briefly, healthy cells (viability >95%, Figure 1C and D) were incubated in medium containing 25 μg/ml of CHX and cell morphology was examined under a microscope. While more than 50% of parent Sp2/0 cells underwent morphology change after two to three hours of incubation and became fragmented (Figure 1A), several E6/E7 transfected clones showed less extent of morphology change, indicating resistance to apoptosis. The best clone, designated as Sp-E26, showed no apparent morphology change upon four hours of treatment (Figure 1B).

To avoid tedious visual examination, MTT assay was used to access the changes in viable cell population. After the healthy cells were incubated with or without CHX under normal culture condition for 2-3 h, MTT dye was added to the wells. After further incubation for two hours, the cells were solubilized by adding a lysis buffer contain SDS and HCl. The plates were incubated overnight at 37°C and OD reading was performed at 590 nm using an ELISA plate reader. As shown in Figure 2, the viable cell population was significantly reduced when Sp2/0 cells were treated with CHX. By comparison, under the same treatment conditions (concentration of CHX and length of time), Sp-E26 cells tolerated better against CHX treatment. With this method, a large number of clones can be screened and selected for further analyses (Figure 2).

<u>Anti-apoptosis property of Sp-E26.</u>

CHX-induced apoptosis in Sp-E26 and the parent Sp2/0 cells was evaluated by Annexin V staining and DNA fragmentation assay. After being incubated in the medium containing 25 μg/ml of CHX, the cells were harvested and stained with Guava Nexin reagent (equivalent of Annexin V staining) and analyzed in a Guava Personal Cell Analysis system (Guava Technologies, Inc.). Figure 3 shows that while more than 30% of Sp2/0 cells became Annexin V positive when exposed to CHX treatment for about 1.5 h, indication of apoptosis, Sp-E26 remained healthy, showing no increase in early apoptotic cells.

The induction of apoptosis by CHX can be revealed by analysis of the formation of intracellular oligonucleosomal DNA fragments, a hallmark of apoptosis. The cellular DNA was extracted from CHX-treated and untreated Sp-E26 and Sp2/0 cells and DNA laddering assay was performed. In Sp2/0 cells treated with CHX, extensive DNA fragmentation was detected (Figure 4). In contrast, under identical treatment conditions, the genomic DNA of Sp-E26 was still intact, showing no appearance of DNA fragmentation (Figure 4).

<u>Presence of HPV E6 and E7genes in Sp-E26</u>

To confirm that E6 and E7 genes are stably present in the genome of Sp-E26 cells, oligonucleotide primers specific for E6 and E7 genes were designed and used in a PCR reaction with the genomic DNA extracted from Sp-E26 as the template, resulting in a ~700 bp DNA fragment. The PCR product was cloned and confirmed to be E6 and E7 genes by DNA sequencing. No E6 and E7 genes were detected in the parent Sp2/0 cells.

<u>Improved growth properties of Sp-E26</u>

The growth properties of Sp-E26 were evaluated in T-flask (Figure 5) and $^{3L-}$batch bioreactor (Figure 6). Sp-E26 showed improved growth properties over the parent Sp2/0 cell in batch cultures, achieving higher maximum cell density and longer survival time.

**Example 2. Generation of apoptosis-resistance cell clones by stable over-expression of HPV16 E7 gene.**

The structure of the poly-cistronic HPV16 E6 and E7 genes integrated into the genome of clone Sp-E26 was analyzed by PCR using the primer pair E6-N8$^+$ (5'-ATG TTT CAG GAC CCA CAG GAG CGA-3') and E7-C8$^-$ (5'-TTA TGG TTT CTG AGA ACA GAT GGG-3') and DNA sequencing. Since the sequences of primer E6-N8$^+$ and E7-C8- match with the coding sequence for the N-terminal 8 amino acid residues of E6 and the complement sequence for the C-terminal 8 codons of E7, respectively, the amplicon of full-length E6 and E7 is expected to be ~850 bp. However, amplification of the genomic DNA prepared from Sp-E26 cell with E6-N8$^+$ and E7-C8- resulted a PCR fragment of only ~700 bp. DNA sequencing of the 700 bp PCR product revealed a deletion of a 182 poly-nucleotide fragment from the E6 gene. The defective E6 gene is likely resulted from splicing and encodes a truncated E6 peptide with N-terminal 43 amino acid residues. Considering the major physiological activity attributed to E6 is its ability to down-regulate p53 expression, the truncated E6 protein is probably not fully functional because the level of p53 expression in Sp-E26 was found to be more stable than that in Sp2/0.

Thus, to evaluate whether HPV-16 E7 gene alone is sufficient to have anti-apoptotic effect and to improve the growth properties of Sp2/0 cells, transfection of Sp2/0 cell with HPV-16 E7 is performed as follows:

(i) The DNA sequence encoding E7 is cloned from Sp-E26 cell by RT-PCR. Proper restriction sites are introduced to facilitate the ligation of the gene into a mammalian expression vector, pRc/CMV (Invitrogen). Transcription of the viral gene within the vector, designated as E7pRc, is directed from CMV promoter-enhancer sequences. The vector also contains a gene conferring neomycin resistance, which is transcribed from the SV40 promoter.

(ii) Sp2/0 cells are transfected with the expression vector containing the expression cassette of HPV-16 E7 gene. Briefly, 5 ug of E7pRc is linearized by ScaI and transfected into the cell by electroporation.

(iii) After recovery for 24 hours, the transfected cells are selected in G418 (1000 μg/ml) for 10 days.

(iv) G418-resistant cells are then cloned in 96-well cell culture plates by limiting dilution (0.5 cells/well). Stable transfectants are selected and screened for resistance to treatment by cycloheximide (CHX), a potent apoptosis-inducing agent.

(v) Healthy cells (viability >95%) are incubated in medium containing 25 μg/ml of CHX or in the absence of CHX for 3-4 hours under normal culture conditions, followed by the addition of MTT dye into the wells. After further incubation for two hours, the cells are solubilized by adding a lysis buffer contain SDS and HC1. The plates are incubated overnight at 37 °C and an OD reading is performed at 590 nm using an ELISA plate reader. Cell clones showing resistance to CHX treatment are selected and expanded for further analyses.

(vi) The anti-apoptosis property of E7-transfected cells is evaluated by Annexin V staining and DNA fragmentation assays. In the Annexin V assay, after being incubated in the medium containing 25 μg/ml of CHX, the cells are harvested and stained with Guava Nexin reagent (equivalent of Annexin V staining) and analyzed in a Guava Personal Cell Analysis system (Guava Technologies, Inc.). In the DNA fragmentation assay, the cellular DNA is extracted from CHX-treated and untreated E7-transfectants and Sp2/0 cells and analyzed with agarose gel electrophoresis.

(vii) Expression of the viral oncogene in E7-transfectants is evaluated by Southern blot (genomic level), Northern blot (mRNA level), and immunoblot (protein level) analysis. Expression of intracellular proteins that are involved in apoptosis processes and affected by E7 protein are examined by immunoblotting analyses.

(viii) The growth properties of selected E7-transfectants are evaluated in T-flask and in a 3L-batch bioreactor. The transfectants show improved growth properties, i.e. achieving higher maximum cell density and longer survival time, over the parent Sp2/0 cell in batch cultures are considered to be better host cells.

**Example 3. High-level expression of hLL2 IgG in Sp-E26.**

In this example, Sp-E26 is used as a host to generate cell clones producing hLL2, a humanized anti-CD22 Ab developed for treating patients with NHL and autoimmune diseases. An hLL2-producing clone, 87-2-C9, was previously generated by using Sp2/0 cell as a host (Losman et al., Cancer 80, 2660-2666, 1997), in which case, only one positive clone (a frequency of ~$2.5 \times 10^{-7}$) was identified after transfection, and the maximum productivity ($P_{max}$), defined as the concentration of the antibody in conditioned terminal culture medium in T-flask, of the only hLL2-producing clone, before amplification, was 1.4 mg/L. Transfection of Sp-E26 cell with the same hLL2pdHL2 vector and by using similar procedures as described by Losman et al. (Cancer 80, 2660-2666, 1997) resulted in more than 200 stable hLL2-producing clones, a frequency of >$10^{-4}$). The $P_{max}$ of 12 randomly selected clones was evaluated and found to be between 13 and 170 mg/L, with a mean of 50 mg/L. The productivities of these clones can be further enhanced by gene amplification with MTX. This example demonstrated the advantage of using Sp-E26 over its parent Sp2/0 cell as a host for the development of cell clones producing recombinant proteins.

**Example 4. Improvement of Ab-producing cell lines by stable expression of HPV16 E6 and E7 genes.**

607-3u-8 cells were originally generated from Sp2/0 by transfection to produce a humanized monoclonal Ab. The clone was developed by gene amplification (with MTX) and subcloning to enhance the maximum (Ab) productivity up to 150 mg/L, which decreased to ~100 mg/L following weaning off serum supplement in the culture medium.. To obtain higher antibody productivity under serum-free condition, E6/E7 genes of HPV-16 were introduced into 607-3u-8 and the effect of E6/E7 on Ab-productivity was evaluated as follows. 607-3u-8 cells maintained in HSFM supplemented with 10% FBS and 3 uM MTX were transduced with an LXSN retroviral vector containing the expression cassette of HPV-16 E6 and E7 genes at an MOI of 10:1. After recovery for 24 h, stably transfected cells were selected in G418 (400 μg/ml) for 10 days. G418-resistant cells were subcloned in 96-well cell culture plates by limiting dilution (0.5 cells/well). A surviving clone, designated as 607E1C12, was obtained for evaluation. Two subclones, designated as 607-3u-8-7G7 and 607-3u-8-2D10, of 607-3u-8 without E6/E7 transfection were also selected. The $P_{max}$ of these three clones were determined and there were no significant difference (Table 1). These results suggest that introducing E6/E7 genes into the cell does not alter the ability of cells producing Ab. Next, 607E1C12, 607-3u-8-7G7 and 607-3u-8-2D10 were adapted to grow in serum-free medium and the productivities of these clones were determined. All cells were growing well in serum-free medium. The final antibody productivity of clone 607E1C12 was maintained at 150 mg/L, while the two clones without E6/E7 were substantially reduced. In addition, the productivity of 607E1C12 were stable after a freeze (for cryopreservation) and thaw cycle (Table 1)

**Table 1.** The productivities of Ab-producing clones

| Clone | $P_{max}$ (mg/L)[a] | |
|---|---|---|
| | With serum | Serum-free |
| 607-3u-8-7G7 | 127 ± 16 (3)[b] | 74 ± 10 (4) |
| 607-3u-8-2D10 | 140 ± 4 (3) | 35 ± 2 (2) |
| 607E1C12 | 154 (1) | 142 ± 13 (6) |
| 607E1C12 (Cryo)[c] | | 145 ± 17 (5) |

a. Determined by protein purification of IgG from terminal culture supernatants.
b. The number in parenthesis indicates the sample size.
c. Cells had been frozen for cryopreservation.

**Example 5. Generation and Characterization of a genetically modified Sp2/0 cell line that Constitutively Expresses a Mutant Bcl-2**

Evidence suggests that a mutant Bcl-2 possessing three point mutations (T69E, S70E and S87E) exhibits significantly more anti-apoptotic activity compared to wild type or single point mutants (Deng et al., PNAS 101: 153 -158, 2004). Thus, an expression vector for this triple mutant (designated as Bcl-2-EEE) was constructed and used to transfect Sp2/0 cells for increased survival and productivity, particularly in bioreactors. Clones were isolated and evaluated for Bcl-2-EEE expression level, growth and apoptotic properties. The nucleic acid sequence for the Bcl-2-EEE is depicted as SEQ. ID. No. 3; the corresponding amino acid sequence for the Bcl-2-EEE protein is depicted as SEQ. ID. No. 4.

A 116 bp synthetic DNA duplex was designed based on the coding sequence for amino acid residues 64 -101 of human Bcl-2. The codons for residues 69, 70 and 87 were all changed to those for glutamic acid (E). The entire sequence was extraordinarily GC rich and had numerous poly G and poly C runs. Conservative changes were made to several codons to break up the G and C runs and decrease the overall GC content.

Two 80-mer oligonucleotides were synthesized that, combined, span the 116 bp sequence and overlap on their 3' ends with 22 bp (See SEQ. ID. No. 5 & 6). The oligonucleotides were annealed and duplex DNA was generated by primer extension with Taq DNA polymerase. The duplex was amplified using the PCR primers, Bcl-2-EEE PCR Left (5'-TATATGGACCCGGTCGCCAGAGAAG-3'), and Bcl-2-EEE PCR Right (5'-TTAATCGCCGGCCTGGCGGAGGGTC-3').

The 126 bp amplimer was then cloned into pGemT PCR cloning vector. The Bcl-2-EEE-pGemT construct was digested with TthI and NgoMI restriction endonucleases and the 105 bp fragment was gel isolated and ligated with hBcl-2-pucl9 vector (ATCC 79804) that was digested with TthI and NgoMI to generate hBcl-2(EEE)-puc19. The sequence of this construct was confirmed.

A 948 bp insert fragment was excised from hBcl-2 (EEE)-puc19 with EcoRI and ligated with pZeoSV2+ vector that was digested with EcoRI and treated with alkaline phosphatase. The resulting construct is hBcl-2 (EEE)-pZeoSV2+.

Sp2/0 cells (5.6 x $10^6$) were then transfected by electroporation with 60 μg of hBcl-2 (EEE)-pZeoSV2+ following the standard protocol for Sp2/0 cells. The cells were plated into six 96-well plates that were incubated without selection for 48 hours. After two days, 800 μg/ml of zeocin was added to the media.
Cells from 40 wells were expanded to 24-well plates and analyzed by western blot with anti-hBcl-2 and anti-β actin. All but 5 of the 40 showed medium to high levels of Bcl-2-EEE expression. The results for one of four gels are shown in Figure 7. An Sp2/0 derived hMN14 cell line (Clone 664.B4) that was previously transfected with wild type Bcl-2 was used as a positive control (+). As was demonstrated by Deng *et al.,* the Bcl-2-EEE migrates slightly slower than wild type Bcl-2 in SDS-PAGE.

Three strongly positive wells (#7, #25 and #87) were chosen for further evaluation and sub-cloning. Limiting dilution plating resulted in <20 positive wells per 96-well plate, indicating a very high probability (>99%) that the cells in individual wells are in fact cloned. Initially, 23 subclones from the three original wells were analyzed by Guava Express using anti-hBcl-2-PE (Figure 8). The results confirmed that the original wells contained mixed cell clones. Well #7 yielded clones with the strongest signal and well #25 had those with the lowest. Clones 7-12, 7-16, 87-2 and 87-10 were expanded for further analysis. Subsequently, some initially slower growing subclones were similarly analyzed and one clone, 87-29, gave a signal that was 20% higher than any other clone and was expanded for further analysis. Two high expressing SP-EEE clones (87-29 and 7-16) were compared to the untransfected Sp2/0, Raji and Daudi cells (Figure 9). The Sp-EEE clones expresses about 20-fold higher than Raji and Daudi cells, which are both known to express Bcl-2 at presumably normal cell levels. Sp2/0 cells were negative. This was further verified by anti-Bcl-2 immunoblot (Figure 10). Bcl-2 was not detected with a human Bcl-2 specific antibody in Sp2/0 cells even with high protein loading (50K cells) and long exposure of X-ray film. Immunoblot analysis with an anti-Bcl-2 MAb (C-2, Santa Cruz Biotech.) that recognizes mouse, rat and human Bcl-2 did not detect any Bcl-2 from untransfected Sp2/0 cells, even with high protein loading (100K cells) and long exposure time (Fig. 10B). If there is any Bcl-2 expressed in Sp2/0 cells, it is at a level that is more than 2 orders of magnitude less than the Bcl-2-EEE in clone 87-29. Growth curves were compared for five Sp-EEE subclones and Sp2/0 cells. Three Sp-EEE subclones displayed a clear advantage over Sp2/0 cells. These three (7-12, 7-16 and 87-29) also express the highest levels of Bcl-2-EEE. As 7-12 and 7-16 are from the same original well and have nearly identical properties (Bcl-2-EEE levels and growth curves), they likely originated from the same initial clone. The best two SP-EEE subclones 7-16 and 87-29 were used for further evaluation.

The clones were plated in media supplemented with 10%, 1% or 0% serum (without weaning) and cell density and viability were monitored. In 10% serum 87-29 grew to a high density and had more than 4 days increased survival compared to Sp2/0 cells (Figure 11). In 1% serum, all cells grew to about 35 - 40% of the density achieved in 10% serum and the Bcl-2-EEE transfectants had a similar survival advantage over Sp2/0 (Figure 12). When transferred directly into serum free media, the Sp2/0 cells only grew to 600K cells/ml while 87-29 cells grew to a two-fold higher density (Figure 13). In each serum concentration 87-29 cells survived 4 - 6 days longer than Sp2/0 cells.

The methotrexate (MTX) sensitivity was determined for 87-29 (Figure 14). The data suggests that a minimum MTX concentration of 0.04 μM is sufficient for initial selection of MTX-resistant clones. Therefore, the same selection and amplification protocols used for Sp2/0 cells can be employed with the SP-EEE cells.

Bcl-2 is a pro-survival/anti-apoptotic protein. It has been demonstrated by several groups that a Bcl-2 deletion mutant missing the flexible loop domain (FLD) has an enhanced ability to inhibit apoptotosis (Figueroa et al., 2001, Biotechnology and Bioengineering, 73, 211-222; Chang et al., 1997, EMBO J.,16, 968-977). More recently, it was demonstrated that mutation of 1 to 3 S/T residues in the FLD of Bcl-2 to glutamic acid, which mimics phosphorylation, significantly enhances its anti-apoptotic ability (Deng et al. 2004, PNAS, 101, 153-158). The triple mutant (T69E, S70E and S87E) provided the most significant survival enhancement. Here, the present invention teaches the generation of a similar Bcl-2 triple mutant construct (Bcl-2-EEE), which is used to stably transfect Sp2/0 cells.

All the aforementioned experiments demonstrate that expression of Bcl-2-EEE reduces apoptosis rates in Sp2/0 cells. This effect was largely dose dependent, in that clones with higher expression levels survived longer than those with lower levels. The best clone, 87-29, grows to a 15 - 20% higher cell density and survives an additional 4-6 days compared to untransfected Sp2/0 cells.

The Bcl-2-EEE level in clone (87-29) is approximately 20-fold higher than normal levels in Daudi or Raji cells. No Bcl-2 expression was detected in untransfected Sp2/0 cells. As described in Example 6, hMN-14-expressing Sp2/0 cells were transfected with a similar construct for expression of wild type Bcl-2 and a clone with exceptional growth properties and enhanced productivity was isolated. When this clone (664.B4) was amplified further with MTX, the Bcl-2 levels increased significantly. Ultimately, the amplified (3 μM MTX) cell line was sub-cloned and the Bcl-2 level of one clone (664.B4.1C1) was two-fold higher than 664.B4. This particular subclone has superior productivity and growth properties. The Bcl-2-EEE level in 87-29 is approximately two-fold higher than the level of Bcl-2 in the amplified 664.B4.1C1. 87-29 cells have a growth rate that is comparable to that of Sp2/0 cells and can apparently continue to grow for one additional day and reach a maximal density that is 15 - 20% higher than Sp2/0. A similar property was found for the E6/E7 expressing Sp-E26 cell line. The Bcl-2-EEE expressing 87-29 clone, which provides an additional 4-6 days survival over the parental Sp2/0 cells, is superior to the Sp-E26 clone, which only survives one additional day.

The Sp-EEE cell line as represented by the 87-29 clone is useful as an apoptosis-resistant host for expressing a recombinant protein upon transfection with a suitable vector containing the gene for that recombinant protein. In order for this cell line to be useful it must maintain its Bcl-2-EEE expression and survival advantage following transfection and amplification and during extended culture. It is unlikely that the stably transfected Bcl-2-EEE gene will be lost during subsequent transfection and therefore the survival properties should not diminish. It is possible that MTX amplification could even improve the survival of producing clones via increasing expression of Bcl-2 proteins. Indeed, this was the case with the hMN-14 664.B4 cell line, which was transfected with wild type Bcl-2. Following amplification and sub-cloning, the Bcl-2 level increased several fold and cell survival improved significantly.

### Example 6. Improvement of Ab-producing cell survival in stationary batch culture by stable expression of a human *Bcl-2* gene.

Generation of a Bcl-2-transfected cell clone

A cell clone 665.2B9 was originally generated from Sp2/0 by transfection to produce a humanized monoclonal anti-CEA Ab (Qu *et al.,* unpublished results). A vector, designated hMN14pdHL2, was used to transfect Sp2/0 cells to obtain the cell clone 665.2B9. The pdHL2 vector was first described by Gillies *et al.,* and had an amplifiable murine *dhfr* gene that allows subsequent selection and amplification by methotrexate treatment (Gillies et al., J. Immunol. Methods 125:191 (1989)). Generally, the pdHL2 vector provides expression of both IgG heavy and light chain genes that are independently controlled by two metallothionine promoters and IgH enhancers. A diagram of the hMN14pdHL2 vector is shown in Figure 16. SEQ. ID. No. 1 shows the sequence of the vector; SEQ. ID. No. 2 shows the 72 bp sequence defined as the enhancer sequence; the promoter sequence corresponds to nt2908-2979 of hMN14pdHL2.

Sp2/0 cells can be generally transfected by electroporation with linearized pdHL2 vectors such as the hMN14pdHL2 vector used in this instance. Selection can be initiated 48 hours after transfection by incubating cells with medium containing 0.05 to 0.1 μM MTX. Amplification of inserted antibody sequences is achieved by a stepwise increase in MTX concentration up to 5 μM.

The clone was subjected to gene amplification with MTX increased stepwise to 0.3 μM, at which point the maximum productivity (Pmax) of the antibody was increased to about 100 mg/L. To improve cell growth properties, 665.2B9 cells were transfected with a plasmid expression vector (Figure 17) containing the human *Bcl-2* gene by electroporation. *Bcl-2* gene was excised from pB4 plasmid purchased from ATCC (pB4, catalog # 79804) using EcoRI sites and inserted into MCS of mammalian expression vector pZeoSV(+) using the same restriction enzyme. Since zeocin resistance gene is part of the vector, transfected cells were placed into medium containing zeocin ranging from 50-300 μg/mL. Stable clones were selected from media containing 300 mg/ml zeocin and subcloned in media without zeocin by plating into 96-well plates at a density of 0.5 cell/100 uL/well. The media without zeocin was used thereafter. Formation of clones in wells was confirmed by visual observation under a microscope. Cells from the wells with only 1 cluster of cells were expanded. Each 96-well plate produced around 30 clones, from which 14 clones were randomly selected for further studies. The growth characteristics of these clones were evaluated by daily cell counting and viability measurements with ViaCount reagent and Guava PCA. From the 14 clones evaluated in 24-well plates (Figures 18, 19), one Bcl-2-transfected clone showing improved growth characteristics (higher cell densities and prolonged cell survival) was identified and designated as 665.2B9#4 (or clone #4). Comparing to the parent 665.2B9 clone, clone #4 grew to a higher cell density (~1.7-fold) and survived 4 to 6 days longer in T-flasks (Figures 20, 21), and as a consequence of better growth, the $P_{max}$ of clone #4 was increased to about 170 mg/L as determined by ELISA titration and Protein A column purification.

<u>Bcl-2 expression in 665.2B9#4</u>

To confirm that the improved growth properties of 665.2B9#4 were resulted from transfection of Bcl-2, intracellular level of human Bcl-2 protein was measured by using Guava Express reagent and Guava PCA instrument. Briefly, 4 x $10^5$ cells placed in 1.5ml spin-tubes were centrifuged for 5 minutes at 1500 rpm, washed three times with 1x PBS. Supernatants were carefully aspirated. Fixation solution (10x, 60 μL) from Santa Cruz Biotechnology (SCB), Inc. (cat. # sc- 3622) was added to cell pellets for 15 min and incubated on ice. Fixation solution was removed with 4x 1 mL PBS at 4°C, each time spinning as described. Permeabilization buffer (0.5 mL) at -20°C (SCB cat. # sc- 3623) was added dropwise while vortexing, followed by 15 min incubation on ice. Cells were then spun and washed two times with 0.5 mL FCM wash buffer (SCB cat. # sc- 3624). Final cell pellet was resuspended in 100 μL of FCM wash buffer and stained for Bcl-2 intracellular protein with 10 μL of anti-Bcl-2 mouse monoclonal antibody conjugated to PE (obtained from SCB). Incubation was performed at room temperature in dark for one hour. Two washes with 0.5 mL of FCM wash buffer followed. The final cell pellet was resuspended with 0.4 mL FCM wash buffer and the cells analyzed on Guava PC. Mean values of the fluorescence intensity (MFI) for each clone were compared to control staining with non-specific, isotype mouse IgG1 conjugated with PE. The results summarized in Table 2 confirm that clone 665.2B9#4 expresses a higher level of Bcl-2 protein compared to the parental cell line. A zeocin-resistant clone (#13) that showed a similar growth profile as the parent 665.2B9 was negative for Bcl-2 staining, confirming that Bcl-2 expression is necessary for the improvement of growth.

**Table 2.** Intracellular level of Bcl-2 determined by Guava Express.

| Cell | Viability[a] (%) | Mean FI (AU) |
|---|---|---|
| 665.2B9 | 84 | 42 |
| 665.2B9#4 | 97 | 110 |
| Clone #13 | 92 | 14 |
| Non-specific antibody staining | | 12 |

a. Determined before the assay to ensure healthy cells were used.
b. 665.2B9 cells stained with an isotype-matched mouse IgG1antibody, PE-conjugated.

With Guava Express analysis it was found that the intensities of fluorescent staining corresponding to Bcl-2 levels are rising with MTX amplification of clone 665.2B9#4, suggesting co-amplification of *Bcl-2* with the dhfr gene. To compare intracellular Bcl-2 levels of amplified cells, Western blotting analysis was performed on cell lysates of clone 665.2B9#4 (Bcl-2 positive) and clone #13 (Bcl-2 negative) using an anti-human Bcl-2 antibody. Densitometric evaluation showed that Bcl-2 signal of clone 665.2B9#4 growing in 1.0 μM MTX is 2x stronger than the cells in 0.6 μM MTX. A lysate of Clone #13 did not reveal the presence of Bcl-2 protein (Figure 22).

**Example 7. Improved Ab-production of clone 665.2B9#4 under batch culture condition.**

By monitoring nutrients consumption in the cell cultures near the terminal phase, it was found that glucose and L-glutamine are the first components to be consumed. Experiments were carried out to determine whether supplementation of these limiting nutrients would improve the final antibody yields. Two types of cultures were initiated: spiked fed batch - where these limiting components were supplemented upon their consumption; and unfed batch - without nutrients supplementation. Tested were Bcl-2-positive clone 665.2B9#4 growing in medium containing 0.6 and 1 μM of MTX and the Bcl-2-negative clone #13 growing in 0.3 μM MTX. Figures 23 and 24 show the profiles of cell viability and cell density in both culture types until they reached terminal stage. Protein yields, expressed as mg/L, are shown in Table 3. The results of this experiment suggest that nutrient spiking improves total yield of produced antibody about 2-fold for all cultures.

**Table 3. Antibody production under batch culture conditions**

| Cell/MTX (μM) | Unfed batch[a] (mg/L) | Spiked fed batch[a] (mg/L) |
|---|---|---|
| 665.2B9#4/0.6 | 117 | 286 |
| 665.2B9#4/1.0 | 156[b] | 296 |
| Clone#13/0.3 | 74.1 | 165 |

a. Determined by Protein A column purification.
b. Average of two purifications.

**Example 8. Introduction of *Bcl-2* gene into a cell line producing low-level of recombinant protein.**

A cell clone 482.2C4A was originally generated from Sp2/0 by transfection to produce a bispecific Ab in the form of an IgG (anti-CEA) and two scFv (anti-DTPA) (Leung et al., J. Nuc. Med. 41: 270P, 2000; Hayes et al., Proc. Am. Asso. Cancer. Res. 43: 969, 2002), each of which is covalently linked to the C-terminus of the IgG heavy chain. The clone was subjected to gene amplification and had a final productivity of ~20 mg/L. To improve the growth property and eventually the Ab productivity, 482.2C4A cells were transfected with a plasmid expression vector containing the human Bcl-2 gene by electroporation as described in Example 6. The transfectants were selected in medium containing 750 μg/ml of Zeocin after three weeks.

Zeocin-resistant cells were treated with 25 μg/ml of CHX for 5 hours to eliminate apoptosis-sensitive cells. Treated cells were washed twice with fresh culture medium to remove CHX and resuspended in fresh growth medium. After recovering for 24 h, the viable cells were cloned into 96-well cell culture plates by limiting dilution (0.5 cells/well). Clones emerged in the wells in two weeks and were screened for Ab production, resistance to CHX-induced apoptosis, as well as growth profiles. Those clones performed better than the parent 482.2C4A in all aspects are selected and further characterized. The best performer is expected to be more robust when growing under stress condition, resist to aging-culture-condition induced apoptosis, and have a higher maximum Ab productivity (ca.150% or better) comparing to the parent 482.2C4A cell.

**Example 9. Introduction of *Bcl-2* gene into Sp-E26 for a further improvement of cell growth properties.**

Sp-E26 cells are transfected with a plasmid expression vector containing the human Bcl-2-EEE gene, as described in Example 5, by electroporation. The transfectants are selected in medium containing 500 μg/ml of Zeocin after three weeks.

Zeocin-resistant cells are treated with 25 μg/ml of CHX for 5 hours to eliminate apoptosis-sensitive cells. Treated cells are washed twice with fresh culture medium to remove CHX and resuspended in fresh growth medium. After recovering for 24 h, the viable cells are cloned into 96-well cell culture plates by limiting dilution (0.5 cells/well). Clones emerge in the wells in two weeks and are screened for resistance to CHX-induced apoptosis, as well as growth profiles. Those clones perform better than the parent Sp-E26, as well as Sp-EEE, in all aspects are selected and further characterized. The best performer containing HPV-16 E6/E7 and Bcl-2-EEE is expected to be more robust when growing under stress condition and resistant to aging-culture-condition-induced apoptosis than the parent Sp-E26 and Sp/EEE cells; therefore, it is a better host cell for recombinant protein production.

**Example 10. Improved production of recombinant proteins with the Sp-EEE cell line.**

There are two paths that can be taken when developing a cell line with enhanced survival for production of recombinant proteins. One method, which has been accomplished quite successfully, as described in Example 6, involves stable transfection of an already producing cell line with a pro-survival gene, such as *Bcl-2.* However, this method requires additional transfection, selection and cloning steps, thereby lengthening the cell line development process by at least two months and possibly much more. Further, screening for the "best" clone is rather involved, since a number of parameters need to be determined for each clone, including growth/survival, Bcl-2 expression level and productivity. Thus, only a small number of clones can be evaluated. It is quite possible that clones with the highest productivity may not have superior survival and vice versa. An alternative strategy, employed here, is to develop a parental cell line with superior growth and survival properties, which is subsequently transfected with the expression vector for production of the desired protein.

Compared to Sp2/0 cells, the Sp-EEE cells continue to grow for one additional day, reach a maximal density that is 15 - 20% higher, and survive an additional 4-6 days in culture. The cells retain their enhanced growth and survival properties when subsequently transfected with genes for the production of recombinant proteins, such as IgG, antibody fragments and fusion proteins, growth factors, such as G-CSF, GM-CFS, EPO, EGF, VEGF, cytokines, such as an interleukin family member (IL-1- IL-31), or interferon family members (such as alpha, beta or gamma interferon), oligonucleotides, peptides, hormones, enzymes, or vaccines (e.g., Hepatitis A, B or C, as well as others described above).

A DNA vector, such as pdHL2, containing one or more expression cassettes for recombinant protein(s), such as an IgG, is used to transfect Sp-EEE cells by standard methods, such as electroporation. The transfectants are plated in 96-well plates and clones are analyzed for protein production by established techniques such as ELISA or Biacore. Productive clones are subjected to increasing concentrations of MTX in the culture media over several months to amplify the genetic copy number. Since the Bcl-2-EEE-expressing clones grow to ~20% higher cell density and survive at least an additional 4 days as compared to clones generated in Bcl-2 negative Sp2/0 cells, the former will produce at least 20% more recombinant protein in standard flask or roller bottle culture. An even greater increase is realized in suspension, perfusion or fed-batch bioreactor cultures.

**Example 11. Improved Ab-production of Bcl-2 transfected clone 665.B4.1C1 in bioreactor**

Both 665.2B9#4 and the parent clone 665.2B9 of Example 6 were weaned into serum-free media. The cells were adapted to a customized formulation of hybridoma serum-free medium (HSFM) (Immunomedics PN 10070) containing 3 μM MTX by continuous subculture in T-flasks for several months. The adapted cells were scaled up from T-flasks to roller bottles for banking. A master cell bank (MCB) for each cell line was created with $1 \times 10^7$ viable cells in each 1-mL vial using an FBS-free cryopreservation solution composed of 45% conditioned medium (medium that is collected as supernatant after centrifugation of a culture in the exponential growth phase), 10% DMSO and 45% HSFM. The MCB cell lines were designated 665.2B9.1E4 (without *Bcl-2* gene) and 665.B4.1C1 (with Bcl-2 gene), respectively. The growth properties and antibody production of these two clones were compared under batch culture conditions.

Experiments were conducted in 3-L bench-scale bioreactors using the above cells expanded from the MCB. The 3-L bioreactor system is the scale-down model of a 2500-L cGMP bioreactor system. Therefore, the evaluation results would support the suitability of these cell lines for large-scale commercial manufacturing.

The same growth HSFM as that used in creating the MCB (Immunomedics PN 10070) was used to maintain the cell line and prepare the inoculum. Basal HSFM, a customized formulation based on the growth HSFM with customized modifications (Immunomedics PN 10194), was used in the 3-L fed-batch bioreactor process. Both media contain insulin and transferrin as the only trace proteins. Additional 0.1% Pluronic F68 was incorporated into the formulation to protect cells from shear caused by agitation and aeration. This media also contained 3 μM MTX.

The specific characteristics of the continuous feeding solutions and the pulse feeding solutions are shown in tables 4 and 5 as follows:

**Table 4:** Continuous feeding solutions

| Solutions | **Formulation (Dissolve in WFI unless specified)** |
|---|---|
| Glucose and glutamine solution (G/G) | Glucose, 13.3g/L; Glutamine, 20mM |
| ImmuC2 solution | Glucose, 13.3g/L; Glutamine, 20mM; PNS A, 50ml/L; NaOH, 50mM |
| ImmuC5 solution | Glucose, 26.6g/L; Glutamine, 40mM; PNS A, 100ml/L; NaOH, 100mM |

**Table 5:** Pulse feeding Solutions

| Solutions | **Formulation (Dissolve in WFI unless specified)** |
|---|---|
| TC Soy Plus | 120g/L |
| Linoleic acid/cyclodextrin | 1.5mg/ml |
| HD lipid | 500X |
| β-mercaptoethanol/EDTA | BME, 0.01M; EDTA, 0.1mM. |
| ImmuVitamin | MEM Vitamin Solution (100x), as solvent; Choline Chloride, 500mg/L; Myo-inositol, 600mg/L. |
| TEC solution | Transferrin solution (4mg/mL), as solvent; CaC12, 125mM; Ethanolamine-HCl 1g/L. |
| Insulin | 4mg/ml |

The fed-batch experiments were conducted in 3L Bellco spinner-flask bioreactor systems (Bellco glasses, Vineland, NJ) with 2 L of working volume. The bioreactor temperature, pH and dissolved oxygen (DO) were monitored and controlled by single loop controllers. The reactor temperature was controlled at 37°C by a heating blanket. The culture pH was controlled at 7.3 by the addition of $CO_2$ or 6% $Na_2CO_3$. Aeration was performed through a cylindrical sintered sparger at 10ml/min. DO was controlled above 40% of air saturation by intermittent sparging of $O_2$ into the medium. A constant agitation rate of 50 ~ 60rpm was used throughout the cultivation.

A frozen vial from MCB was thawed and recovered in T-flasks in approximately 1 to 2 weeks. The cells were then expanded from T-flasks to roller bottles prior to inoculation into the bioreactors. Cells were cultured at 37°C in a 5% $CO_2$ atmosphere and maintained in the exponential growth phase throughout the expansion process.

Prior to the inoculation, 1.2 liters of Basal HSFM was pump-transferred into the bioreactor aseptically. The medium was air saturated to calibrate the dissolved oxygen (DO) probe. A medium sample was also taken to calibrate the pH probe. Once pH probes and DO probes were calibrated, both controllers were set to AUTO modes. Once the system reached set points of pH (7.3) and temperature (37°C), calculated amount of inoculum from roller bottle was pump transferred into the bioreactor. The post-inoculation viable cell density (VCD) was around $2\times10^5$ vial cells/ml.

The feeding strategy is as follows. During the cultivation, concentrated nutrient solutions were fed into the bioreactor to provide the cells with necessary and non-excessive nutrients (See figure 25 for the overall process schematics). Concentrated nutrient solutions were delivered to the culture via continuous feeding and pulse feeding. The continuous feeding solutions were pump transferred into the reactor continuously using peristaltic pumps (Watson-Marlow 101U/R). The pulse feeding solutions were pulse fed once a day into the culture.

Two fed-batch feeding strategies were developed and applied to both cell lines. Process #1 does not feed recombinant insulin during the cultivation. Process #2 is designed based on Process #1 with a modified linoleic acid and lipid feeding schedule and an additional feeding of insulin.

The following tables summarize the feedings of both processes for both cell lines.

**Table 6:** Process #1 for cell line 665.2B9.1E4

| **Continuous feeding** | | | | |
|---|---|---|---|---|
| **Day** | **Expected Viable Cell Density (cells/mL)** | **Continuous Feeding Rate (ml/day)** | | |
| | | Glucose and Glutamine | ImmuC2 | ImmuC5 |
| Day2 | 0.4~0.7 E6 | 60 | 0 | 0 |
| Day3am | 1.0~1.7E6 | 0 | 60 | 0 |
| Day3pm | 1.01~2.5E6 | 0 | 90 | 0 |
| Day4am | 2.51~3.5E6 | 0 | 90 | 0 |
| Day4pm | 2.51~4.5E6 | 0 | 150 | 0 |
| Day5am | 4.51~6.5E6 | 0 | 0 | 90 |
| Day5pm | 4.51~7.5E6 | 0 | 0 | 120 |
| Day6 | 7.51~12E6 | 0 | 0 | 120 |

(continued)

| Continuous feeding | | | | |
|---|---|---|---|---|
| **Day** | **Expected Viable Cell Density (cells/mL)** | **Continuous Feeding Rate (ml/day)** | | |
| | | Glucose and Glutamine | ImmuC2 | ImmuC5 |
| Day7 | if<13E6 | 0 | 0 | 120 |
| | if>13.1E6 | 0 | 0 | 150 |

**Pulse feeding**

| **Day** | **Pulse Feeding (mL)** | | | | | |
|---|---|---|---|---|---|---|
| | TC Soy Plus (120g/L) | LA/CD (1.5mg/ml) | Lipid (500X) | TEC Solution | Immu Vitamin | BME/EDTA |
| Day3 | 12.5 | 4 | 3 | - | - | 15 |
| Day4 | 25 | 8 | - | - | - | - |
| Day5 | 50 | 12 | 12-4 | 4 | 15 | 15 |
| Day6 | 60 | 8 | 2 | 8 | - | - |
| Day7 | 60 | - | 1 | - | - | - |
| Day8 | 25 | | | | | |

**Table 7:** Process #2 for cell line 665.2B9.1E4

| Continuous feeding | | | | |
|---|---|---|---|---|
| **Day** | **Expected Viable Cell Density (cells/mL)** | **Continuous Feeding Rate (ml/day)** | | |
| | | Glucose and Glutamine | ImmuC2 | ImmuC5 |
| Day2 | 0.4~0.7 E6 | 60 | 0 | 0 |
| Day3am | 1.0~1.7E6 | 0 | 60 | 0 |
| Day3pm | 1.01~2.5E6 | 0 | 90 | 0 |
| Day4am | 2.51~3.5E6 | 0 | 90 | 0 |
| Day4pm | 2.51~4.5E6 | 0 | 150 | 0 |
| Day5am | 4.51~6.5E6 | 0 | 0 | 90 |
| Day5pm | 4.51~7.5E6 | 0 | 0 | 120 |
| Day6 | 7.51~12E6 | 0 | 0 | 120 |
| Day7 | if<13E6 | 0 | 0 | 120 |
| | if>13.1E6 | 0 | 0 | 150 |
| Day8 | if<10E6 | 0 | 0 | 90 |
| | if 10.1~13E6 | 0 | 0 | 120 |
| | if>13.1E6 | 0 | 0 | 150 |

**Pulse feeding**

| **Day** | **Pulse Feeding (mL)** | | | | | | |
|---|---|---|---|---|---|---|---|
| | TC Soy Plus (120g/L) | LA/CD (1.5mg/ml) | Lipid (500X) | TEC Solution | Immu Vitamin | BME /EDTA | Insulin (4mg/ml) |
| Day3 | 12.5 | 2 | 3 | - | - | 15 | - |

(continued)

| Pulse feeding | | | | | | | |
|---|---|---|---|---|---|---|---|
| **Day** | **Pulse Feeding (mL)** | | | | | | |
| | TC Soy Plus (120g/L) | LA/CD (1.5mg/ml) | Lipid (500X) | TEC Solution | Immu Vitamin | BME /EDTA | Insulin (4mg/ml) |
| Day4 | 25 | 4 | - | - | - | - | - |
| Day5 | 50 | 6 | - | 4 | 15 | 15 | 4 |
| Day6 | 60 | 4 | - | 8 | - | - | 8 |
| Day7 | 60 | 4 | - | - | - | 15 | 8 |
| Day8 | 50 | - | - | - | - | - | 8 |

**Table 8:** Process #1 for cell line 665.B4.1C1

| Continuous feeding | | | | |
|---|---|---|---|---|
| **Day** | **Expected Viable Cell Density (cells/mL)** | **Continuous Feeding Rate (ml/day)** | | |
| | | Glucose and Glutamine | ImmuC2 | ImmuC5 |
| Day2 | 0.4~0.7E6 | 60 | 0 | 0 |
| Day3am | 1.0~1.7E6 | 0 | 90 | 0 |
| Day3pm | 1.01~2.5E6 | 0 | 120 | 0 |
| Day4am | 2.51~3.5E6 | 0 | 0 | 60 |
| Day4pm | 2.51~4.5E6 | 0 | 0 | 90 |
| Day5am | 4.51~6.5E6 | 0 | 0 | 120 |
| Day5pm | 4.51~7.5E6 | 0 | 0 | 150 |
| Day6 | 7.51~12E6 | 0 | 0 | 180 |
| Day7, 8, 9 | if<15E6 | 0 | 0 | 180 |
| | if>15.1E6 | 0 | 0 | 240 |
| Day10 | if<10E6 | 0 | 0 | 120 |
| | if 10.1~13E6 | 0 | 0 | 150 |
| | if>13.1E6 | 0 | 0 | 180 |

| Pulse feeding | | | | | | |
|---|---|---|---|---|---|---|
| **Day** | **Pulse Feeding (mL)** | | | | | |
| | TC Soy Plus (120g/L) | LA/CD (1.5mg/ml) | Lipid (500X) | TEC Solution | Immu Vitamin | BME/EDTA |
| Day3 | 12.5 | 4 | 3 | - | - | 15 |
| Day4 | 25 | 8 | - | - | - | - |
| Day5 | 50 | 12 | 12-4 | 4 | 15 | 15 |
| Day6 | 60 | 8 | 2 | 8 | - | - |
| Day7 | 60 | - | 1 | - | - | 15 |
| Day8 | 60 | - | - | - | - | - |
| Day9 | 60 | - | - | - | - | 15 |

(continued)

| Pulse feeding | | | | | | |
|---|---|---|---|---|---|---|
| **Day** | **Pulse Feeding (mL)** | | | | | |
| | TC Soy Plus (120g/L) | LA/CD (1.5mg/ml) | Lipid (500X) | TEC Solution | Immu Vitamin | BME/EDTA |
| Day10 | 50 | - | - | - | - | - |

**Table 9:** Process #2 for cell line 665.B4.1C1

| Continuous feeding | | | | |
|---|---|---|---|---|
| **Day** | **Expected Viable Cell Density (cells/mL)** | **Continuous Feeding Rate (ml/day)** | | |
| | | Glucose and Glutamine | ImmuC2 | ImmuC5 |
| Day2 | 0.4~0.7 E6 | 60 | 0 | 0 |
| Day3am | 1.0~1.7E6 | 0 | 90 | 0 |
| Day3pm | 1.01~2.5E6 | 0 | 120 | 0 |
| Day4am | 2.51~3.5E6 | 0 | 0 | 60 |
| Day4pm | 2.51~4.5E6 | 0 | 0 | 90 |
| Day5am | 4.51~6.SE6 | 0 | 0 | 120 |
| Day5pm | 4.51~7.5E6 | 0 | 0 | 150 |
| Day6 | 7.51~12E6 | 0 | 0 | 180 |
| Day7, 8, 9 | if<15E6 | 0 | 0 | 180 |
| | if>15.1E6 | 0 | 0 | 240 |
| Day10 | if<10E6 | 0 | 0 | 120 |
| | if 10.1~13E6 | 0 | 0 | 150 |
| | if>13.1E6 | 0 | 0 | 180 |

| Pulse feeding | | | | | | | |
|---|---|---|---|---|---|---|---|
| **Day** | **Pulse Feeding (mL)** | | | | | | |
| | TC Soy Plus (120g/L) | LA/CD (1.5mg/ml) | Lipid (500X) | TEC Solution | Immu Vitamin | BME /EDTA | Insulin (4mg/ml) |
| Day3 | 12.5 | 2 | 3 | - | - | 15 | - |
| Day4 | 25 | 4 | - | - | - | - | - |
| Day5 | 50 | 6 | - | 4 | 15 | 15 | 4 |
| Day6 | 60 | 4 | - | 8 | - | - | 8 |
| Day7 | 60 | 4 | - | - | - | 15 | 8 |
| Day8 | 60 | 4 | - | - | - | - | 8 |
| Day9 | 60 | 4 | - | 4 | 15 | 15 | 8 |
| Day10 | 60 | - | - | - | - | 1- | 8 |

During the cultivation, bioreactor samples were taken periodically for off-line analysis. The viable cell density (VCD) and the cell viability were measured by microscopic counting using a hemocytometer after staining with 0.4% trypan blue dye. The glucose, lactate, glutamine, ammonia concentrations were measured using a Nova Bioprofile 200. The antibody concentration was determined by HPLC using a protein A affinity chromatography column (Applied Biosystems, P/N 2-1001-00).

The specific antibody productivity was calculated by dividing the cumulative antibody produced by the time integral of the total viable cell in the culture:

$$Q_{[MAb]} = \frac{([Mab]_{t1} \bullet V_{t1} - [Mab]_{t0} \bullet V_{t0}}{\int_0^{t1} VCD \bullet Vdt},$$

in which $\int_0^{t1} VCD \bullet Vdt$ is approximated by the Trapezium Rule : $\frac{(VCDt0 \bullet Vt0 + VCDt1 \bullet Vt1)(t1 - t0)}{2}$

Figure 26 shows the growth curves (VCD and the viability) of both cell lines by Process #1 and Process #2. By Process #1, 665.2B9.1E4 cells grew to attain a maximal VCD of $1\times10^7$ viable cells/ml on day 6 with 86% of viability. After day 6, VCD and V% decreased quickly and the culture was harvested on day 8. Process #2 helped the culture reach a higher VCD of $1.2\times10^7$ viable cells/ml and sustain one day longer.

As compared to 665.2B9.1E4 cells, 665.B4.1C1 cells exhibited much better growth in both processes. In Process #1, its VCD reached $2\times10^7$ viable cells/ml on day 7 with 97% viability. The culture also maintained this VCD and V% for two more days before it started to decline. The culture was harvested on day 11. In Process #2, 665.B4.1C1 cells showed a similar growth profile as in Processes #1. More specifically, the cells reached the highest VCD of $2.3\times10^7$ viable cells/ml and the viability declined a little slower with the harvest occurring on day 11. This observation was somewhat different from the 665.2B9.1E4 cell line, which demonstrated a growth advantage in Process #2.

The antibody yields of two cell lines in Processes #1 and #2 were compared in figure 27. The final yield of 665.2B9.1E4 cells was 0.42 g/L in Process #1 and 0.55 g/L in Process #2. For comparison, 665.B4.1C1 cells delivered a higher final yield of 1.5 g/L in both processes.

The daily specific antibody productivities (per cell basis) were calculated and are shown in figure 28. As shown in the figure, the 665.2B9.1E4 cells had an average daily $Q_{[MAb]}$ of approximately 15 pg/cell/day throughout the course of cultivation for both processes. The additional day of growth at the highest VCD in Process #2 resulted in a higher final antibody concentration.

The 665.B4.1C1 cells showed a similar daily specific antibody productivity profile in both processes with Process #1 yielding slightly higher productivity. The daily $Q_{[MAb]}$ were maintained between 20~25 pg/cell/day until day 9. Thereafter the productivity declined.

Comparing with the 665.2B9.1E4 cell line, the 665.B4.1C1 cell line exhibited a higher specific antibody productivity of 20~25 pg/cell/day as compared to 15 pg/cell/day. Combining with its better growth, the 665.B4.1C1 cell line tripled the final antibody yield to 1.5 g/L as compared to 0.55 g/L achieved by the 665.2B9.1E4 cell line. These results demonstrate the benefit of incorporating *Bcl-2* gene into the host cell line to enhance its growth and antibody yield in serum-free media in a bioreactor modeled for large-scale commercial preparation of a recombinant protein, in this case an antibody for clinical use.

**SEQUENCE LISTINGS**

SEQ.: ID. No. 1: Complete sequence of the hMN14pdHL2 vector.
LOCUS hMN14pdHL2 9780 bp DNA circular SYN 26-may-2004 DEFINITION hMN14pdHL2 vector.
BASE COUNT 2441 a 2630 c 2366 g 2343 t ORIGIN

```
   1 ttccataggc tccgcccccc tgacgagcat cacaaaaatc gacgctcaag tcagaggtgg
  61 cgaaacccga caggactata aagataccag gcgtttcccc ctggaagctc cctcgtgcgc
 121 tctcctgttc cgaccctgcc gcttaccgga tacctgtccg cctttctccc ttcgggaagc
 181 gtggcgcttt ctcatagctc acgctgtagg tatctcagtt cggtgtaggt cgttcgctcc
 241 aagctgggct gtgtgcacga accccccgtt cagcccgacc gctgcgcctt atccggtaac
 301 tatcgtcttg agtccaaccc ggtaagacac gacttatcgc cactggcagc agccactggt
 361 aacaggatta gcagagcgag gtatgtaggc ggtgctacag agttcttgaa gtggtggcct
 421 aactacggct acactagaag gacagtattt ggtatctgcg ctctgctgaa gccagttacc
 481 ttcggaaaaa gagttggtag ctcttgatcc ggcaaacaaa ccaccgctgg tagcggtggt
 541 ttttttgttt gcaagcagca gattacgcgc agaaaaaaag gatctcaaga agatcctttg
 601 atcttttcta cggggtctga cgctcagtgg aacgaaaact cacgttaagg gattttggtc
 661 atgagattat caaaaaggat cttcacctag atccttttaa attaaaaatg aagttttaaa
 721 tcaatctaaa gtatatatga gtaaacttgg tctgacagtt accaatgctt aatcagtgag
 781 gcacctatct cagcgatctg tctatttcgt tcatccatag ttgcctgact ccccgtcgtg
 841 tagataacta cgatacggga gggcttacca tctggcccca gtgctgcaat gataccgcga
 901 gacccacgct caccggctcc agatttatca gcaataaacc agccagccgg aagggccgag
 961 cgcagaagtg gtcctgcaac tttatccgcc tccatccagt ctattaattg ttgccgggaa
1021 gctagagtaa gtagttcgcc agttaatagt ttgcgcaacg ttgttgccat tgctgcaggc
1081 atcgtggtgt cacgctcgtc gtttggtatg gcttcattca gctccggttc ccaacgatca
1141 aggcgagtta catgatcccc catgttgtgc aaaaaagcgg ttagctcctt cggtcctccg
1201 atcgttgtca gaagtaagtt ggccgcagtg ttatcactca tggttatggc agcactgcat
1261 aattctctta ctgtcatgcc atccgtaaga tgcttttctg tgactggtga gtactcaacc
1321 aagtcattct gagaatagtg tatgcggcga ccgagttgct cttgcccggc gtcaacacgg
1381 gataataccg cgccacatag cagaacttta aaagtgctca tcattggaaa acgttcttcg
1441 gggcgaaaac tctcaaggat cttaccgctg ttgagatcca gttcgatgta acccactcgt
1501 gcacccaact gatcttcagc atcttttact ttcaccagcg tttctgggtg agcaaaaaca
1561 ggaaggcaaa atgccgcaaa aaagggaata agggcgacac ggaaatgttg aatactcata
1621 ctcttccttt ttcaatatta ttgaagcatt tatcagggtt attgtctcat gagcggatac
1681 atatttgaat gtatttagaa aaataaacaa ataggggttc cgcgcacatt tccccgaaaa
1741 ggccacctga cgtctaagaa accattatta tcatgacatt aacctataaa aataggcgta
1801 tcacgaggcc ctttcgtctt caagaattcc gatccagaca tgataagata cattgatgag
1861 tttggacaaa ccacaactag aatgcagtga aaaaaatgct ttatttgtga aatttgtgat
1921 gctattgctt tatttgtaac cattataagc tgcaataaac aagttaacaa caacaattgc
1981 attcatttta tgtttcaggt tcagggggag gtgtgggagg ttttttaaag caagtaaaac
2041 ctctacaaat gtggtatggc tgattatgat ctaaagccag caaaagtccc atggtcttat
2101 aaaaatgcat agctttagga ggggagcaga gaacttgaaa gcatcttcct gttagtcttt
2161 cttctcgtag acttcaaact tatacttgat gccttttttcc tcctggacct cagagaggac
2221 gcctgggtat tctgggagaa gtttatattt ccccaaatca atttctggga aaaacgtgtc
2281 actttcaaat tcctgcatga tccttgtcac aaagagtctg aggtggcctg gttgattcat
2341 ggcttcctgg taaacagaac tgcctccgac tatccaaacc atgtctactt tacttgccaa
2401 ttccggttgt tcaataagtc ttaaggcatc atccaaactt ttggcaagaa aatgagctcc
2461 tcgtggtggt tctttgagtt ctctactgag aactatatta attctgtcct ttaaaggtcg
2521 attcttctca ggaatggaga accaggtttt cctacccata atcaccagat tctgtttacc
2581 ttccactgaa gaggttgtgg tcattctttg gaagtacttg aactcgttcc tgagcggagg
2641 ccagggtcgg tctccgttct tgccaatccc catattttgg gacacggcga cgatgcagtt
2701 caatggtcga accatgaggg caccaagcta gctttttgca aaagcctagg cctccaaaaa
2761 agcctcctca ctacttctgg aatagctcag aggccgaggc ggcctcggcc tctgcataaa
2821 taaaaaaaat tagtcagcca tggggcggag aatgggcgga actgggcgga gttaggggcg
2881 ggatgggcgg agttaggggc gggactatgg ttgctgacta attgagatgc atgctttgca
2941 tacttctgcc tgctggggag cctggggact ttccacacct ggttgctgac taattgagat
3001 gcatgctttg catacttctg cctgctgggg agcctgggga ctttccacac cctaactgac
```

```
3061 acacattcca cagtcgacta gaatatggat agtgggtgtt tatgactctg gataagcctg
3121 aacaattgat gattaatgcc cctgagctct gttcttagta acatgtgaac atttacttgt
3181 gtcagtgtag tagatttcac atgacatctt ataataaacc tgtaaatgaa agtaatttgc
3241 attactagcc cagcccagcc catactaaga gttatattat gtctgtctca cagcctgctg
3301 ctgaccaata ttgaaaagaa tagaccttcg actggcagga agcaggtcat gtggcaaggc
3361 tatttgggga agggaaaata aaaccactag gtaaacttgt agctgtggtt tgaagaagtg
3421 gttttgaaac actctgtcca gccccaccaa accgaaagtc caggctgagc aaaacaccac
3481 ctgggtaatt tgcatttcta aaataagttg aggattcagc cgaaactgga gaggtcctct
3541 tttaacttat tgagttcaac cttttaattt tagcttgagt agttctagtt tccccaaact
3601 taagtttatc gacttctaaa atgtatttag aatttcgacc aattctcatg tttgacagct
3661 tatcatcgct gcactccgcc cgaaaagtgc gctcggctct gccaaggacg cggggcgcgt
3721 gactatgcgt gggctggagc aaccgcctgc tgggtgcaaa ccctttgcgc ccggactcgt
3781 ccaacgacta taaagagggc aggctgtcct ctaagcgtca ccacgacttc aacgtcctga
3841 gtaccttctc ctcacttact ccgtagctcc agcttcacca gatccctcga ctctagaggc
3901 cttaagggcc ttactgagca cacaggacct caccatggga tggagctgta tcatcctctt
3961 cttggtagca acagctacag gtaaggggct cacagtagca ggcttgaggt ctggacatat
4021 atatgggtga caatgacatc cactttgcct ttctctccac aggtgtccac tccgacatcc
4081 agctgaccca gagcccaagc agcctgagcg ccagcgtggg tgacagagtg accatcacct
4141 gtaaggccag tcaggatgtg ggtacttctg tagcctggta ccagcagaag ccaggtaagg
4201 ctccaaagct gctgatctac tggacatcca cccggcacac tggtgtgcca agcagattca
4261 gcggtagcgg tagcggtacc gacttcacct tcaccatcag cagcctccag ccagaggaca
4321 tcgccaccta ctactgccag caatatagcc tctatcggtc gttcggccaa gggaccaagg
4381 tggaaatcaa acgtgagtag aatttaaact ttgcttcctc agttggatcc cgcaattcta
4441 aactctgagg gggtcggatg acgtggccat tctttgccta aagcattgag tttactgcaa
4501 ggtcagaaaa gcatgcaaag ccctcagaat ggctgcaaag agctccaaca aaacaattta
4561 gaactttatt aaggaatagg gggaagctag gaagaaactc aaaacatcaa gattttaaat
4621 acgcttcttg gtctccttgc tataattatc tgggataagc atgctgtttt ctgtctgtcc
4681 ctaacatgcc ctgtgattat ccgcaaacaa cacacccaag ggcagaactt tgttacttaa
4741 acaccatcct gtttgcttct ttcctcagga actgtggctg caccatctgt cttcatcttc
4801 ccgccatctg atgagcagtt gaaatctgga actgcctctg ttgtgtgcct gctgaataac
4861 ttctatccca gagaggccaa agtacagtgg aaggtggata acgccctcca atcgggtaac
4921 tcccaggaga gtgtcacaga gcaggacagc aaggacagca cctacagcct cagcagcacc
4981 ctgacgctga gcaaagcaga ctacgagaaa cacaaagtct acgcctgcga agtcacccat
5041 cagggcctga gctcgcccgt cacaaagagc ttcaacaggg gagagtgtta gagggagaag
5101 tgcccccacc tgctcctcag ttccagcctg accccctccc atcctttggc ctctgaccct
5161 ttttccacag gggacctacc cctattgcgg tcctccagct catctttcac ctcacccccc
5221 tcctcctcct tggctttaat tatgctaatg ttggaggaga atgaataaat aaagtgaatc
5281 tttgcacctg tggtttctct ctttcctcat ttaataatta ttatctgttg ttttaccaac
5341 tactcaattt ctcttataag ggactaaata tgtagtcatc ctaaggcgca taaccattta
5401 taaaaatcat ccttcattct attttaccct atcatcctct gcaagacagt cctccctcaa
5461 acccacaagc cttctgtcct cacagtcccc tgggccatgg taggagagac ttgcttcctt
5521 gttttcccct cctcagcaag ccctcatagt ccttttttaag ggtgacaggt cttacagtca
5581 tatatccttt gattcaattc cctgagaatc aaccaaagca aatttttcaa aagaagaaac
5641 ctgctataaa gagaatcatt cattgcaaca tgatataaaa taacaacaca ataaaagcaa
5701 ttaaataaac aaacaatagg gaaatgttta agttcatcat ggtacttaga cttaatggaa
5761 tgtcatgcct tatttacatt tttaaacagg tactgaggga ctcctgtctg ccaagggccg
5821 tattgagtac tttccacaac ctaatttaat ccacactata ctgtgagatt aaaaacattc
5881 attaaaatgt tgcaaaggtt ctataaagct gagagacaaa tatattctat aactcagcaa
5941 ttcccacttc taggggttcg actggcagga agcaggtcat gtggcaaggc tatttgggga
6001 agggaaaata aaaccactag gtaaacttgt agctgtggtt tgaagaagtg gttttgaaac
6061 actctgtcca gccccaccaa accgaaagtc caggctgagc aaaacaccac ctgggtaatt
6121 tgcatttcta aaataagttg aggattcagc cgaaactgga gaggtcctct tttaacttat
6181 tgagttcaac cttttaattt tagcttgagt agttctagtt tccccaaact taagtttatc
6241 gacttctaaa atgtatttag aatttcgacc aattctcatg tttgacagct tatcatcgct
6301 gcactccgcc cgaaaagtgc gctcggctct gccaaggacg cggggcgcgt gactatgcgt
6361 gggctggagc aaccgcctgc tgggtgcaaa ccctttgcgc ccggactcgt ccaacgacta
6421 taaagagggc aggctgtcct ctaagcgtca ccacgacttc aacgtcctga gtaccttctc
6481 ctcacttact ccgtagctcc agcttcacca gatccctcga gcacacagga cctcaccatg
6541 ggatggagct gtatcatcct cttcttggta gcaacagcta caggtaaggg gctcacagta
6601 gcaggcttga ggtctggaca tatatatggg tgacaatgac atccactttg cctttctctc
```

```
6661 cacaggtgtc cactcccagg tccaactgca ggagagcggt ggaggtgttg tgcaacctgg
6721 ccggtccctg cgcctgtcct gctccgcatc tggcttcgat ttcaccacat attggatgag
6781 ttgggtgcga caggcacctg gaaaaggtct tgagtggatt ggagaaattc atccagatag
6841 cagtacgatt aactatgcgc cgtcgctaaa agatagattt acaatatcgc gagacaacgc
6901 caagaacaca ttgttcctgc aaatggacag cctgagaccc gaagacaccg ggtctattt
6961 ttgtgcaagc ctttacttcg gcttcccctg gtttgcttat tggggccaag ggaccccggt
7021 caccgtctcc tcaggtgagt ccttacaacc tctctcttct attcagctta aatagatttt
7081 actgcatttg ttgggggga aatgtgtgta tctgaatttc aggtcatgaa ggactaggga
7141 caccttggga gtcagaaagg gtcattggga gccccaagct ttctggggca ggccaggcct
7201 gaccttggct ttggggcagg gagggggcta aggtgaggca ggtggcgcca gccaggtgca
7261 cacccaatgc ccatgagccc agacactgga cgctgaacct cgcggacagt taagaaccca
7321 ggggcctctg cgccctgggc ccagctctgt cccacaccgc ggtcacatgg caccacctct
7381 cttgcagcct ccaccaaggg cccatcggtc ttccccctgg caccctcctc caagagcacc
7441 tctgggggca cagcggccct gggctgcctg gtcaaggact acttccccga accggtgacg
7501 gtgtcgtgga actcaggcgc cctgaccagc ggcgtgcaca ccttcccggc tgtcctacag
7561 tcctcaggac tctactccct cagcagcgtg gtgaccgtgc cctccagcag cttgggcacc
7621 cagacctaca tctgcaacgt gaatcacaag cccagcaaca ccaaggtgga caagagagtt
7681 ggtgagaggc cagcacaggg agggagggtg tctgctggaa gccaggctca gcgctcctgc
7741 ctggacgcat cccggctatg cagccccagt ccagggcagc aaggcaggcc ccgtctgcct
7801 cttcacccgg agcctctgcc cgccccactc atgctcaggg agagggtctt ctggcttttt
7861 cccaggctct gggcaggcac aggctaggtg cccctaaccc aggccctgca cacaaagggg
7921 caggtgctgg gctcagacct gccaagagcc atatccggga ggaccctgcc cctgacctaa
7981 gcccacccca aaggccaaac tctccactcc ctcagctcgg acaccttctc tcctcccaga
8041 ttccagtaac tcccaatctt ctctctgcag agcccaaatc ttgtgacaaa actcacacat
8101 gcccaccgtg cccaggtaag ccagcccagg cctcgccctc cagctcaagg cgggacaggt
8161 gccctagagt agcctgcatc cagggacagg ccccagccgg gtgctgacac gtccacctcc
8221 atctcttcct cagcacctga actcctgggg ggaccgtcag tcttcctctt ccccccaaaa
8281 cccaaggaca ccctcatgat ctcccggacc cctgaggtca catgcgtggt ggtggacgtg
8341 agccacgaag accctgaggt caagttcaac tggtacgtgg acggcgtgga ggtgcataat
8401 gccaagacaa agccgcggga ggagcagtac aacagcacgt accgggtggt cagcgtcctc
8461 accgtcctgc accaggactg gctgaatggc aaggagtaca agtgcaaggt ctccaacaaa
8521 gccctcccag cccccatcga gaaaaccatc tccaaagcca aaggtgggac ccgtggggtg
8581 cgagggccac atggacagag gccggctcgg cccaccctct gccctgagag tgaccgctgt
8641 accaacctct gtcctacagg gcagccccga gaaccacagg tgtacaccct gcccccatcc
8701 cgggaggaga tgaccaagaa ccaggtcagc ctgacctgcc tggtcaaagg cttctatccc
8761 agcgacatcg ccgtggagtg ggagagcaat gggcagccgg agaacaacta caagaccacg
8821 cctcccgtgc tggactccga cggctccttc ttcctctata gcaagctcac cgtggacaag
8881 agcaggtggc agcaggggaa cgtcttctca tgctccgtga tgcatgaggc tctgcacaac
8941 cactacacgc agaagagcct ctccctgtct ccgggtaaat gagtgcgacg gccggcaagc
9001 ccccgctccc cgggctctcg cggtcgcacg aggatgcttg gcacgtaccc cgtctacata
9061 cttcccaggc acccagcatg gaaataaagc acccaccact gccctgggcc cctgcgagac
9121 tgtgatggtt ctttccacgg gtcaggccga gtctgaggcc tgagtggcat gagggaggca
9181 gagcgggtcc cactgtcccc acactggccc aggctgtgca ggtgtgcctg ggccgcctag
9241 ggtggggctc agccaggggc tgccctcggc agggtggggg atttgccagc gtggccctcc
9301 ctccagcagc agctgcctcg cgcgtttcgg tgatgacggt gaaaacctct gacacatgca
9361 gctcccggag acggtcacag cttgtctgta agcggatgcc gggagcagac aagcccgtca
9421 gggcgcgtca gcgggtgttg gcgggtgtcg gggcgcagcc atgacccagt cacgtagcga
9481 tagcggagtg tatactggct taactatgcg gcatcagagc agattgtact gagagtgcac
9541 catatgcggt gtgaaatacc gcacagatgc gtaaggagaa aataccgcat caggcgctct
9601 tccgcttcct cgctcactga ctcgctgcgc tcggtcgttc ggctgcggcg agcggtatca
9661 gctcactcaa aggcggtaat acggttatcc acagaatcag gggataacgc aggaaagaac
9721 atgtgagcaa aaggccagca aaaggccagg aaccgtaaaa aggccgcgtt gctggcgttt
```

SEQ: ID. No. 2: Enhancer and promoter sequences of the SV40 enhancer and promoter respectively, that are present in the hMN-14pdHL2 vector used to transfect the SP2/0 cells to obtain the 665.2B9 clone.

hLL2pdHL2

Rep ori

2701 CAATGGTCGA ACCATGAGGG CACCAAGCTA **GCTTTTTGCA AAAGCCTAGG CCTCCAAAAA**

*dhfr*

SV40 ori (2749-2832) 1

2761 **AGCCTCCTCA CTACTTCTGG AATAGCTCAG AGGCCGAGGC GGCCTCGGCC TCTGCATAAA**

nt1 of SV40 genome

2821 **TAAAAAAAAT TAGTCAGCCA TGGGGCGGAG AATGGGCGGA ACTGGGCGGA GTTAGGGGCG**

SV40 Early promoter

2881 **GGATGGGCGG AGTTAGGGGC GGGACTA**TGG TTGCTGACTA ATTGAGATGC ATGCTTTGCA

SV40 Enh (2908-2979)

2941 TACTTCTGCC TGCTGGGGAG CCTGGGGACT TTCCACACCT GGTTGCTGAC TAATTGAGAT

SV40 Enh (2980-3051)

3001 GCATGCTTTG CATACTTCTG CCTGCTGGGG AGCCTGGGGA CTTTCCACAC C**CTAACTGAC**

L1 pro

3061 **ACACATTCCA CAGTCGAC**TA GAATATGGAT AGTGGGTGTT TATGACTCTG GATAAGCCTG

SEQ. ID. NO. 3: Nucleic acid sequence of Bcl-2-EEE gene

5'-
ATGGCGCACG CTGGGAGAAC GGGGTACGAT AACCGGGAGA TAGTGATGAA GTACATCCAT
TATAAGCTGT CGCAGAGGGG CTACGAGTGG GATGCGGGAG ATGTGGGCGC CGCGCCCCCG
GGGGCCGCCC CCGCACCGGG CATCTTCTCC TCCCAGCCCG GGCACACGCC CCATCCAGCC
GCATCCCGCG ACCCGGTCGC CAGAGAAGAA CCGCTGCAGA CTCCGGCTGC TCCTGGAGCA
GCTGCAGGAC CTGCGCTCGA ACCGGTGCCA CCTGTGGTCC ACCTGACCCT CCGCCAGGCC
GGCGACGACT TCTCCCGCCG CTACCGCCGC GACTTCGCCG AGATGTCCAG CCAGCTGCAC
CTGACGCCCT TCACCGCGCG GGGACGCTTT GCCACGGTGG TGGAGGAGCT CTTCAGGGAC
GGGGTGAACT GGGGGAGGAT TGTGGCCTTC TTTGAGTTCG GTGGGGTCAT GTGTGTGGAG
AGCGTCAACC GGGAGATGTC GCCCCTGGTG GACAACATCG CCCTGTGGAT GACTGAGTAC
CTGAACCGGC ACCTGCACAC CTGGATCCAG GATAACGGAG GCTGGGATGC CTTTGTGGAA
CTGTACGGCC CCAGCATGCG GCCTCTGTTT GATTTCTCCT GGCTGTCTCT GAAGACTCTG
CTCAGTTTGG CCCTGGTGGG AGCTTGCATC ACCCTGGGTG CCTATCTGGG CCACAAGTGA-3'

SEQ. ID. NO. 4: Amino acid sequence of Bcl-2-EEE protein

1 MAHAGRTGYD NREIVMKYIH YKLSQRGYEW DAGDVGAAPP GAAPAPGIFS SQPGHTPHPA

61 ASRDPVAREE PLQTPAAPGA AAGPALEPVP PVVHLTLRQA GDDFSRRYRR DFAEMSSQLH

121 LTPFTARGRF ATVVEELFRD GVNWGRIVAF FEFGGVMCVE SVNREMSPLV DNIALWMTEY

181 LNRHLHTWIQ DNGGWDAFVE LYGPSMRPLF DFSWLSLKTL LSLALVGACI TLGAYLGHK

SEQ. ID. NO. 5: Bcl-2-EEE Left (80)

5'GGACCCGGTCGCCAGAGAAGAACCGCTGCAGACTCCGGCTGCTCCTGGAGCAG CTGCAGGACCTGCGCTCGAACCGGTGC-3'

SEQ. ID. NO. 6: Bcl-2-EEE Right (80)

5'CGCCGGCCTGGCGGAGGGTCAGGTGGACCACAGGTGGCACCGGTTCGAGCGCA GGTCCTGCAGCTGCTCCAGGAGCAGCC-3'

## Claims

1. A method of making a recombinant protein, comprising culturing a host cell in a suitable serum-free medium under conditions suitable for expression of said recombinant protein, wherein said host cell is a myeloma Sp2/0 cell comprising a nucleic acid sequence encoding said recombinant protein, wherein said host cell has been transfected with an expression vector encoding the Bcl-2 triple mutant T69E, S70E and S87 E and wherein said host cell, upon transfection with the expression vector encoding the Bcl-2 triple mutant, is capable of expressing the recombinant protein.

2. The method according to claim 1, wherein said mutant Bcl-2 protein is encoded by a nucleic acid sequence integrated in the chromosomal DNA of said host cell.

3. The method according to claim 1, wherein said medium further comprises at least one caspase inhibitor.

4. The method according to claim 3, wherein said caspase inhibitor is selected from the group consisting of caspase-1, caspase-3, caspase-9, caspase-12 and pan-caspase inhibitors.

5. The method according to claim 3, wherein said inhibitor is selected from the group consisting of Z-VAD-fink, Ac-DEVD-cho, Aven and XIAP.

6. The method according to claim 1, wherein said medium further comprises an exogenously added agent that inhibits apoptosis and/or functions as a cytoprotective agent.

7. The method according to claim 6, wherein said exogenous agent is a member of the cytokine type I superfamily.

8. The method according to claim 7, wherein said member of the cytokine type I superfamily is erythropoietin.

9. The method according to claim 1, wherein said recombinant protein is selected from the group consisting of immunoglobulins, peptides, enzymes, growth factors, hormones, vaccines, lymphokines and cytokines.

10. The method according to claim 9, wherein said recombinant protein is an immunoglobulin selected from the group consisting of antibodies, antibody fragments, multispecific antibodies, and single chain antibodies.

11. The method according to claim 9 wherein said protein is a growth factor selected from the group consisting of erythropoietin, G-CSF, GM-CSF, EGF, VEGF, and thrombopoietin.

12. The method according to claim 9, wherein said protein is a lymphokine selected from the group consisting of any of IL-1 to IL-31, alpha interferon, beta interferon, gamma interferon and consensus interferon.

13. A host cell transfected with an expression vector encoding the Bcl-2 triple mutant T69E, S70E and S87E and wherein said host cell is a myeloma Sp2/0 cell comprising a nucleic acid sequence encoding a recombinant protein of interest and which is capable of expressing the recombinant protein in serum-free medium.

14. The host cell according to claim 13, wherein said recombinant protein is selected from the group consisting of immunoglobulins, peptides, enzymes, growth factors, hormones, vaccines, lymphokines and cytokines.

15. The method of claim 1, wherein the modification of said host cell results in an increase in cell culture longevity.

16. The method of claim 1, wherein said host cell is cultured in a perfusion reactor or said host cell is cultured in suspension, resulting in an increase in cell culture longevity of at least 2 days.

17. The method of claim 1, wherein said host cell is cultured in a perfusion reactor, said host cell is cultured in a fed-batch culture or said host cell is cultured in suspension, resulting in an increase in cell culture longevity of at least 4 days.

18. The method of claim 1, wherein said host cell is cultured in a perfusion reactor, said host cell is cultured in a fed-batch culture or said host cell is cultured in suspension, resulting in an increase in cell culture longevity of at least 6 days.

**Patentansprüche**

1. Verfahren zum Herstellen eines rekombinanten Proteins, umfassend Kultivierung einer Wirtszelle in einem geeigneten Serum-freien Medium unter Bedingungen, die für die Expression des rekombinanten Proteins geeignet sind, wobei die Wirtszelle eine Myelom-Sp2/0-Zelle ist, die eine Nukleinsäuresequenz umfasst, die das rekombinante Protein codiert, wobei die Wirtszelle mit einem Expressionsvektor transfiziert worden ist, der die Bcl-2-Dreifachmutante T69E, S70E und S87E codiert, und wobei die Wirtszelle, nach Transfektion mit dem Expressionsvektor, der die Bcl-2-Dreifachmutante codiert, in der Lage ist, das rekombinante Protein zu exprimieren.

2. Verfahren nach Anspruch 1, wobei das mutierte Bcl-2-Protein durch eine Nukleinsäure codiert wird, die in die chromosomale DNA der Wirtszelle integriert ist.

3. Verfahren nach Anspruch 1, wobei das Medium weiter wenigstens einen Caspase-Inhibitor umfasst.

4. Verfahren nach Anspruch 3, wobei der Caspase-Inhibitor ausgewählt ist aus der Gruppe bestehend aus Caspase-1-, Caspase-3-, Caspase-9-, Caspase-12- und Pan-Caspase-Inhibitoren.

5. Verfahren nach Anspruch 3, wobei der Inhibitor ausgewählt ist aus der Gruppe bestehend aus Z-VAD-fink, Ac-DEVD-cho, Aven und XIAP.

6. Verfahren nach Anspruch 1, wobei das Medium weiter ein Exogen hinzugegebenes Agens umfasst, das Apoptose inhibiert und/oder wirksam ist als ein cytoprotektives Agens.

7. Verfahren nach Anspruch 6, wobei das exogene Agens ein Mitglied der Cytokin-Typ I-Superfamilie ist.

8. Verfahren nach Anspruch 7, wobei das Mitglied der Cytokin-Typ-I-Superfamilie Erythropoietin ist.

9. Verfahren nach Anspruch 1, wobei das rekombinante Protein ausgewählt ist aus der Gruppe bestehend aus Immunglobulinen, Peptiden, Enzymen, Wachstumsfaktoren, Hormonen, Vakzinen, Lymphokinen und Cytokinen.

10. Verfahren nach Anspruch 9, wobei das rekombinante Protein ein Immunglobulin ist, das ausgewählt ist aus der Gruppe bestehend aus Antikörpern, Antikörperfragmenten, multispezifischen Antikörpern und einzelkettigen Antikörpern.

11. Verfahren nach Anspruch 9, wobei das Protein ein Wachstumsfaktor ist, der ausgewählt ist aus der Gruppe bestehend aus Erythropoietin, G-CSF, GM-CSF, EGF, VEGF und Thrombopoietin.

12. Verfahren nach Anspruch 9, wobei das Protein ein Lymphokin ist, das ausgewählt ist aus der Gruppe bestehend aus einem jeglichen von IL-1 bis IL-31, alpha-Interferon, beta-Interferon, gamma-Interferon und Konsens-Interferon.

13. Wirtszelle transfiziert mit einem Expressionsvektor, der die Bcl-2-Dreifachmutante T69E, S70E und S87E codiert, und wobei die Wirtszelle eine Myelom-Sp2/0-Zelle ist umfassend eine Nukleinsäuresequenz, die ein interessierendes rekombinantes Protein codiert und in der Lage ist, das rekombinante Proteinen in Serum-freiem Medium zu exprimieren.

14. Wirtszelle nach Anspruch 13, wobei das rekombinante Protein ausgewählt ist aus der Gruppe bestehend aus Immunoglobulinen, Peptiden, Enzymen, Wachstumsfaktoren, Hormonen, Vakzinen, Lymphokinen und Cytokinen.

15. Verfahren nach Anspruch 1, wobei die Modifikation der Wirtszelle zu einer erhöhten Langlebigkeit in Zellkultur führt.

16. Verfahren nach Anspruch 1, wobei die Wirtszelle in einem Perfusionsreaktor kultiviert wird oder die Wirtszelle in Suspension kultiviert wird, was zu einer Erhöhung der Langlebigkeit in Zellkultur von wenigstens 2 Tagen führt.

17. Verfahren nach Anspruch 1, wobei die Wirtszelle in einem Perfusionsreaktor kultiviert wird, die Wirtszelle in einer Fed-Batch-Kultur kultiviert wird oder die Wirtszelle in Suspension kultiviert wird, was zu einer Erhöhung der Langlebigkeit in Zellkultur von wenigstens 4 Tagen führt.

18. Verfahren nach Anspruch 1, wobei die Wirtszelle in einem Perfusionsreaktor kultiviert wird, die Wirtszelle in einer Fed-Batch-Kultur kultiviert wird oder die Wirtszelle in Suspension kultiviert wird, was zu einer Erhöhung der Langlebigkeit in Zellkultur von wenigstens 6 Tagen führt.

## Revendications

1. Procédé de production d'une protéine recombinante, comprenant la culture d'une cellule hôte dans un milieu sans sérum approprié dans des conditions appropriées pour l'expression de ladite protéine recombinante, dans lequel ladite cellule hôte est une cellule myélome Sp2/0 comprenant une séquence d'acide nucléique codant pour ladite protéine recombinante, dans lequel ladite cellule hôte a été transfectée par un vecteur d'expression codant pour le triple mutant de Bcl-2 T69E, S70E et S87 E et dans lequel ladite cellule hôte, lors de la transfection avec le vecteur expression codant pour le triple mutant Bcl-2, est en mesure d'exprimer la protéine recombinante.

2. Procédé selon la revendication 1, dans lequel ladite protéine mutante Bcl-2 est codée par une séquence d'acide nucléique intégrée dans l'ADN chromosomique de ladite cellule hôte.

3. Procédé selon la revendication 1, dans lequel ledit milieu comprend également au moins un inhibiteur de la caspase.

4. Procédé selon la revendication 3, dans lequel ledit inhibiteur de la caspase est choisi dans le groupe composé des inhibiteurs de la caspase-1, de la caspase-3, de la caspase-9, de la caspase-12 et de la pan-caspase.

5. Procédé selon la revendication 3, dans lequel ledit inhibiteur est choisi dans le groupe composé de Z-VAD-fink, Ac-DEVD-cho, Aven et XIAP.

6. Procédé selon la revendication 1, dans lequel ledit milieu comprend également un agent ajouté de façon exogène qui inhibe l'apoptose et/ou qui fonctionne comme un agent cytoprotecteur.

7. Procédé selon la revendication 6, dans lequel ledit agent exogène est un membre de la superfamille de cytokine de type I.

**8.** Procédé selon la revendication 7, dans lequel ledit membre de la superfamille de cytokine de type I est l'érythropoïétine.

**9.** Procédé selon la revendication 1, dans lequel ladite protéine recombinante est choisie dans le groupe composé des immunoglobulines, des peptides, des enzymes, des facteurs de croissance, des hormones, des vaccins, des lymphokines et des cytokines.

**10.** Procédé selon la revendication 9, dans lequel ladite protéine recombinante est une immunoglobuline choisie dans le groupe composé d'anticorps, de fragments d'anticorps, d'anticorps multi-spécifiques et d'anticorps à chaîne unique.

**11.** Procédé selon la revendication 9, dans lequel ladite protéine est un facteur de croissance choisi dans le groupe composé de l'érythropoïétine, du G-CSF, du GM-CSF, de l'EGF, du VEGF et de la thrombopoïétine.

**12.** Procédé selon la revendication 9, dans lequel ladite protéine est une lymphokine choisie dans le groupe composé de l'une quelconque de l'IL-1 à IL-31, de l'interféron alpha, de l'interféron bêta, de l'interféron gamma et de l'interféron consensus.

**13.** Cellule hôte transfectée par un vecteur d'expression codant pour le triple mutant Bcl-2 T69E, S70E et S87E et dans laquelle ladite cellule hôte est une cellule myélome Sp2/0 comprenant une séquence d'acide nucléique codant pour une protéine recombinante d'intérêt et qui est en mesure d'exprimer la protéine recombinante dans un milieu sans sérum.

**14.** Cellule hôte selon la revendication 13, dans laquelle ladite protéine recombinante est choisie dans le groupe composé des immunoglobulines, des peptides, des enzymes, des facteurs de croissance, des hormones, des vaccins, des lymphokines et des cytokines.

**15.** Procédé de la revendication 1, dans lequel la modification de ladite cellule hôte entraîne une augmentation de la longévité en culture cellulaire.

**16.** Procédé de la revendication 1, dans lequel ladite cellule hôte est cultivée dans un réacteur d'infusion ou ladite cellule hôte est cultivée en suspension, entraînant une augmentation de la longévité de la culture cellulaire d'au moins 2 jours.

**17.** Procédé de la revendication 1, dans lequel ladite cellule hôte est cultivée dans un réacteur d'infusion, ladite cellule hôte est cultivée en culture alimentée en discontinu ou ladite cellule hôte est cultivée en suspension, entraînant une augmentation de la longévité de la culture cellulaire d'au moins 4 jours.

**18.** Procédé de la revendication 1, dans lequel ladite cellule hôte est cultivée dans un réacteur d'infusion, ladite cellule hôte est cultivée en culture alimentée en discontinu ou ladite cellule hôte est cultivée en suspension, entraînant une augmentation de la longévité de la culture cellulaire d'au moins 6 jours.

Fig. 1

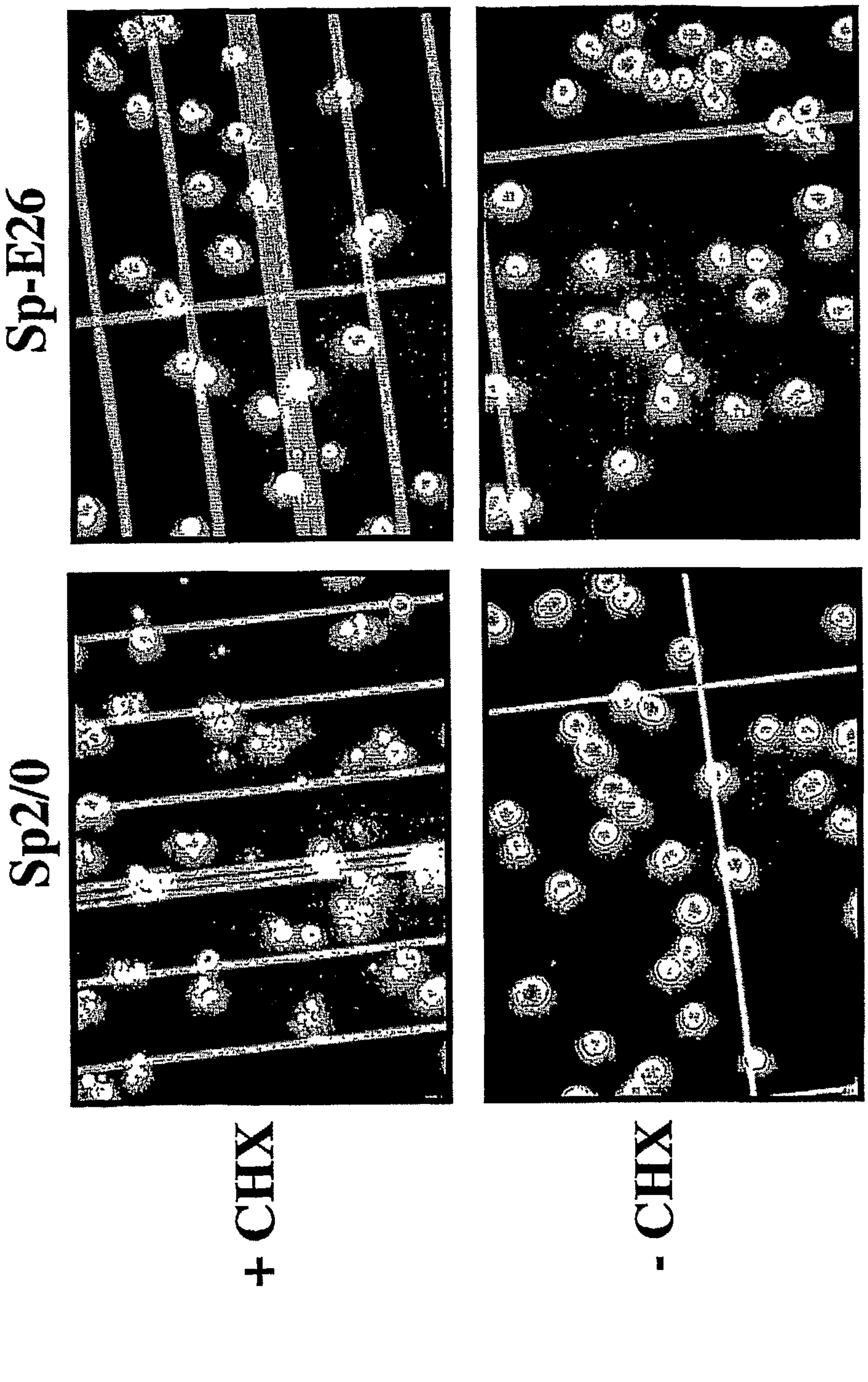

Fig. 2

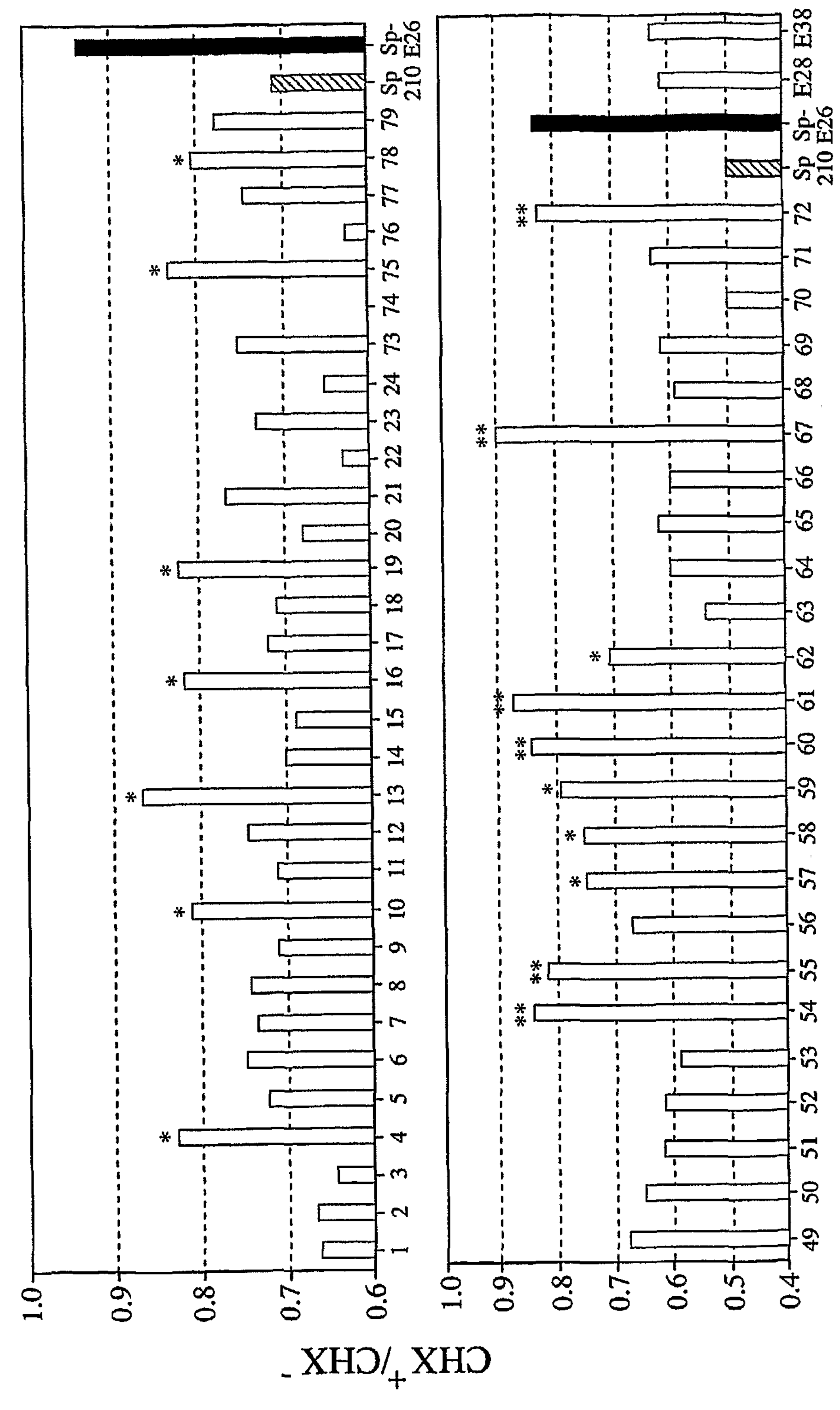
CHX+/CHX-
1.0
0.9
0.8
0.7
0.6
0.5
0.4
1 2 3 4 5 6 7 8 9 10 11 12 13 14 15 16 17 18 19 20 21 22 23 24 73 74 75 76 77 78 79
Sp 210
Sp-E26
49 50 51 52 53 54 55 56 57 58 59 60 61 62 63 64 65 66 67 68 69 70 71 72
E28
E38
*
**

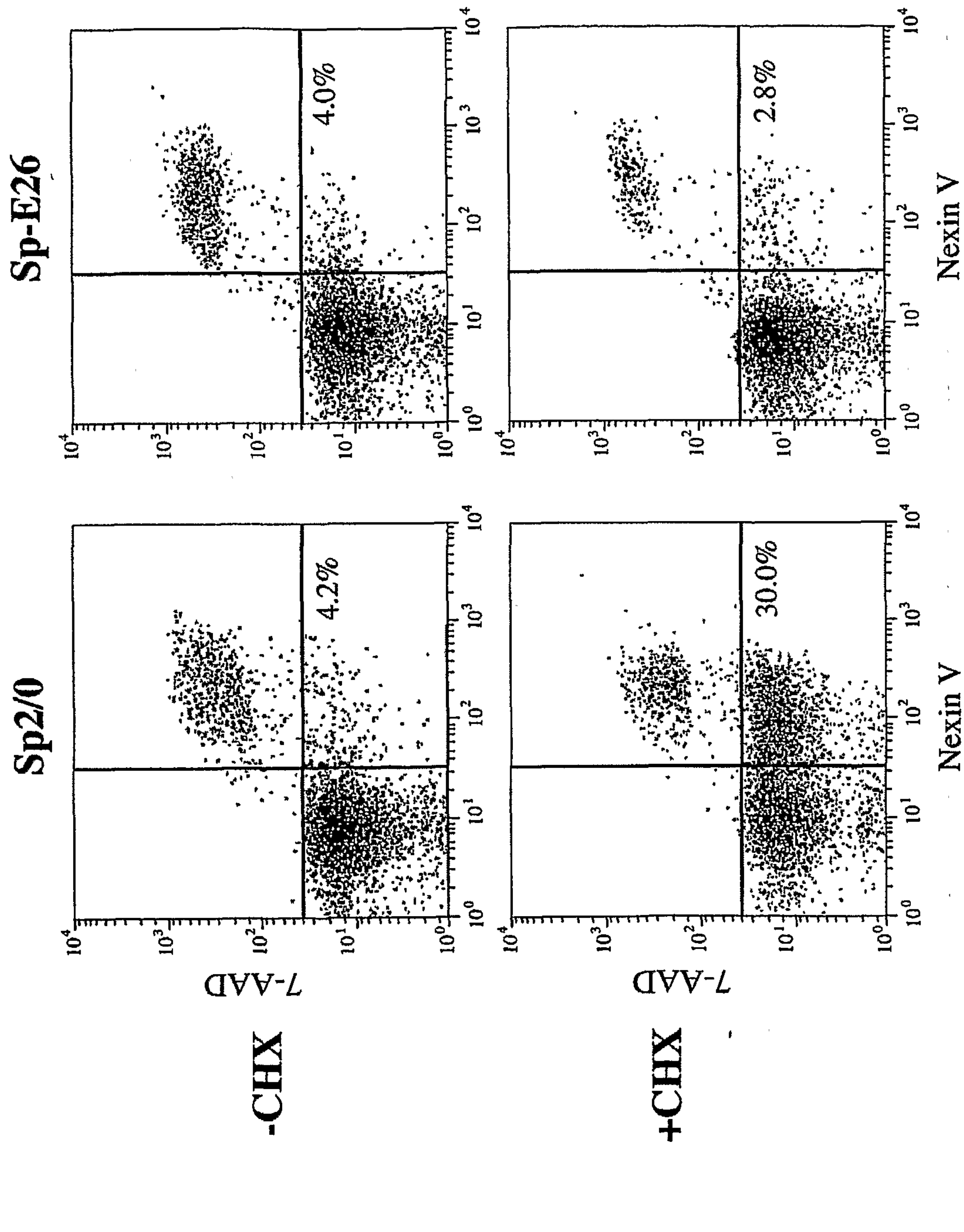


Fig. 3

# Fig. 4

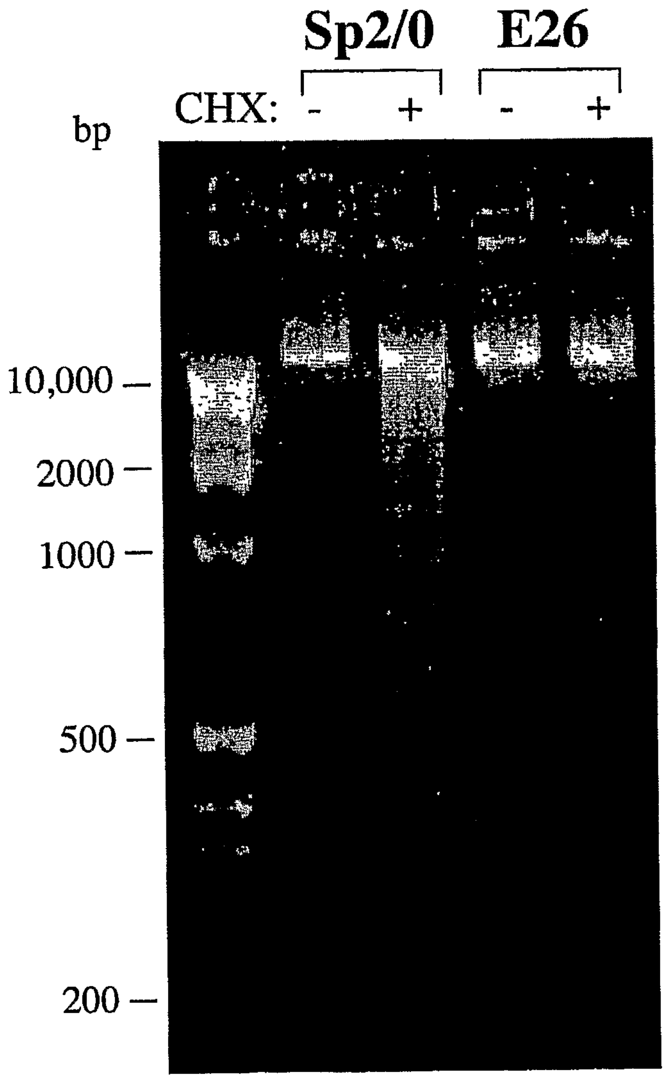

# Fig. 5

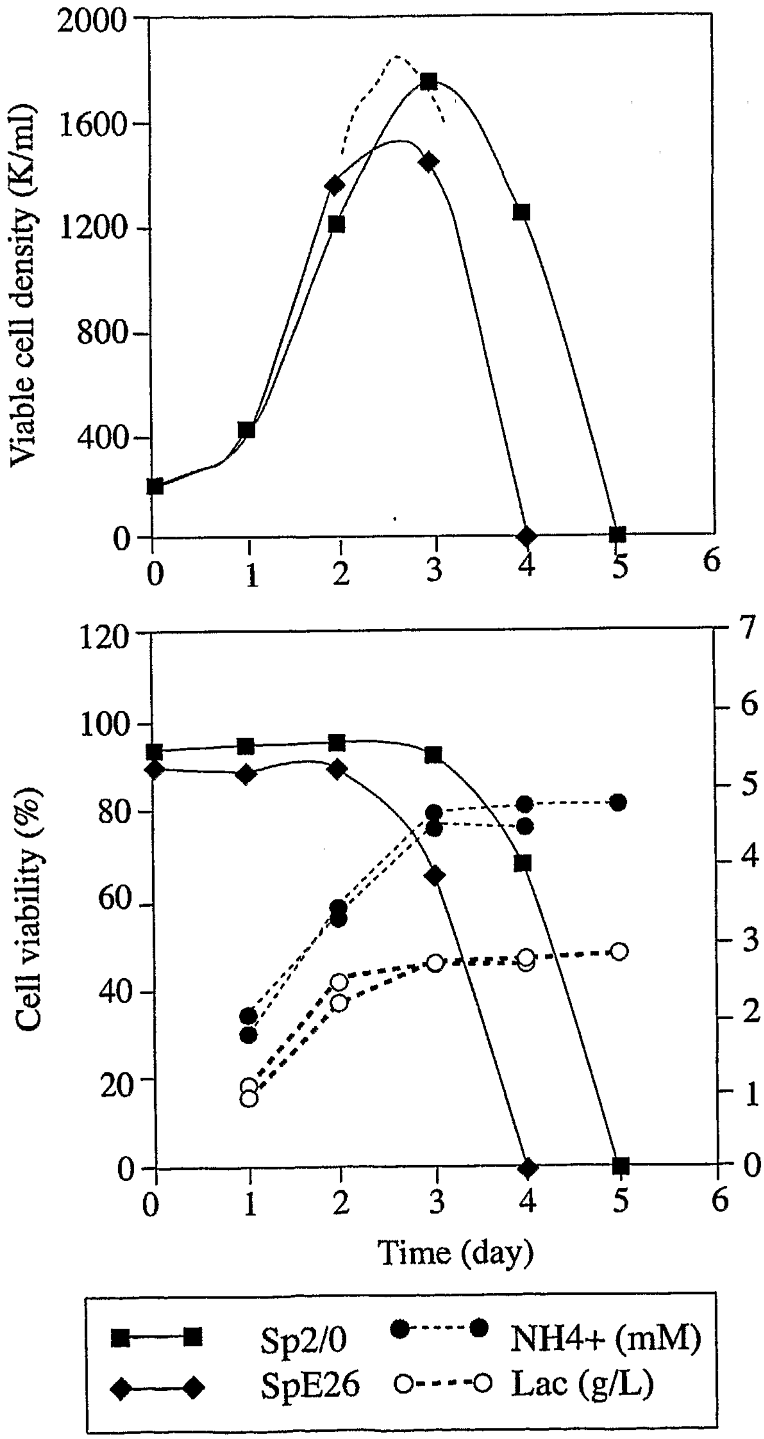
2000
1600
1200
800
400
0
Viable cell density (K/ml)
0 1 2 3 4 5 6
120
100
80
60
40
20
0
Cell viability (%)
7
6
5
4
3
2
1
0
0 1 2 3 4 5 6
Time (day)
Sp2/0
SpE26
NH4+ (mM)
Lac (g/L)

# Fig. 6

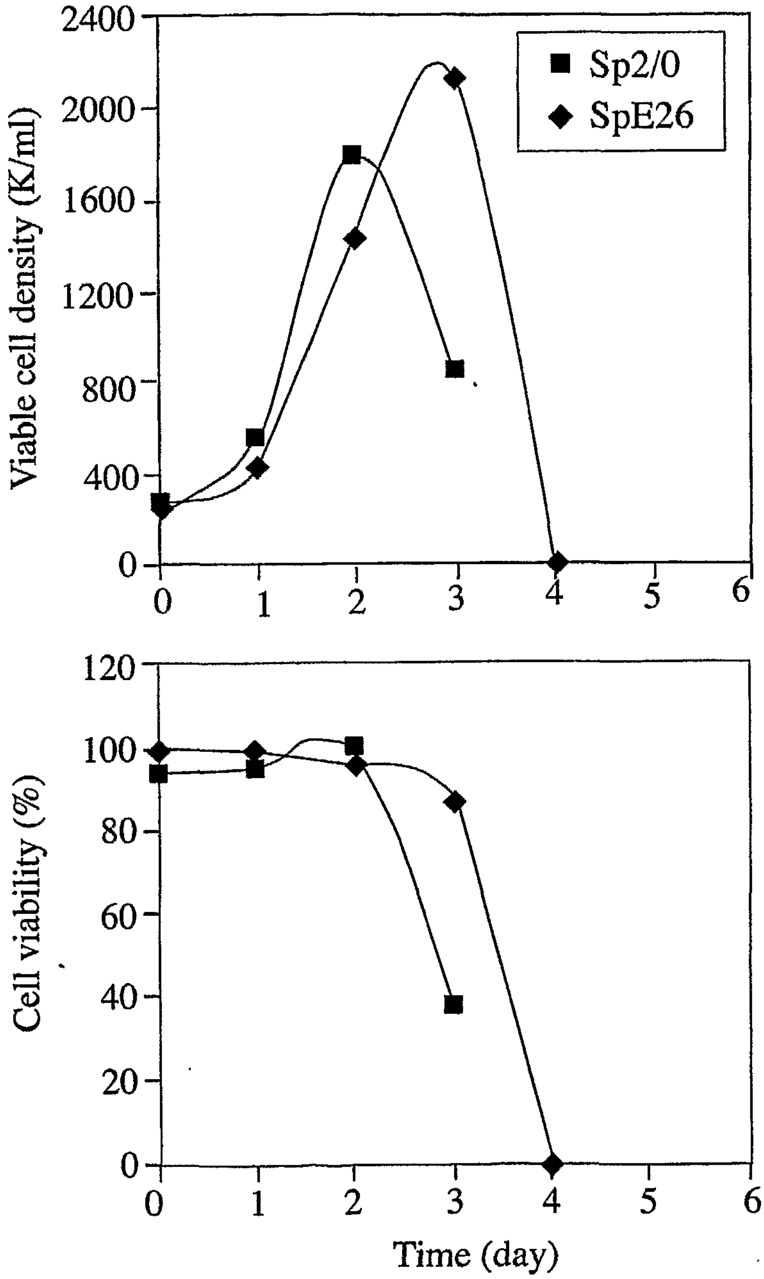
2400
2000
1600
1200
800
400
0
Viable cell density (K/ml)
Sp2/0
SpE26
0
1
2
3
4
5
6
120
100
80
60
40
20
0
Cell viability (%)
0
1
2
3
4
5
6
Time (day)

Fig. 7

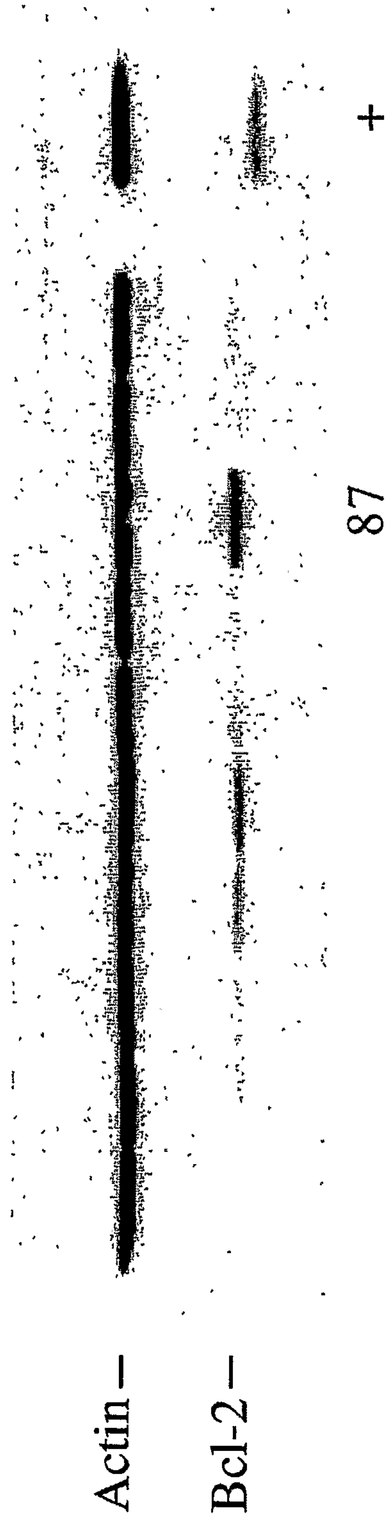

Fig. 8

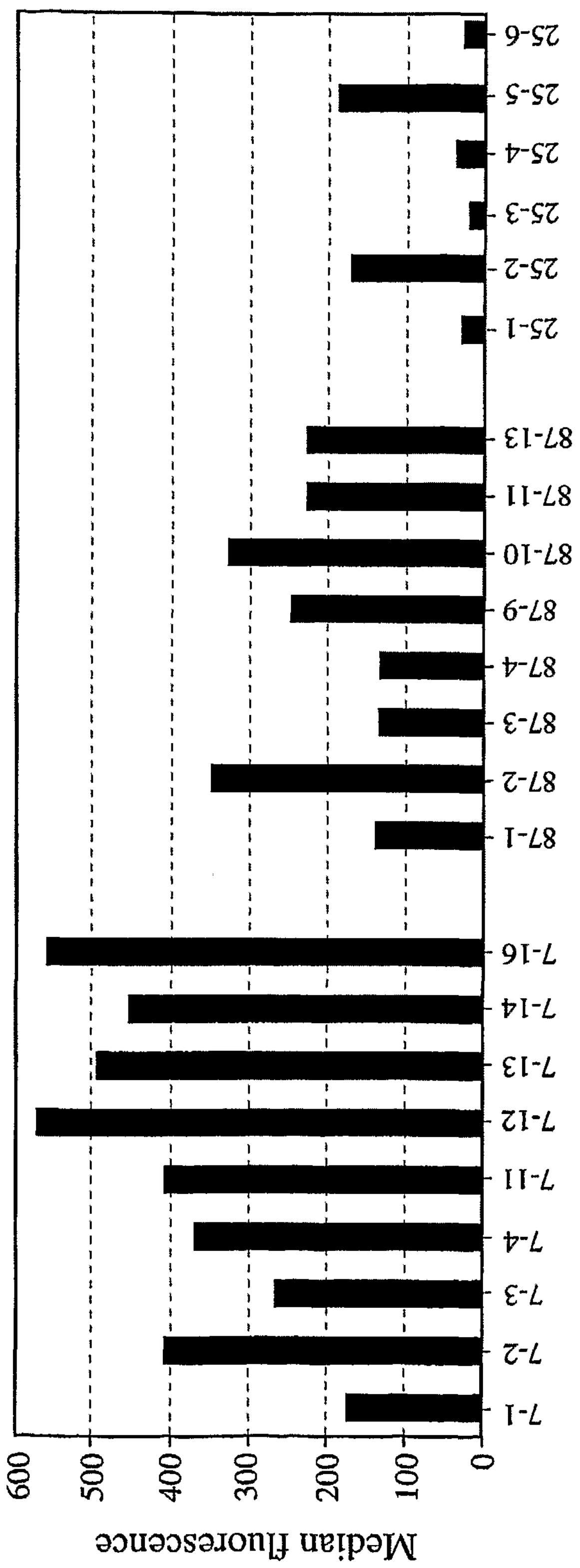
Median fluorescence
0
100
200
300
400
500
600
7-1
7-2
7-3
7-4
7-11
7-12
7-13
7-14
7-16
87-1
87-2
87-3
87-4
87-9
87-10
87-11
87-13
25-1
25-2
25-3
25-4
25-5
25-6

Fig. 9

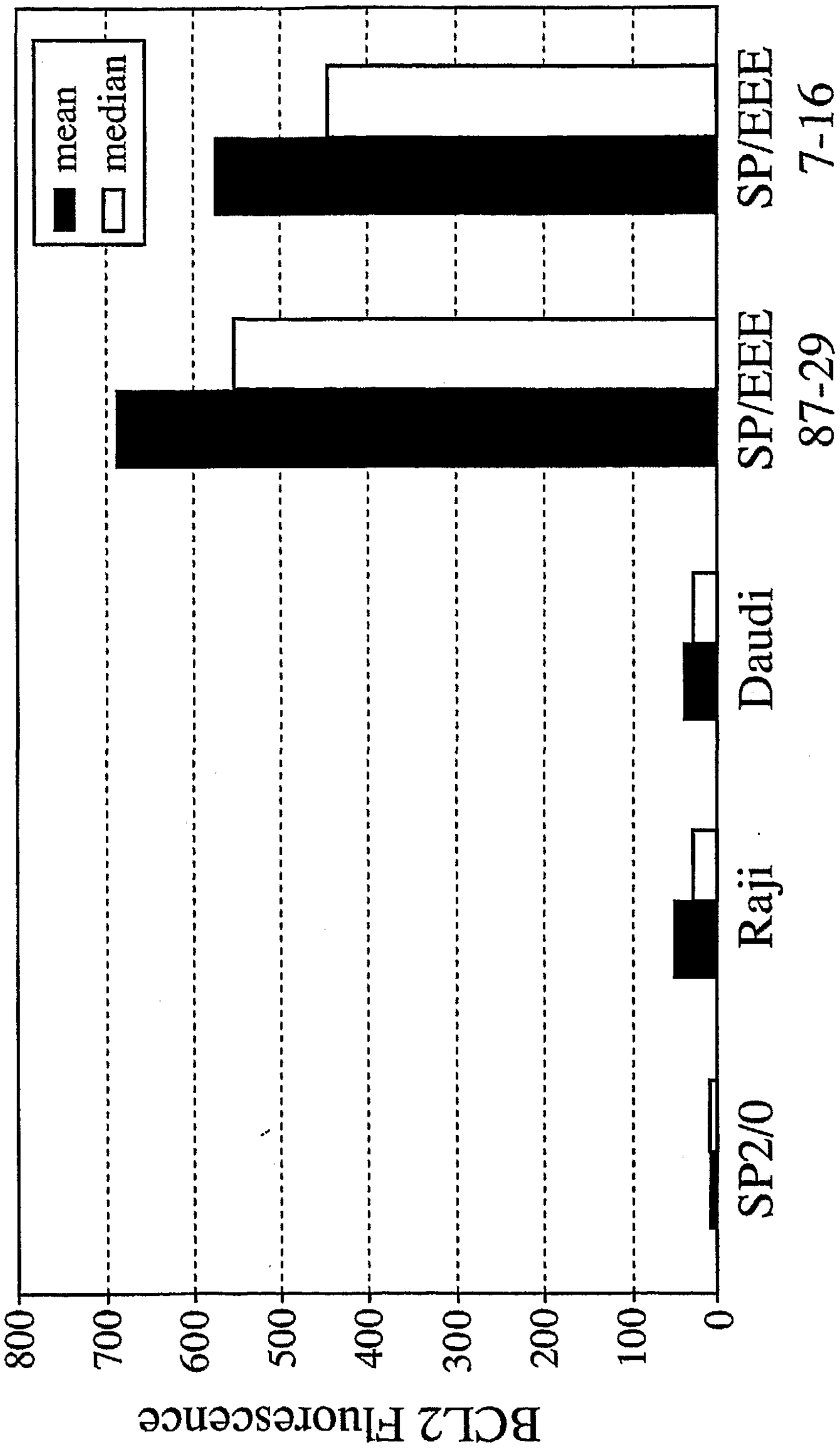
mean
median
BCL2 Fluorescence
0
100
200
300
400
500
600
700
800
SP2/0
Raji
Daudi
SP/EEE 87-29
SP/EEE 7-16

# Fig. 10

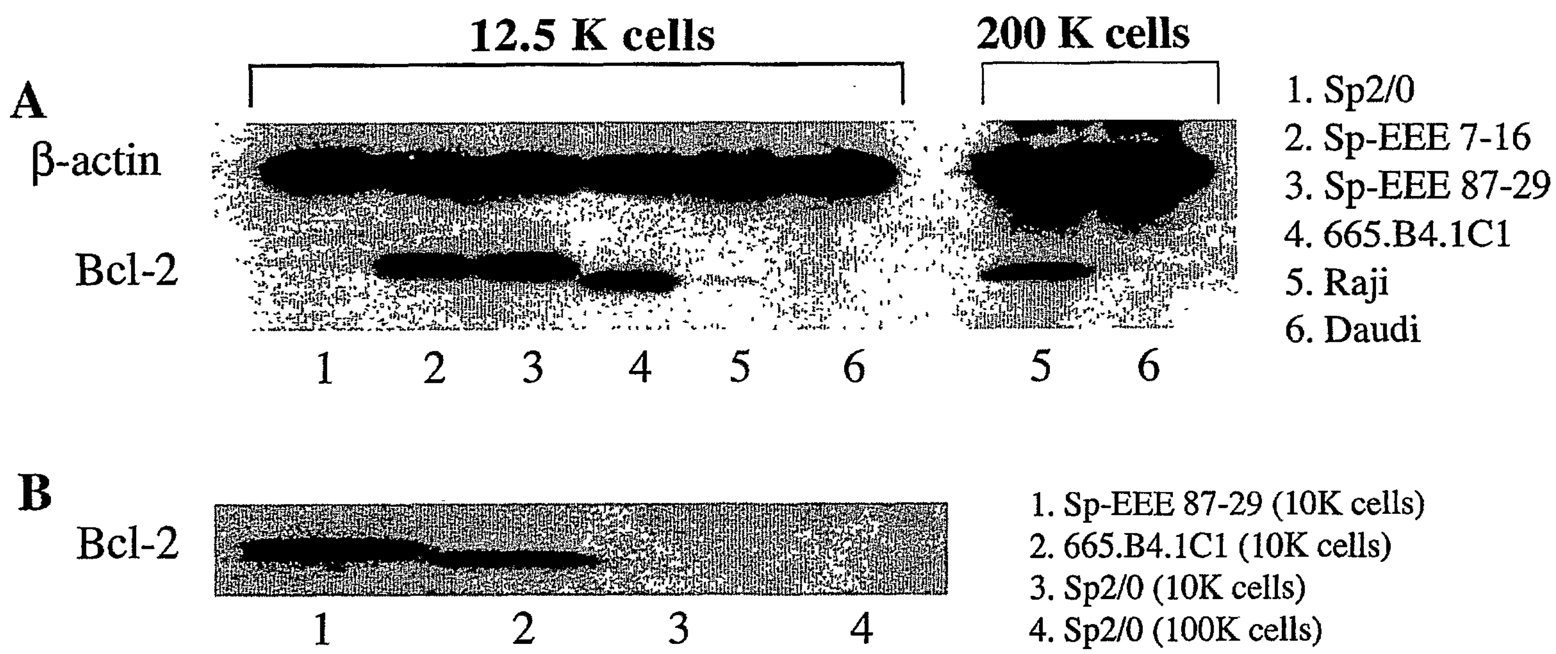
A
β-actin
Bcl-2
12.5 K cells
200 K cells
1 2 3 4 5 6
5 6
1. Sp2/0
2. Sp-EEE 7-16
3. Sp-EEE 87-29
4. 665.B4.1C1
5. Raji
6. Daudi
B
Bcl-2
1 2 3 4
1. Sp-EEE 87-29 (10K cells)
2. 665.B4.1C1 (10K cells)
3. Sp2/0 (10K cells)
4. Sp2/0 (100K cells)

# Fig. 11

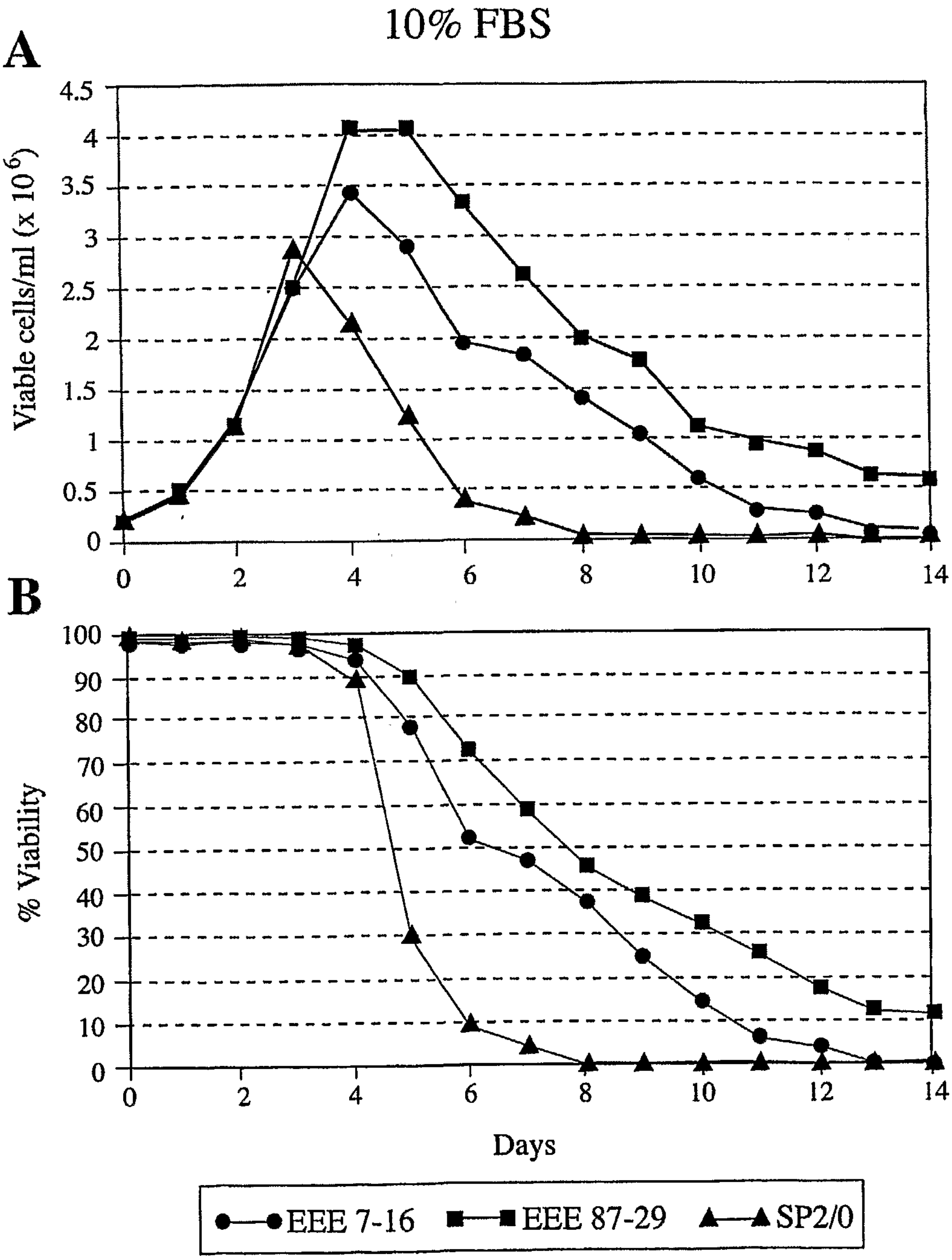
10% FBS
A
Viable cells/ml (x 10^6)
4.5
4
3.5
3
2.5
2
1.5
1
0.5
0
0
2
4
6
8
10
12
14
B
% Viability
100
90
80
70
60
50
40
30
20
10
0
Days
EEE 7-16
EEE 87-29
SP2/0

# Fig. 12

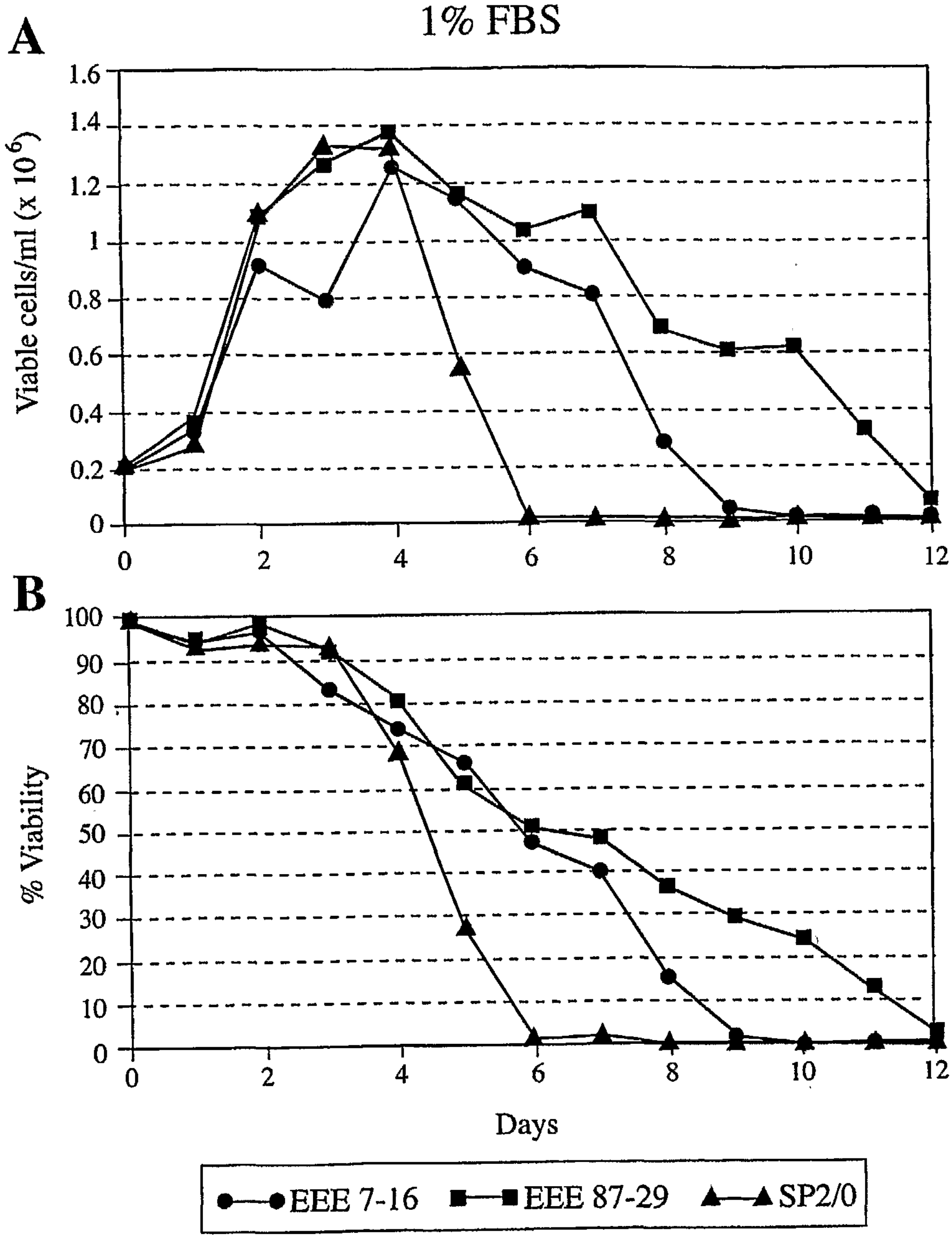
A
1% FBS
Viable cells/ml (x 10^6)
1.6
1.4
1.2
1
0.8
0.6
0.4
0.2
0
0
2
4
6
8
10
12
B
% Viability
100
90
80
70
60
50
40
30
20
10
0
Days
EEE 7-16
EEE 87-29
SP2/0

# Fig. 13

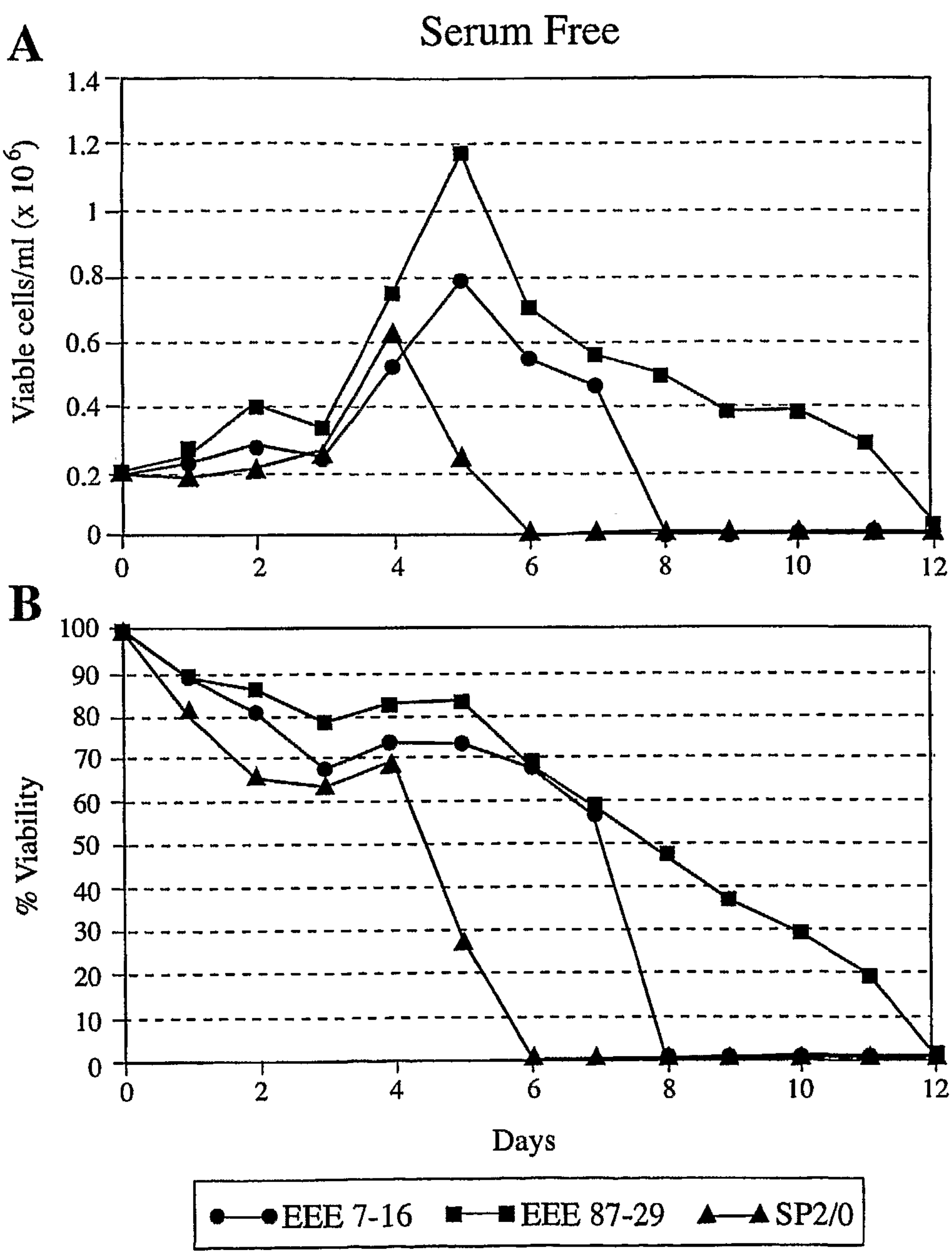
A
Serum Free
Viable cells/ml (x $10^6$)
1.4
1.2
1
0.8
0.6
0.4
0.2
0
0
2
4
6
8
10
12
B
% Viability
100
90
80
70
60
50
40
30
20
10
0
0
2
4
6
8
10
12
Days
EEE 7-16
EEE 87-29
SP2/0

Fig. 14

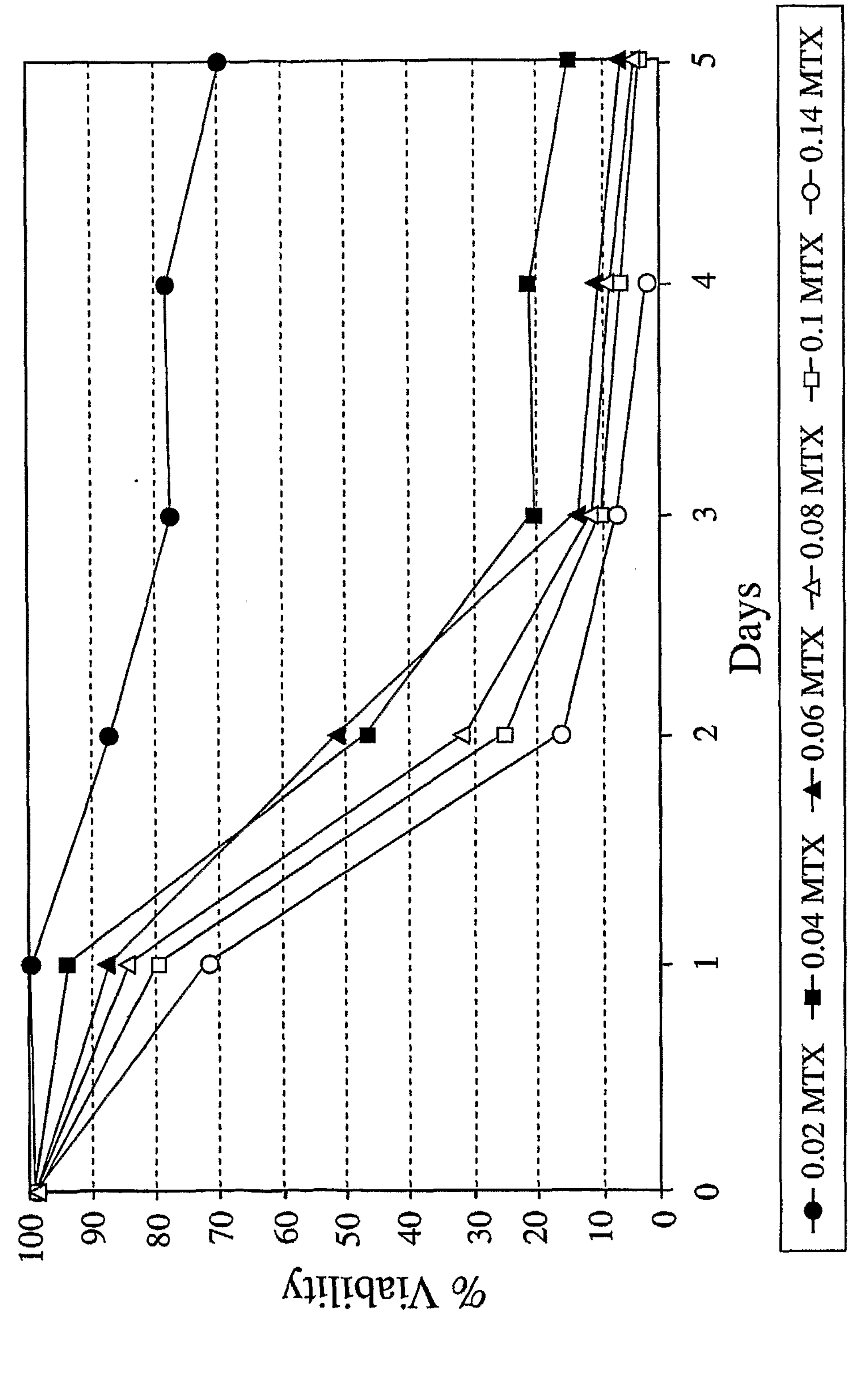
% Viability
100
90
80
70
60
50
40
30
20
10
0
Days
0
1
2
3
4
5
0.02 MTX
0.04 MTX
0.06 MTX
0.08 MTX
0.1 MTX
0.14 MTX

Fig. 15

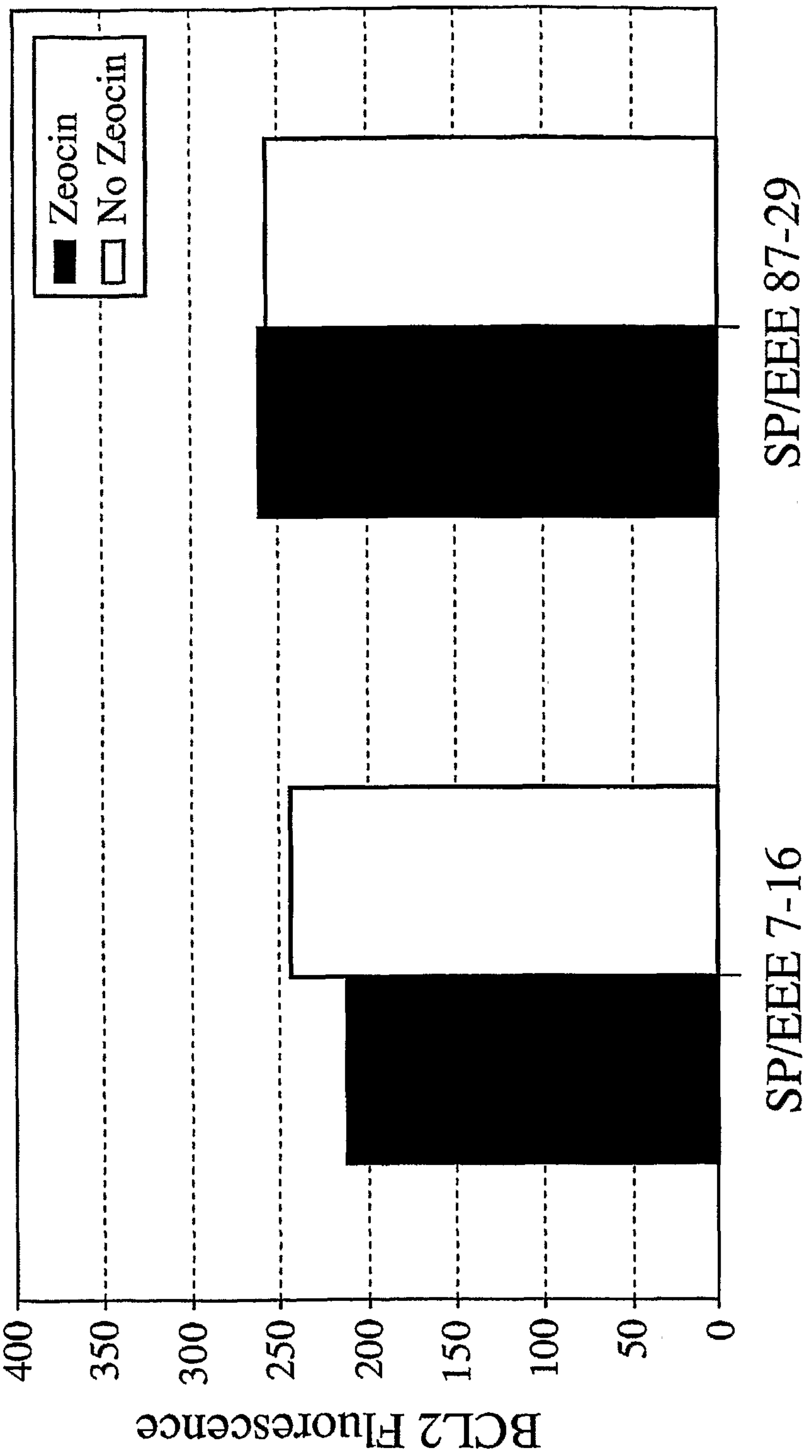
Zeocin
No Zeocin
BCL2 Fluorescence
0
50
100
150
200
250
300
350
400
SP/EEE 7-16
SP/EEE 87-29

Fig. 16

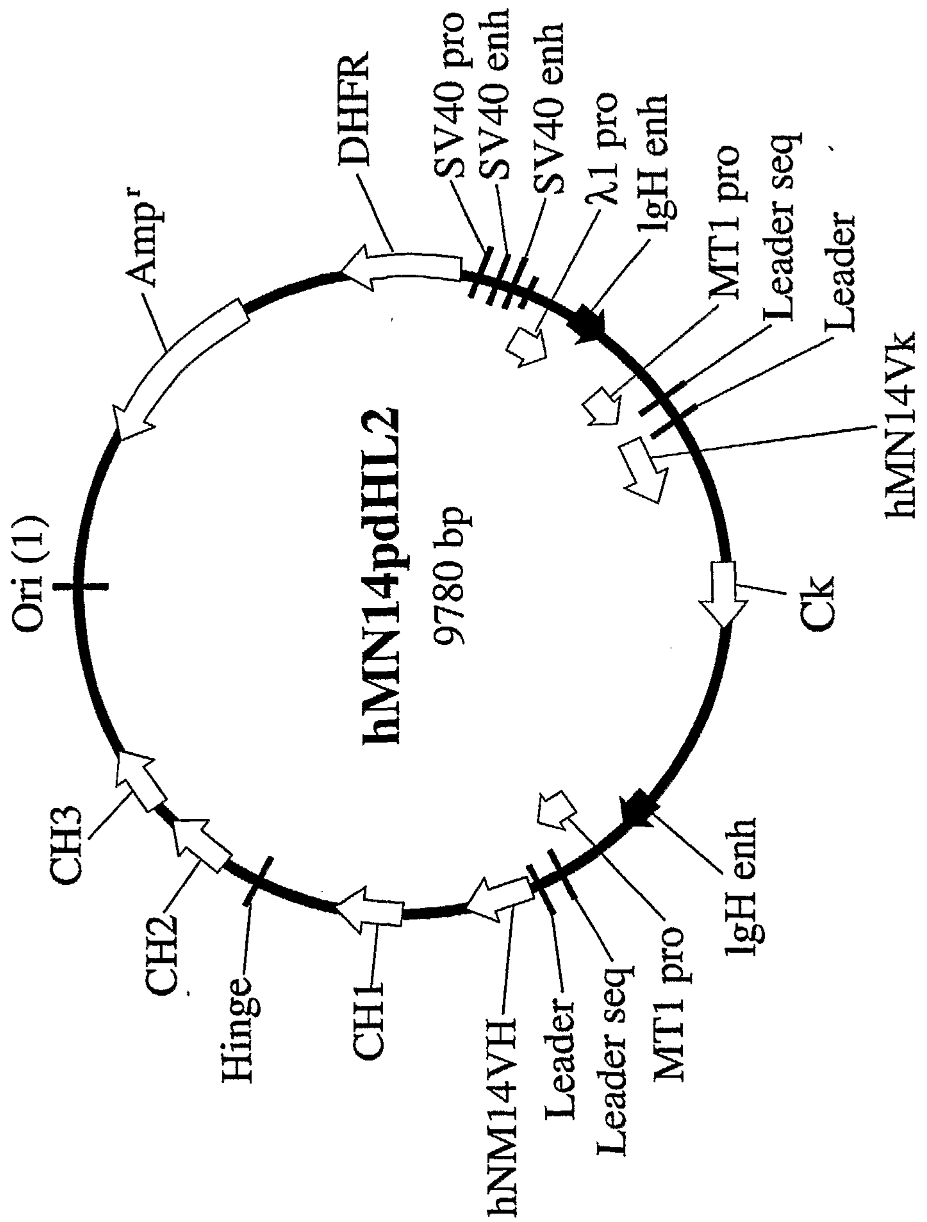
hMN14pdHL2
9780 bp
Ori (1)
Amp^r
DHFR
SV40 pro
SV40 enh
SV40 enh
λ1 pro
IgH enh
MT1 pro
Leader seq
Leader
hMN14Vk
Ck
IgH enh
MT1 pro
Leader seq
Leader
hNM14VH
CH1
Hinge
CH2
CH3

# Fig. 17

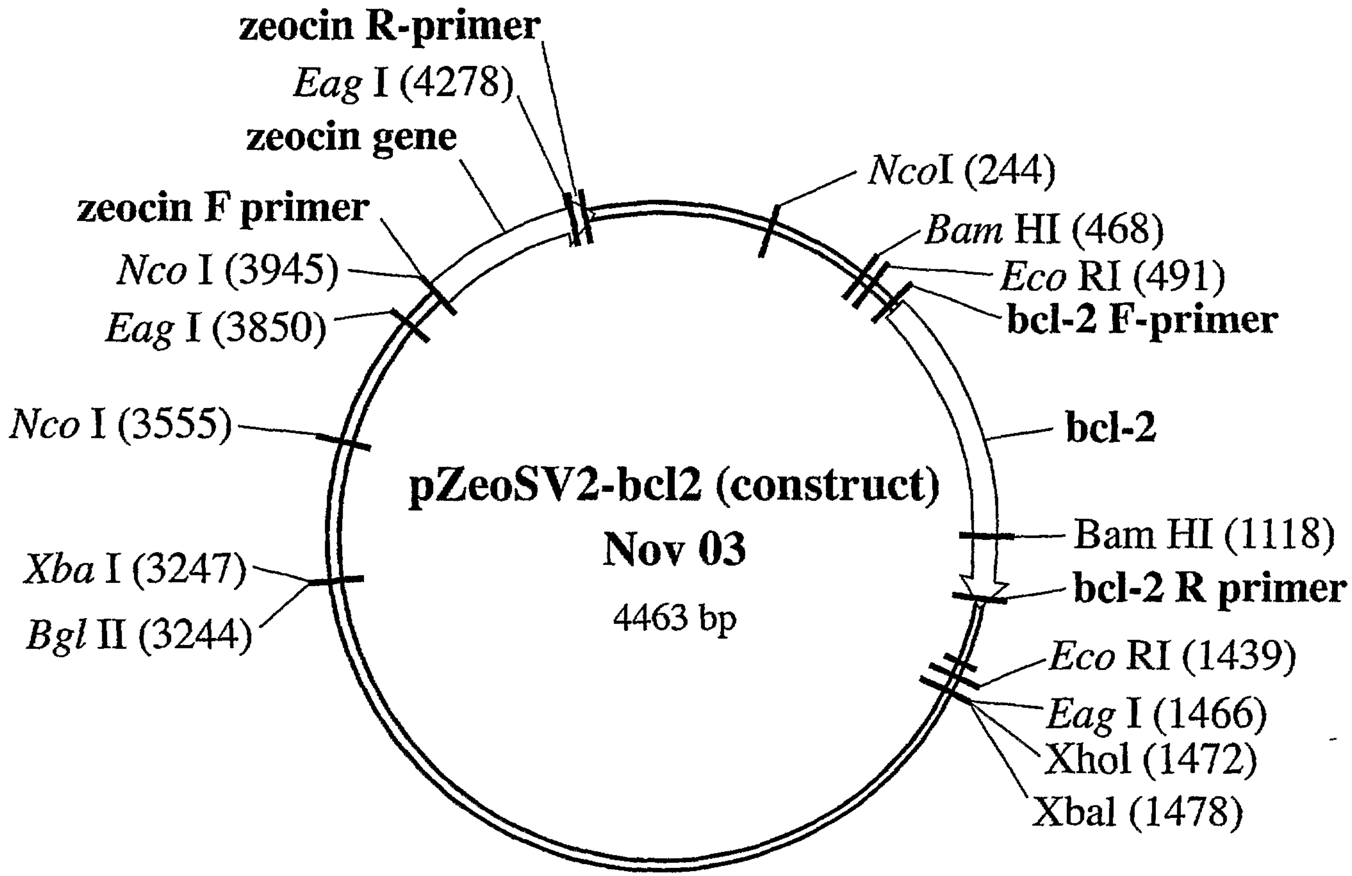
zeocin R-primer
Eag I (4278)
zeocin gene
zeocin F primer
Nco I (3945)
Eag I (3850)
Nco I (3555)
Xba I (3247)
Bgl II (3244)
NcoI (244)
Bam HI (468)
Eco RI (491)
bcl-2 F-primer
bcl-2
Bam HI (1118)
bcl-2 R primer
Eco RI (1439)
Eag I (1466)
XhoI (1472)
XbaI (1478)
pZeoSV2-bcl2 (construct)
Nov 03
4463 bp

Fig. 18

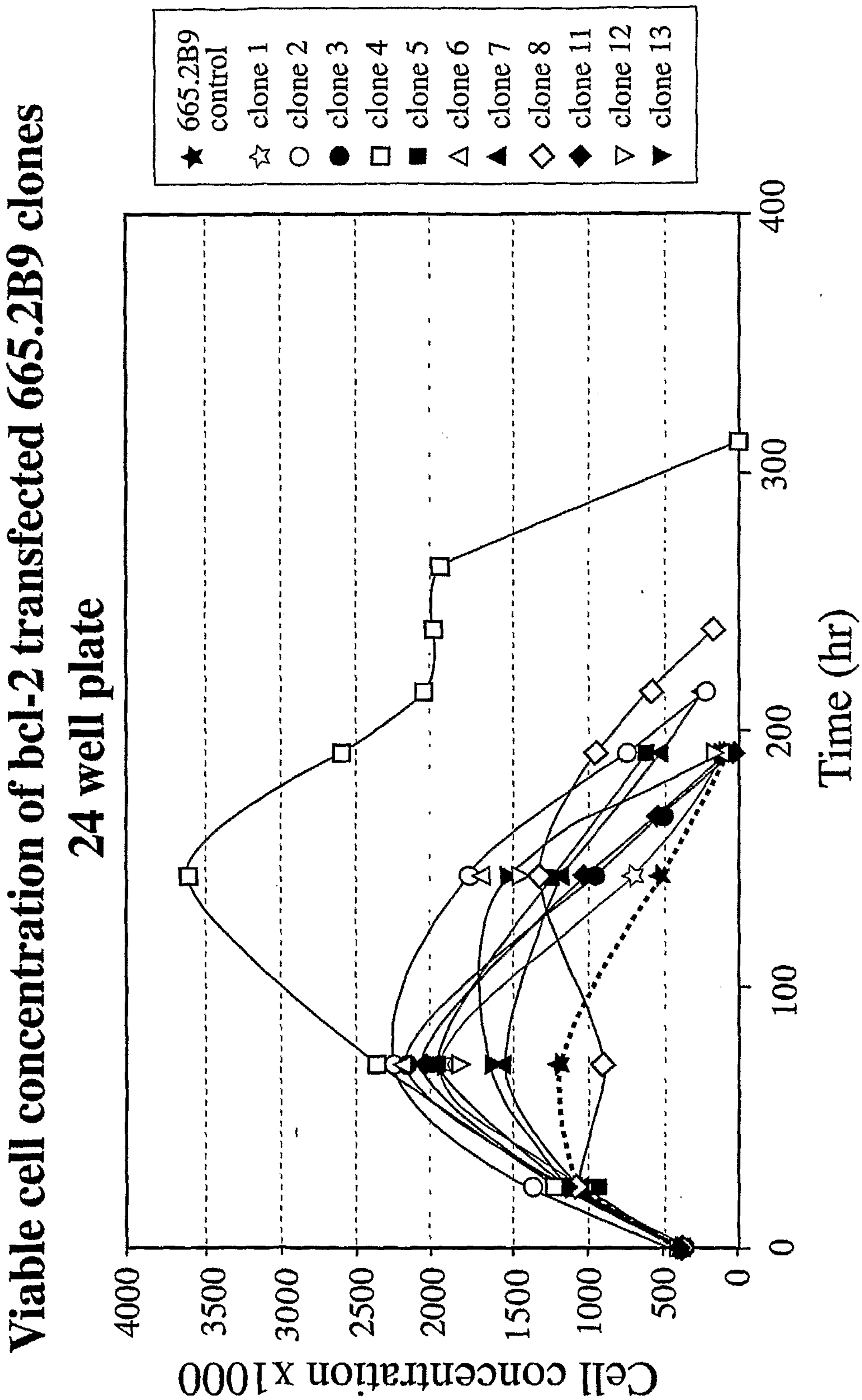
Viable cell concentration of bcl-2 transfected 665.2B9 clones
24 well plate
Cell concentration x1000
4000
3500
3000
2500
2000
1500
1000
500
0
0
100
200
300
400
Time (hr)
665.2B9 control
clone 1
clone 2
clone 3
clone 4
clone 5
clone 6
clone 7
clone 8
clone 11
clone 12
clone 13

Fig. 19

**Viability of bcl-2 transfected 665.2B9 clones**
**24 well plate**

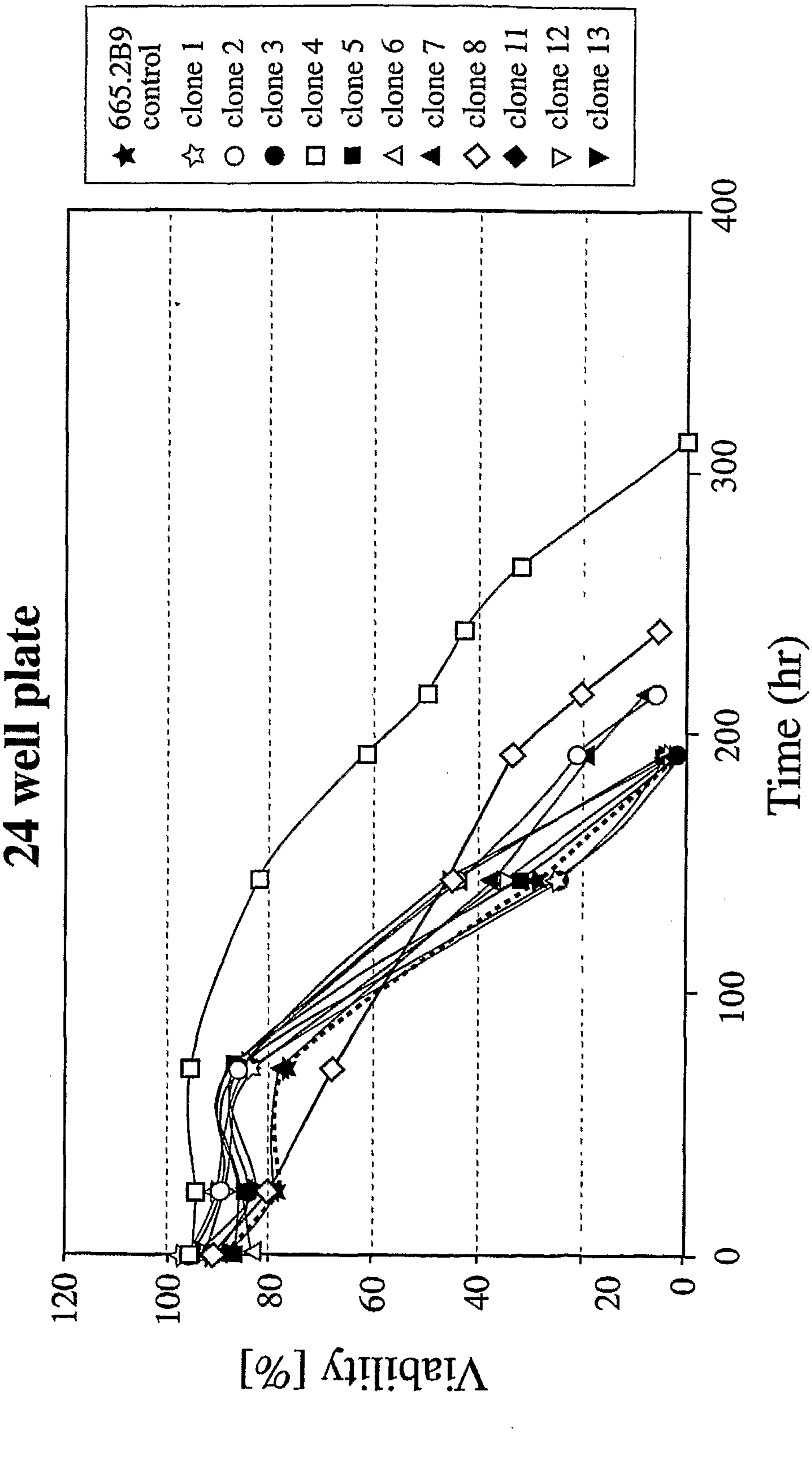

Fig. 20

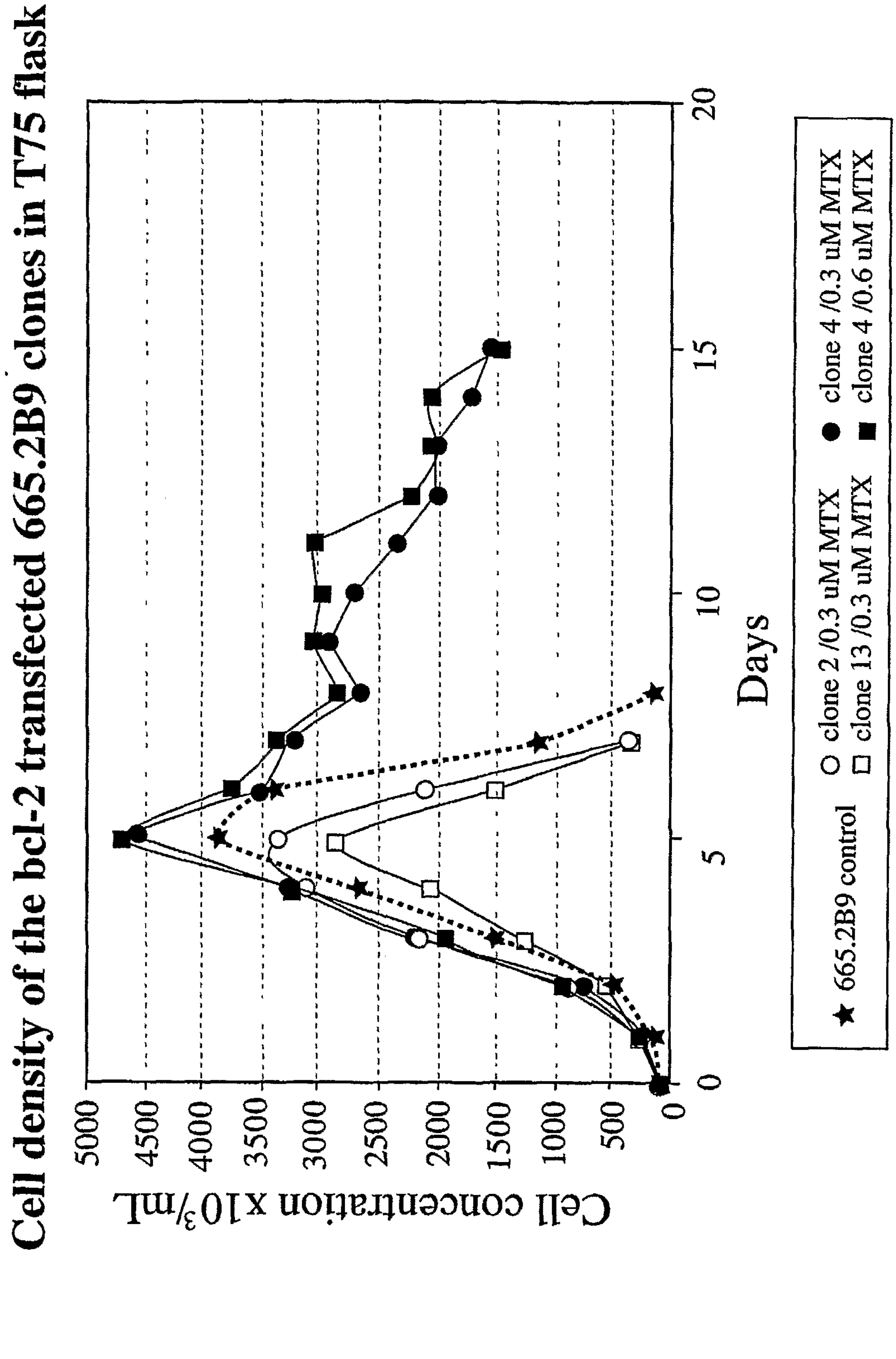
Cell density of the bcl-2 transfected 665.2B9 clones in T75 flask
Cell concentration x10^3/mL
0
500
1000
1500
2000
2500
3000
3500
4000
4500
5000
0
5
10
15
20
Days
665.2B9 control
clone 2 /0.3 uM MTX
clone 13 /0.3 uM MTX
clone 4 /0.3 uM MTX
clone 4 /0.6 uM MTX

Fig. 21

Viability profile of bcl-2 transfected 665.2B9 clones in T75 flask

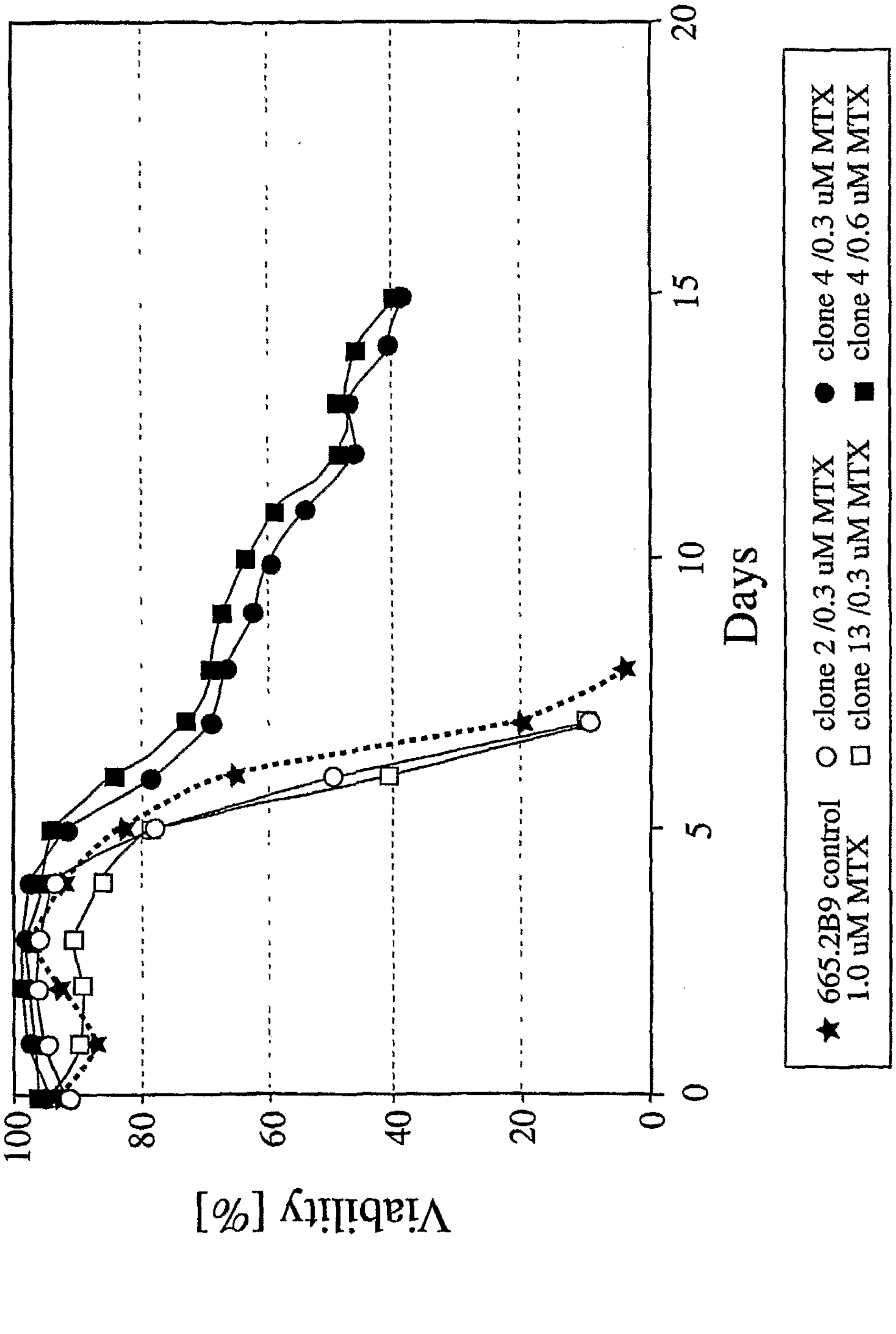

# Fig. 22

## Western Blot bcl-2, β-Actin

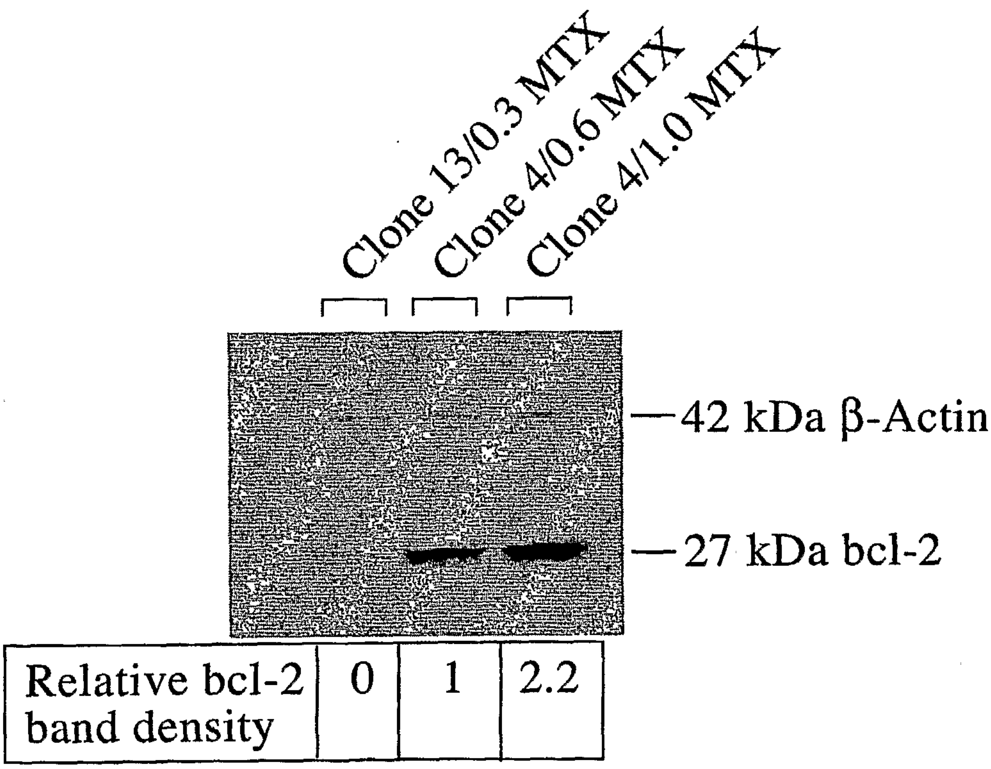


| | | | |
|---|---|---|---|
| Relative bcl-2 band density | 0 | 1 | 2.2 |

# Fig. 23

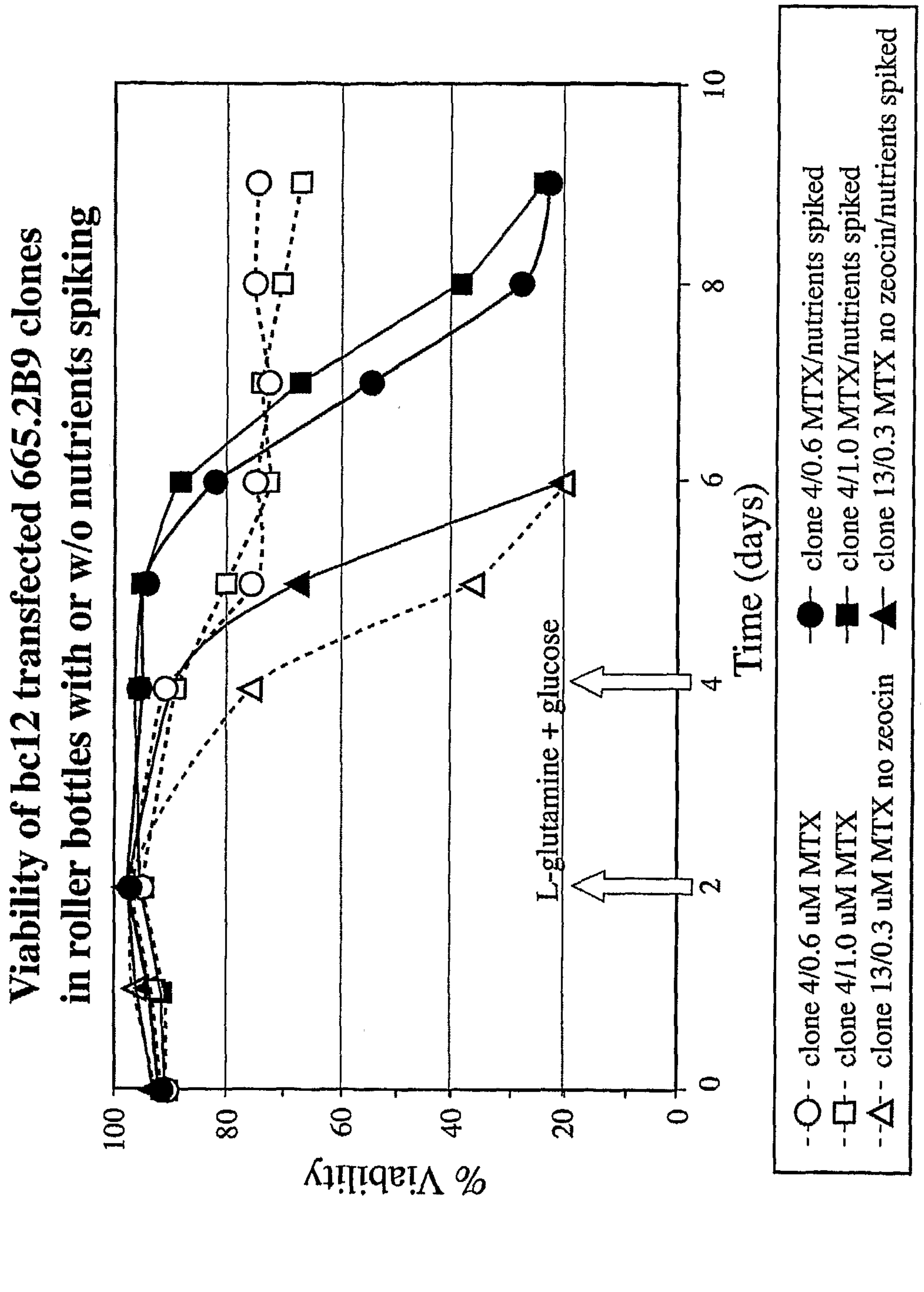

Viability of bc12 transfected 665.2B9 clones
in roller bottles with or w/o nutrients spiking
% Viability
100
80
60
40
20
0
0
2
4
6
8
10
Time (days)
L-glutamine + glucose
clone 4/0.6 uM MTX
clone 4/1.0 uM MTX
clone 13/0.3 uM MTX no zeocin
clone 4/0.6 MTX/nutrients spiked
clone 4/1.0 MTX/nutrients spiked
clone 13/0.3 MTX no zeocin/nutrients spiked

Fig. 24

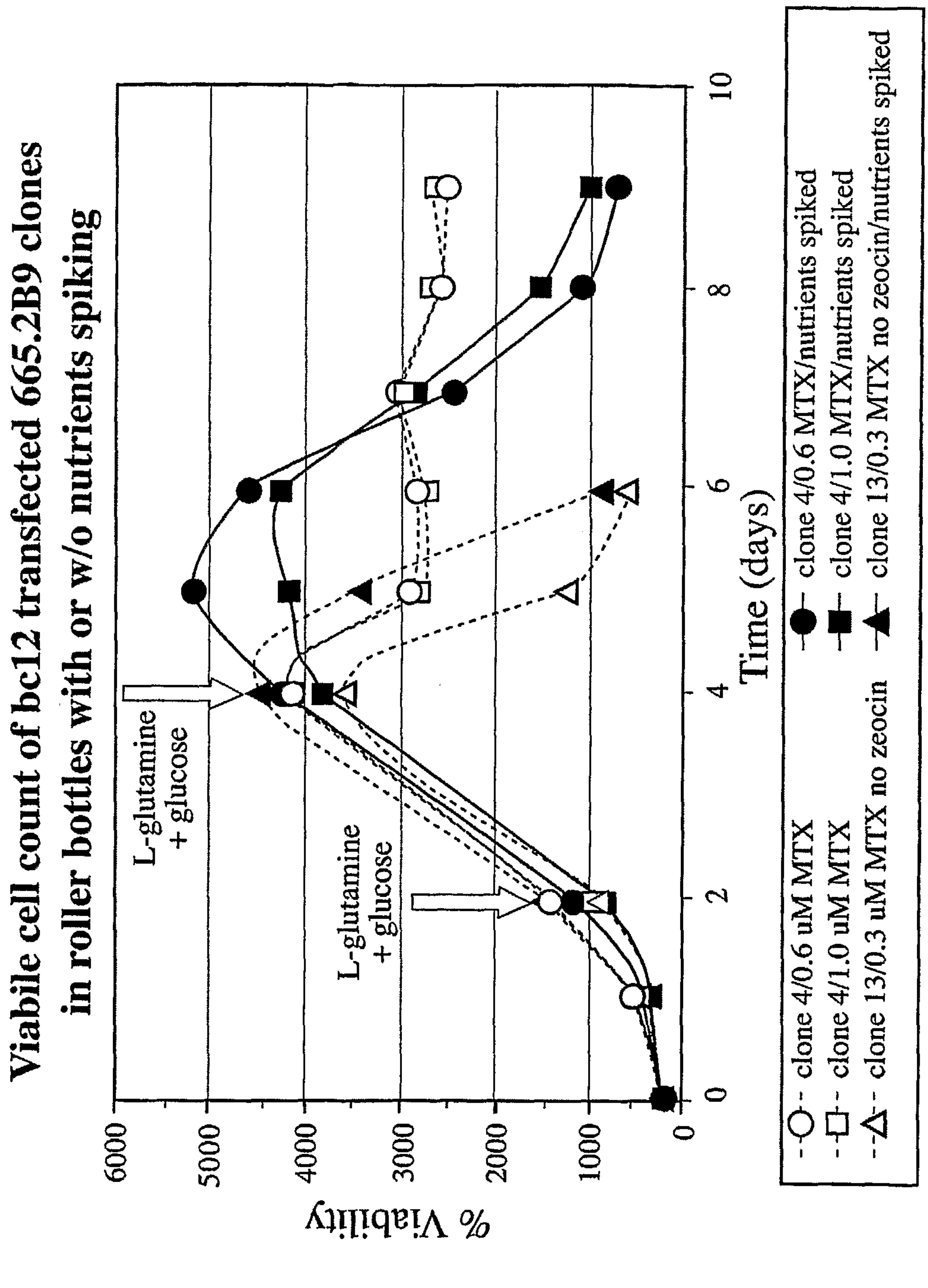
Viabile cell count of bc12 transfected 665.2B9 clones
in roller bottles with or w/o nutrients spiking
% Viability
6000
5000
4000
3000
2000
1000
0
0
2
4
6
8
10
Time (days)
L-glutamine
+ glucose
L-glutamine
+ glucose
clone 4/0.6 uM MTX
clone 4/1.0 uM MTX
clone 13/0.3 uM MTX no zeocin
clone 4/0.6 MTX/nutrients spiked
clone 4/1.0 MTX/nutrients spiked
clone 13/0.3 MTX no zeocin/nutrients spiked

Fig. 25

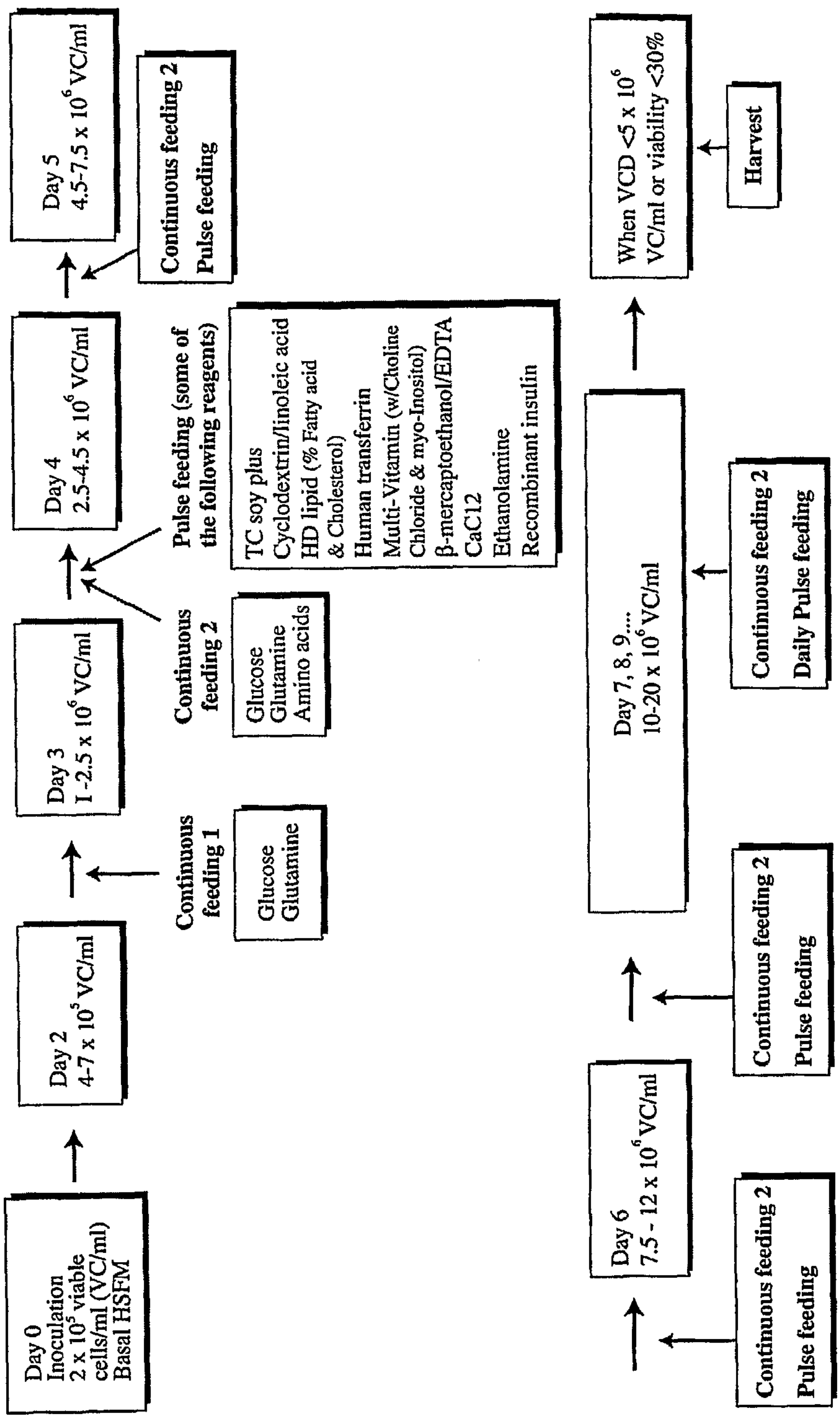
Day 0
Inoculation
2 x 10^5 viable
cells/ml (VC/ml)
Basal HSFM
Day 2
4-7 x 10^5 VC/ml
Continuous
feeding 1
Glucose
Glutamine
Day 3
1 -2.5 x 10^6 VC/ml
Continuous
feeding 2
Glucose
Glutamine
Amino acids
Pulse feeding (some of
the following reagents)
TC soy plus
Cyclodextrin/linoleic acid
HD lipid (% Fatty acid
& Cholesterol)
Human transferrin
Multi-Vitamin (w/Choline
Chloride & myo-Inositol)
β-mercaptoethanol/EDTA
CaC12
Ethanolamine
Recombinant insulin
Day 4
2.5-4.5 x 10^6 VC/ml
Continuous feeding 2
Pulse feeding
Day 5
4.5-7.5 x 10^6 VC/ml
Continuous feeding 2
Pulse feeding
Day 6
7.5 - 12 x 10^6 VC/ml
Continuous feeding 2
Pulse feeding
Day 7, 8, 9....
10-20 x 10^6 VC/ml
Continuous feeding 2
Daily Pulse feeding
When VCD <5 x 10^6
VC/ml or viability <30%
Harvest

Fig. 26

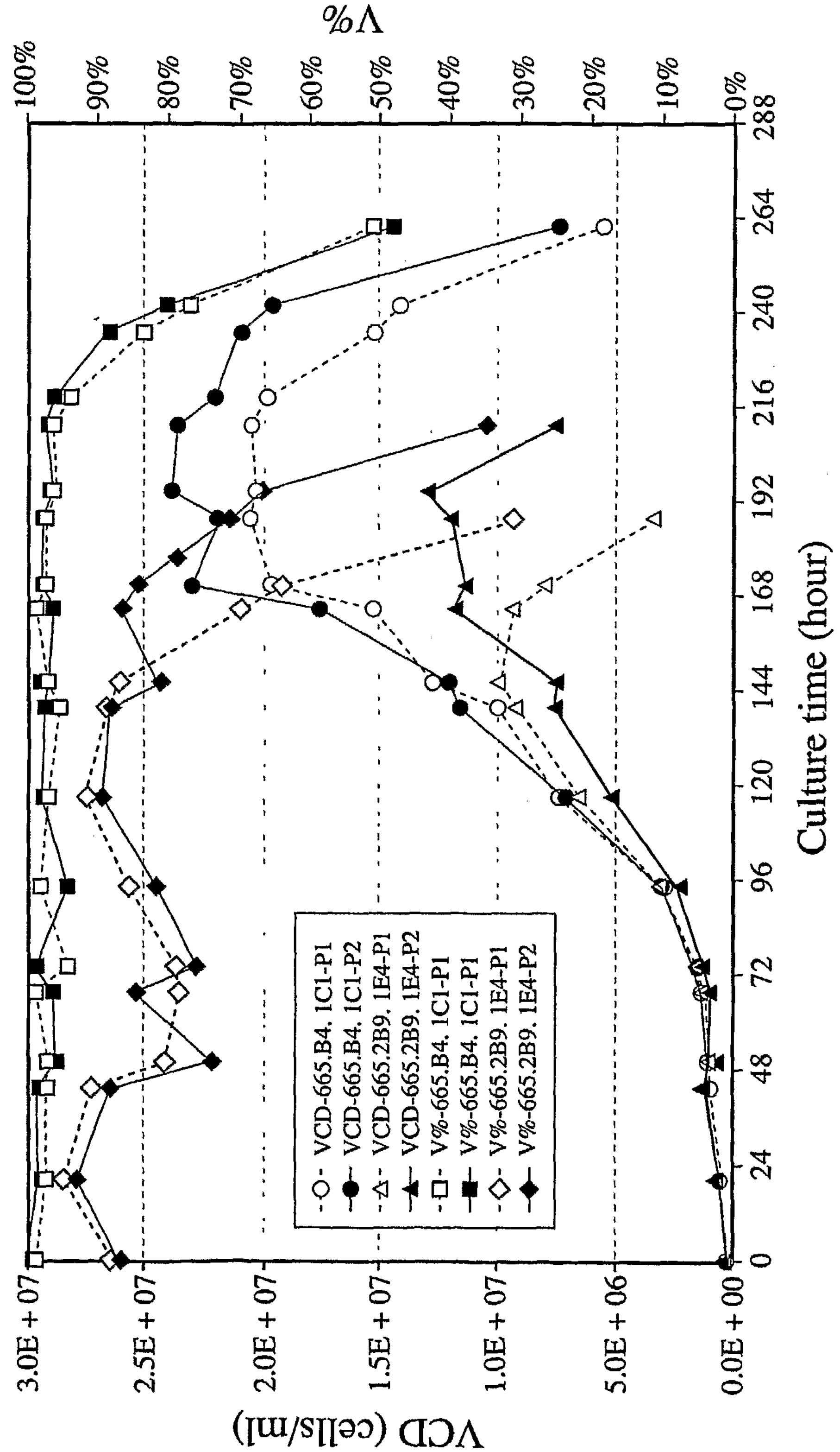
V%
100%
90%
80%
70%
60%
50%
40%
30%
20%
10%
0%
VCD (cells/ml)
3.0E + 07
2.5E + 07
2.0E + 07
1.5E + 07
1.0E + 07
5.0E + 06
0.0E + 00
Culture time (hour)
0
24
48
72
96
120
144
168
192
216
240
264
288
VCD-665.B4. 1C1-P1
VCD-665.B4. 1C1-P2
VCD-665.2B9. 1E4-P1
VCD-665.2B9. 1E4-P2
V%-665.B4. 1C1-P1
V%-665.B4. 1C1-P1
V%-665.2B9. 1E4-P1
V%-665.2B9. 1E4-P2

Fig. 27

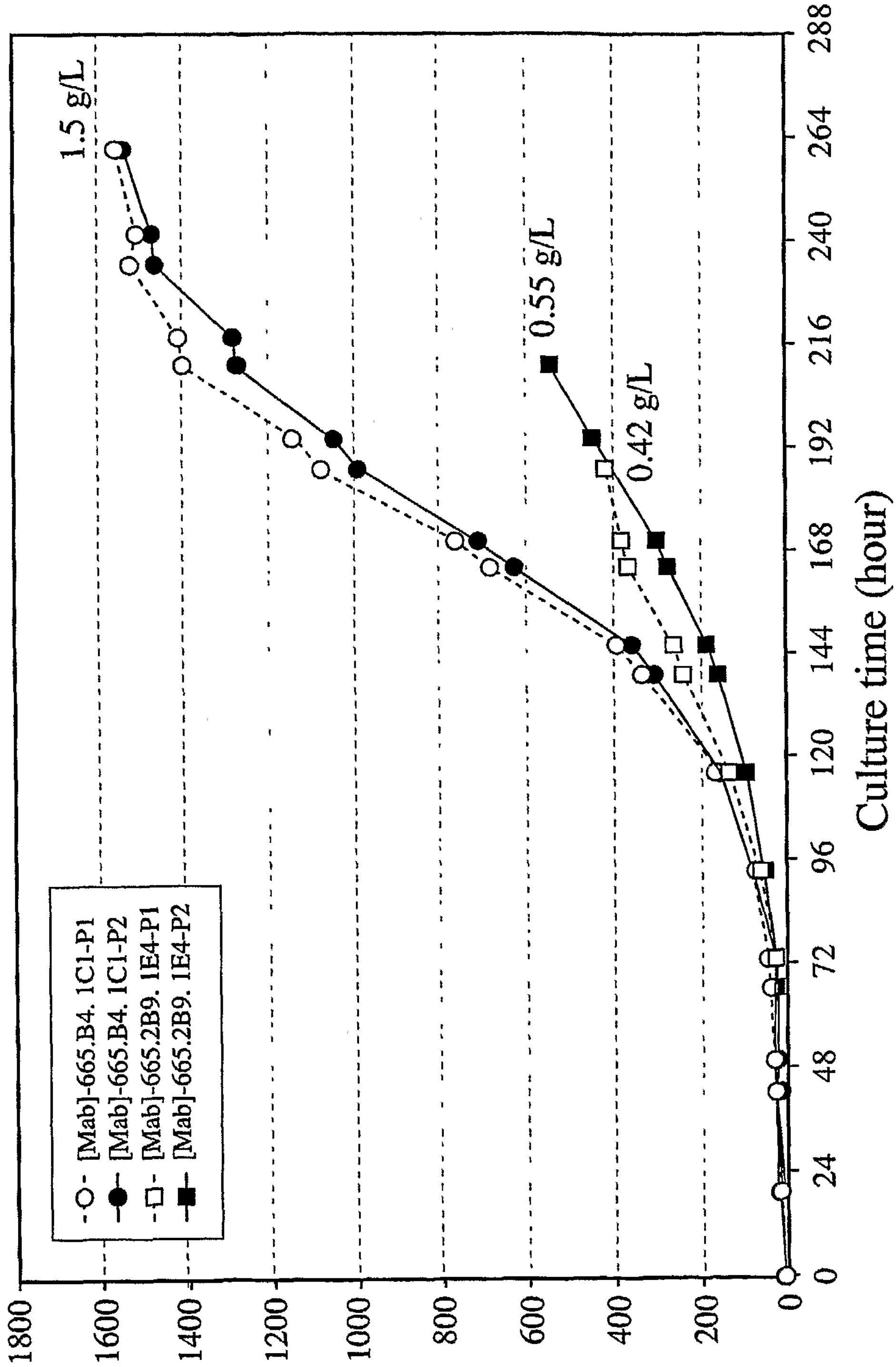
[Mab]-665.B4. 1C1-P1
[Mab]-665.B4. 1C1-P2
[Mab]-665.2B9. 1E4-P1
[Mab]-665.2B9. 1E4-P2
1.5 g/L
0.55 g/L
0.42 g/L
Antibody Concentration (mg/L)
0
200
400
600
800
1000
1200
1400
1600
1800
Culture time (hour)
0
24
48
72
96
120
144
168
192
216
240
264
288

Fig. 28

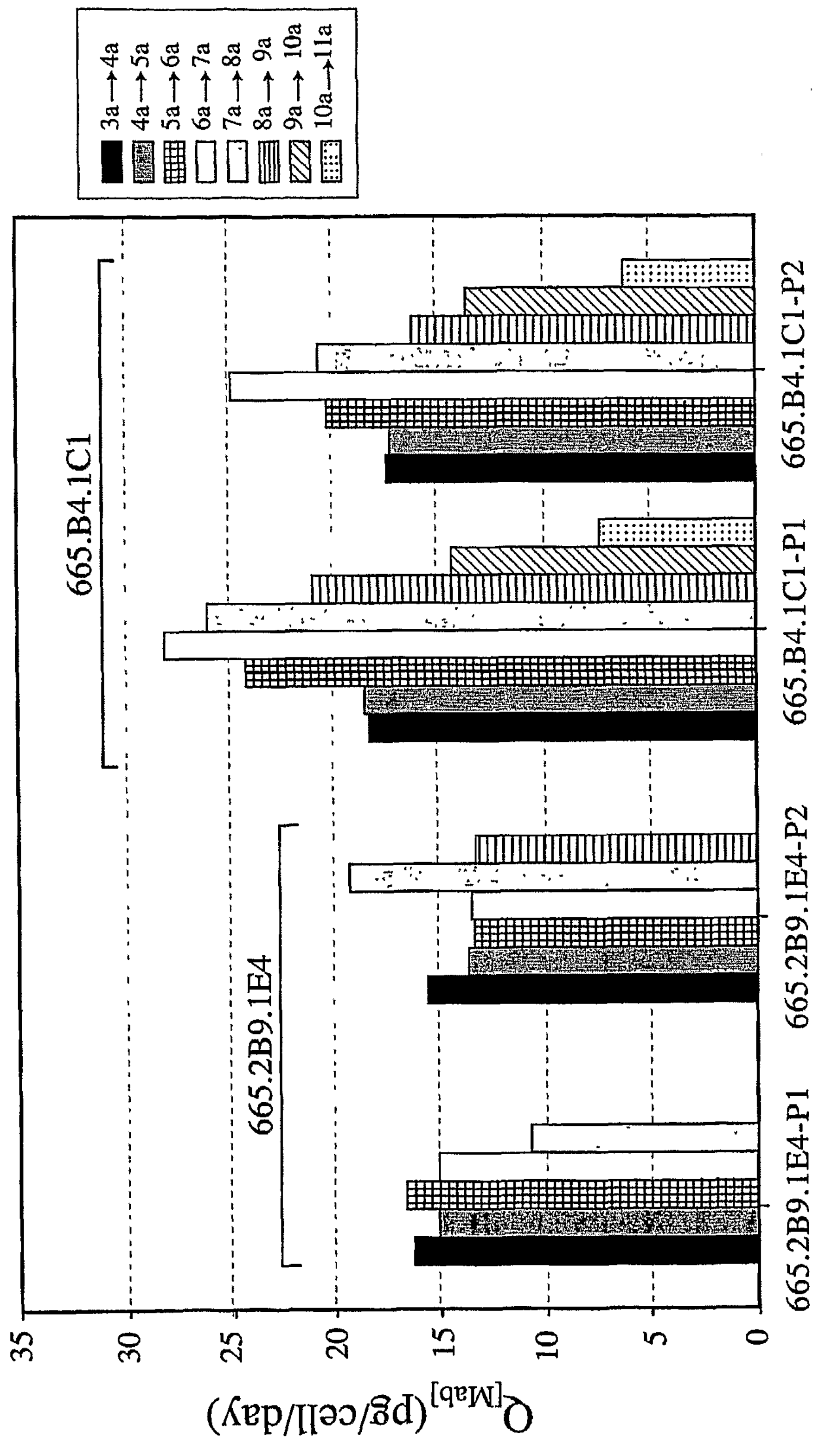
3a→4a
4a→5a
5a→6a
6a→7a
7a→8a
8a→9a
9a→10a
10a→11a
665.2B9.1E4
665.B4.1C1
665.2B9.1E4-P1
665.2B9.1E4-P2
665.B4.1C1-P1
665.B4.1C1-P2
0
5
10
15
20
25
30
35
Q[Mab](pg/cell/day)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20020160450 A1, Bucciarelli
- WO 0216590 A2
- WO 0240665 A2, Vogels
- US 6586206 B
- WO 03083093 A
- WO 03040374 A
- US 6964199 B


### Non-patent literature cited in the description


- **GOLDSTEIN ; SINGAL.** *Exp Cell Res,* 1974, vol. 88, 359-64
- **BRENNER et al.** *Oncogene,* 1998, vol. 17, 199-205
- **CHANG et al.** *Proc Natl Acad Sci USA,* 2000, vol. 97, 4291-6
- **JAVELAND et al.** *Oncogene,* 2000, vol. 19, 61-8
- **ARDEN et al.** *Bioprocessing Journal,* 2004, vol. 3, 23-28
- **TEY et al.** *Biotechnol. Bioeng.,* 2000, vol. 68, 31-43
- **P. GHEZZI ; M. BRINES.** *Cell Death and Differentiation,* July 2004, vol. 11 (1), s37-s44
- **TEY et al.** *J. Biotechnol.,* 2000, vol. 79, 147-159
- **ZHANG et al.** *J. Chem. Technol. Biotechnol.,* 2004, vol. 79, 171-181
- **ZHOU et al.** *Biotechnol. Bioeng.,* 1997, vol. 55, 783-792
- **BIN YANG et al.** *Nephron Experimental Nephrology,* 2004, vol. 96, e39-e51
- **KERR et al.** *Br J Cancer.,* 1972, vol. 26, 239-257
- **ELLIS et al.** *Annu Rev Cell Biol,* 1991, vol. 7, 663-698
- **VAUX et al.** *Nature,* 1988, vol. 335, 440-2
- **HUANG et al.** *EMBO J,* 1997, vol. 16, 4628-38
- **OLTVAI et al.** *Cell,* 1993, vol. 74, 609-19
- **CHITTENDEN et al.** *Nature,* 1995, vol. 374, 733-6
- **FARROW et al.** *Nature,* 1995, vol. 374, 731-3
- **KIEFER et al.** *Nature,* 1995, vol. 374, 736-9
- **SCHWARZ et al.** *Cancer Res,* 1995, vol. 55, 2262-5
- **DENG et al.** *PNAS,* 2004, vol. 101, 153-158
- **FINZER et al.** *Cancer Lett,* 2002, vol. 188, 15-24
- **STOREY.** *Trends Mol Med,* 2002, vol. 8, 417-21
- **JACKSON et al.** *Br J Cancer,* 2002, vol. 87, 319-23
- **LIU et al.** *Virology,* 2002, vol. 295, 230-7
- **LEE et al.** *Yonsei Med J,* 2001, vol. 42, 471-9
- **BUTZ et al.** *Proc Natl Acad Sci USA,* 2000, vol. 97, 6693-7
- **WEBSTER et al.** *J Biol Chem,* 2000, vol. 275, 87-94
- **DEFILIPPIS et al.** *J Virol,* 2003, vol. 77, 1551-63
- **MILLER et al.** *Biotechniques,* 1989, vol. 7, 980-90
- **LEUNG et al.** *Tumor Targeting,* 1996, vol. 2, 184
- **LOSMAN et al.** *Cancer,* 1997, vol. 80, 2660-2667
- **LOSMAN et al.** *Cancer,* 1997, vol. 80, 2660-2666
- **FIGUEROA et al.** *Biotechnology and Bioengineering,* 2001, vol. 73, 211-222
- **CHANG et al.** *EMBO J.,* 1997, vol. 16, 968-977
- **DENG et al.** *PNAS,* 2004, vol. 101, 153-158
- **GILLIES et al.** *J. Immunol. Methods,* 1989, vol. 125, 191
- **LEUNG et al.** *J. Nuc. Med.,* 2000, vol. 41, 270P
- **HAYES et al.** *Proc. Am. Asso. Cancer. Res.,* 2002, vol. 43, 969